(19) Europäisches Patentamt
European Patent Office
Office européen des brevets

(11) **EP 2 547 398 B1**

(12) **EUROPÄISCHE PATENTSCHRIFT**

(45) Veröffentlichungstag und Bekanntmachung des Hinweises auf die Patenterteilung:
**12.03.2014  Patentblatt 2014/11**

(21) Anmeldenummer: **11720022.0**

(22) Anmeldetag: **16.02.2011**

(51) Int Cl.:
*A61N 5/06* (2006.01)      *A61M 21/00* (2006.01)
*A61H 23/00* (2006.01)

(86) Internationale Anmeldenummer:
**PCT/DE2011/075025**

(87) Internationale Veröffentlichungsnummer:
**WO 2011/127918 (20.10.2011 Gazette 2011/42)**

(54) **VORRICHTUNG ZUR KONDITIONIERTEN DESYNCHRONISIERENDEN NICHT-INVASIVEN STIMULATION**

DEVICE FOR CONDITIONED DESYNCHRONIZING NON-INVASIVE STIMULATION

DISPOSITIF POUR LA STIMULATION DÉSYNCHRONISANTE CONDITIONNÉE NON INVASIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.04.2010  DE 102010016404**

(43) Veröffentlichungstag der Anmeldung:
**23.01.2013  Patentblatt 2013/04**

(73) Patentinhaber: **Forschungszentrum Jülich GmbH
52425 Jülich (DE)**

(72) Erfinder: **TASS, Peter Alexander
81541 München (DE)**

(74) Vertreter: **Manitz, Finsterwald & Partner GbR
Martin-Greif-Strasse 1
80336 München (DE)**

(56) Entgegenhaltungen:
**EP-A2- 2 103 288      WO-A1-2009/136931
DE-A1-102005 014 383**

**Beschreibung**

Die Erfindung betrifft eine Vorrichtung zur konditionierten desynchronisierenden nicht-invasiven Stimulation.

Bei Patienten mit neurologischen oder psychiatrischen Erkrankungen, z.B. Morbus Parkinson, essentiellem Tremor, Dystonie oder Zwangserkrankungen, sind Nervenzellverbände in umschriebenen Bereichen des Gehirns, z.B. des Thalamus und der Basalganglien, krankhaft, z.B. übersteigert synchron, aktiv. In diesem Fall bildet eine große Anzahl von Neuronen synchron Aktionspotentiale aus, d.h. die beteiligten Neuronen feuern übermäßig synchron. Beim Gesunden hingegen feuern die Neuronen in diesen Hirngebieten qualitativ anders, z.B. auf unkorrelierte Weise.

Beim Morbus Parkinson verändert die pathologisch synchrone Aktivität die neuronale Aktivität in anderen Hirngebieten, z.B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z.B. ein rhythmisches Zittern (Tremor), entfalten.

Neurologische und psychiatrische Erkrankungen mit übermäßig stark ausgeprägter neuronaler Synchronisation werden bis jetzt - bei Versagen einer medikamentösen Therapie - durch elektrische Hirnstimulation behandelt.

Die Druckschrift EP 2 103 288 A2 beschreibt eine Vorrichtung zur auditorischen Stimulation, mit der sich eine krankhaft synchrone Neuronenpopulation desynchronisieren lässt. Die Druckschrift WO 2009 / 136 931 A beschreibt eine Vorrichtung mit einer Mehrzahl von Stimulationseinheiten zur Applikation von vibratorischen Reizen.

Vor diesem Hintergrund wird eine Vorrichtung gemäß Anspruch 1 angegeben. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

| | |
|---|---|
| Fig. 1 | eine schematische Darstellung einer Vorrichtung zur konditionierten desynchronisierenden nicht-invasiven Stimulation gemäß einem Ausführungsbeispiel während des Betriebs; |
| Fig. 2A und 2B | schematische Darstellungen von zwei unterschiedlichen Betriebsmodi der in Fig. 1 dargestellten Vorrichtung; |
| Fig. 3 | eine schematische Darstellung einer Vorrichtung zur konditionierten desynchronisierenden nicht-invasiven Stimulation gemäß einem weiteren Ausführungsbeispiel während des Betriebs; |
| Fig. 4A und 4B | schematische Darstellungen einer Stimulationseinheit zur Erzeugung und Applikation von unspezifischen optischen, akustischen, taktilen, vibratorischen und/oder thermischen Reizen gemäß einem Ausführungsbeispiel; |
| Fig. 5 | eine schematische Darstellung einer Stimulationseinheit zur Erzeugung und Applikation von unspezifischen akustischen Reizen gemäß einem weiteren Ausführungsbeispiel; |
| Fig. 6 | eine schematische Darstellung einer Stimulationseinheit zur Erzeugung und Applikation von spezifischen optischen Reizen gemäß einem Ausführungsbeispiel; |
| Fig. 7 | eine schematische Darstellung des Gesichtsfelds eines Patienten; |
| Fig. 8 | eine schematische Darstellung einer Stimulationseinheit zur Erzeugung und Applikation von spezifischen optischen Reizen gemäß einem weiteren Ausführungsbeispiel; |
| Fig. 9 | eine schematische Darstellung einer Stimulationseinheit zur Erzeugung und Applikation von spezifischen optischen Reizen gemäß einem weiteren Ausführungsbeispiel; |
| Fig. 10 | eine schematische Darstellung einer Vorrichtung zur konditionierten desynchronisierenden nicht-invasiven Stimulation gemäß einem weiteren Ausführungsbeispiel während des Betriebs; |
| Fig. 11 und 12 | schematische Darstellungen von Transmissionsbrillen; |
| Fig. 13 bis 16 | schematische Darstellungen von mittels einer Transmissionsbrille erzeugten spezifischen optischen Reizen; |

| | |
|---|---|
| Fig. 17 und 18 | schematische Darstellungen von Lichtbrillen; |
| Fig. 19 | eine schematische Darstellung von mittels einer Lichtbrille erzeugten spezifischen optischen Reizen; |
| Fig. 20 | eine schematische Darstellung einer Stimulationseinheit zur Erzeugung und Applikation von spezifischen akustischen Reizen gemäß einem Ausführungsbeispiel; |
| Fig. 21 | eine Darstellung von Sinusschwingungen mit verschiedenen Frequenzen; |
| Fig. 22 | eine Darstellung einer mit einer Rechteckfunktion amplitudenmodulierten Sinusschwingung; |
| Fig. 23 | eine schematische Darstellung einer Stimulationseinheit zur Erzeugung und Applikation von spezifischen akustischen Reizen gemäß einem weiteren Ausführungsbeispiel; |
| Fig. 24 | eine schematische Darstellung einer Stimulationseinheit zur Erzeugung und Applikation von spezifischen akustischen Reizen gemäß einem weiteren Ausführungsbeispiel; |
| Fig. 25 | eine schematische Darstellung einer Vorrichtung zur konditionierten desynchronisierenden nicht-invasiven Stimulation gemäß einem weiteren Ausführungsbeispiel während des Betriebs; |
| Fig. 26 bis 30 | schematische Darstellungen von akustischen Stimulationsverfahren; |
| Fig. 31A und 31B | schematische Darstellungen der Generierung von Modulationssignalen; |
| Fig. 32 | eine schematische Darstellung einer Stimulationseinheit zur Erzeugung und Applikation von spezifischen taktilen, vibratorischen und/oder thermischen Reizen gemäß einem Ausführungsbeispiel; |
| Fig. 33 | eine schematische Darstellung eines taktilen, vibratorischen und/oder thermischen Stimulationsverfahrens; |
| Fig. 34A bis 34D | schematische Darstellungen von spezifischen vibratorischen Reizen; |
| Fig. 35 | eine schematische Darstellung eines spezifischen taktilen Reizes; |
| Fig. 36A bis 36C | schematische Darstellungen von spezifischen thermischen Reizen; |
| Fig. 37 | eine schematische Darstellung einer Stimulationseinheit zur Erzeugung und Applikation von spezifischen taktilen, vibratorischen und/oder thermischen Reizen gemäß einem Ausführungsbeispiel; |
| Fig. 38 bis 40 | schematische Darstellungen von taktilen, vibratorischen und/oder thermischen Stimulationsverfahren; |
| Fig. 41 | eine schematische Darstellung einer Stimulationseinheit zur Erzeugung und Applikation von spezifischen taktilen, vibratorischen und/oder thermischen Reizen gemäß einem weiteren Ausführungsbeispiel; |
| Fig. 42A bis 44C | schematische Darstellungen eines Stimulationselements zur Erzeugung und Applikation von spezifischen taktilen und/oder vibratorischen Reizen; |
| Fig. 45A bis 46C | schematische Darstellungen eines Stimulationselements zur Erzeugung und Applikation von spezifischen thermischen Reizen; |
| Fig. 47 und 48 | schematische Darstellungen von spezifischen taktilen, vibratorischen und/oder thermischen Stimulationsverfahren; |
| Fig. 49 bis 50C | schematische Darstellungen einer Stimulationseinheit zur Erzeugung und Applikation von spezifischen taktilen, vibratorischen und/oder thermischen Reizen gemäß einem weiteren Ausführungs- |

beispiel; und

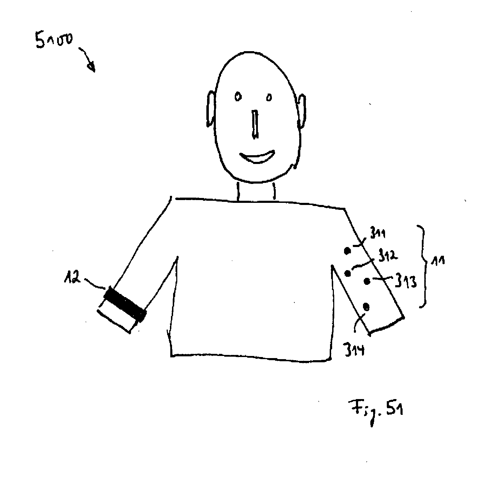

Fig. 51          eine schematische Darstellung einer Vorrichtung zur konditionierten desynchronisierenden nicht-invasiven Stimulation gemäß einem weiteren Ausführungsbeispiel während des Betriebs.

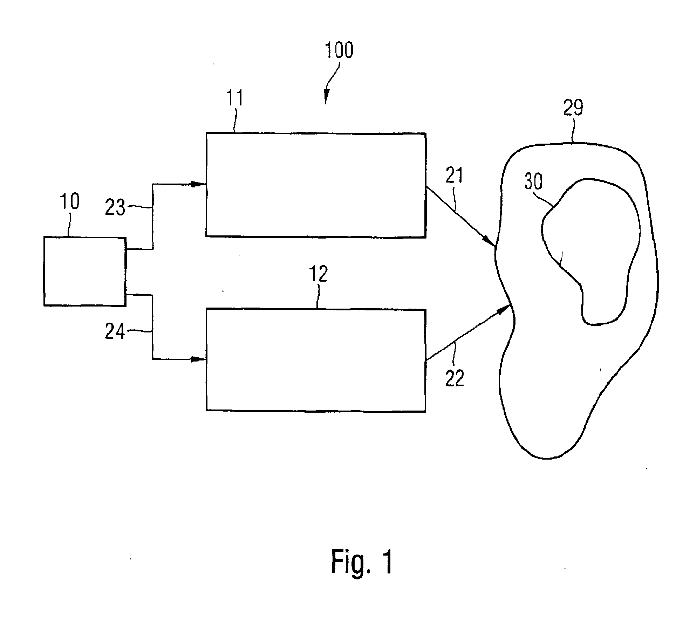

In Fig. 1 ist schematisch eine Vorrichtung 100 zur konditionierten desynchronisierenden nicht-invasiven Stimulation dargestellt. Die Vorrichtung 100 besteht aus einer Steuereinheit 10, einer ersten Stimulationseinheit 11 und einer zweiten Stimulationseinheit 12. Die erste Stimulationseinheit 11 erzeugt erste Reize 21 und die zweite Stimulationseinheit erzeugt zweite Reize 22. Sowohl die erste Stimulationseinheit 11 als auch die zweite Stimulationseinheit 12 sind nicht-invasive Einheiten, d.h. während des Betriebs der Vorrichtung 100 befinden sie sich außerhalb des Körpers des Patienten und werden nicht operativ in den Körper des Patienten implantiert. Die ersten und zweiten Reize 21, 22 können jeweils Reize sein aus der Gruppe von optischen, akustischen, taktilen, vibratorischen und thermischen Reizen sein. Die ersten und/oder zweiten Reize 21, 22 können vom Patienten bewusst wahrnehmbar sein. Die Steuereinheit 10 dient zur Steuerung der beiden Stimulationseinheiten 11 und 12 mittels Steuersignalen 23 bzw. 24.

Es kann durchaus vorgesehen sein, dass die einzelnen Komponenten der Vorrichtung 100, insbesondere die Steuereinheit 10, die erste Stimulationseinheit 11 und/oder die zweite Stimulationseinheit 12, baulich voneinander getrennt sind. Die Vorrichtung 100 kann daher auch als System aufgefasst werden.

Die Vorrichtung 100 kann insbesondere zur Behandlung von neurologischen oder psychiatrischen Erkrankungen, z.B. Morbus Parkinson, essentiellem Tremor, Dystonie, Epilepsie, Tremor infolge von Multipler Sklerose sowie anderen pathologischen Tremores, Depression, Bewegungsstörungen, Kleinhirnerkrankungen, Zwangserkrankungen, Tourette-Syndrom, Funktionsstörungen nach Schlaganfall, Spastik, Tinnitus, Schlafstörungen, Schizophrenie, Reizdarm-Syndrom, Suchterkrankungen, Persönlichkeitsstörungen, Aufmerksamkeits-Defizit-Syndrom, Aufmerksamkeits-Defizit-Hyperaktivitäts-Syndrom, Spielsucht, Neurosen, Fresssucht, Burnout-Syndrom, Fibromyalgie, Migräne, Cluster-Kopfschmerz, allgemeiner Kopfschmerz, Neuralgie, Ataxie, Tic-Störung oder Hypertonie, aber auch anderen Krankheiten verwendet werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h. die beteiligten Neuronen feuern übermäßig synchron. Hinzu kommt, dass die kranke Neuronenpopulation eine oszillatorische neuronale Aktivität aufweist, d.h. die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z.B. auf unkorrelierte Weise.

In Fig. 1 ist die Vorrichtung 100 während ihres bestimmungsgemäßen Betriebs dargestellt. Im Gehirn 29 oder Rückenmark 29 des Patienten weist mindestens eine Neuronenpopulation 30 eine wie vorstehend beschriebene krankhaft synchrone neuronale Aktivität auf. Die erste Stimulationseinheit 11 verabreicht dem Patienten die ersten Reize 21 derart, dass die ersten Reize 21 je nach Modalität über die Augen, die Ohren oder die Haut des Patienten aufgenommen werden und von dort über das Nervensystem an die krankhaft aktive Neuronenpopulation 30 im Gehirn 29 und/oder Rückenmark 29 weitergeleitet werden. Die ersten Reize 21 sind so ausgestaltet, dass sie die krankhaft synchrone Aktivität der Neuronenpopulation 30 unterdrücken. Eine Unterdrückung der synchronen Aktivität kann bedeuten, dass die Koinzidenzrate der Neuronen gesenkt wird oder dass die Neuronenpopulation 30 gar desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen. Da es sich bei den ersten Reizen 21 um therapeutisch wirksame sensorische Reize handelt, werden sie auch als "spezifische" Reize bezeichnet.

Die von der zweiten Stimulationseinheit 12 erzeugten zweiten Reize 22 werden je nach Modalität ebenfalls über die Augen, die Ohren oder die Haut sowie tiefer liegende Gewebe des Patienten aufgenommen und von dort an das Nervensystem weitergeleitet. Die zweiten Reize 22 haben für sich alleine angewandt, d.h. ohne das weiter unten beschriebene Zusammenwirken mit den ersten Reizen 21 in der Lernphase, keine oder kaum eine desynchronisierende oder Koinzidenzraten-senkende Wirkung auf die krankhaft synchrone neuronale Aktivität der Neuronenpopulation 30. Die von der zweiten Stimulationseinheit 12 applizierten zweiten Reize 22 werden daher auch als "unspezifische" Reize bezeichnet.

Bei der Applikation von optischen (bzw. visuellen) oder akustischen (bzw. auditorischen) ersten oder zweiten Reizen 21, 22 werden diese über mindestens ein Auge bzw. mindestens ein Ohr des Patienten aufgenommen. Die taktilen, vibratorischen und thermischen ersten oder zweiten Reize 21, 22 (bzw. Tast-, Vibrations- und Thermoreize) werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen Reize 21, 22 wirken. Die vibratorischen Reize 21, 22 zielen vorwiegend auf die Tiefensensibilität ab. Die vibratorischen Reize 21, 22 können von in der Haut, den Muskeln, dem Subkutangewebe und/oder den Sehnen des Patienten gelegenen Rezeptoren aufgenommen werden. Als Rezeptoren für die vibratorischen Reize 21, 22 seien beispielhaft die Vater-Pacini-Körperchen genannt, die Vibrationsempfindungen und Beschleunigungen vermitteln. Die thermischen Reize 21, 22 werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer.

Zur Applikation der ersten und zweiten Reize 21, 22 kann die Vorrichtung 100 in zwei verschiedenen Betriebsmodi betrieben werden. Der jeweilige Betriebsmodus kann beispielsweise vorgegeben sein oder kann von der Steuereinheit 10 ausgewählt werden. Die Steuereinheit 10 steuert die beiden Stimulationseinheiten 11 und 12 entsprechend dem ausgewählten Betriebsmodus an.

In einem ersten Betriebsmodus, der auch als Lernphase bezeichnet wird, werden die unspezifischen zweiten Reize 22 zumindest teilweise zeitlich eng gekoppelt an die Applikation der spezifischen ersten Reize 21 dem Patienten verabreicht, d.h. die ersten und zweiten Reize 21, 22 werden im ersten Betriebsmodus zumindest teilweise in Paaren verabreicht. Das Nervensystem des Patienten wird hierdurch konditioniert, d.h. es lernt, auf die unspezifischen zweiten Reize 22 so zu reagieren wie auf die spezifischen ersten Reize 21 (bzw. in etwas abgeschwächter Form), auch wenn die spezifischen ersten Reize 21 nicht appliziert werden. Dies wird ausgenutzt, indem in dem zweiten Betriebsmodus, der eigentlichen Stimulationsphase, die ersten und zweiten Reize 21, 22 nicht immer gepaart verabreicht werden; vielmehr werden zwischen solchen Paaren aus ersten und zweiten Reizen 21, 22 auch unspezifische zweite Reize 22 alleine appliziert. Da die unspezifischen zweiten Reize 22 durch die im ersten Betriebsmodus, d.h. der Lernphase, erzielte Konditionierung des Nervensystems des Patienten auch therapeutische Effekte erzielen, wird der Bedarf an spezifischen ersten Reizen 21 im zweiten Betriebsmodus gesenkt.

Bei einer effizienten Konditionierung kann über längere Zeiträume hinweg nur noch mit den unspezifischen zweiten Reizen 22 stimuliert werden, ohne dass vom Patienten die erste Stimulationseinheit 11, die zur Verabreichung der spezifischen ersten Reize 21 dient, getragen bzw. benutzt werden muss. Im Unterschied zur ersten Stimulationseinheit 11 ist die zweite Stimulationseinheit 12, mit der die unspezifischen zweiten Reize 22 erzeugt werden, in der Regel deutlich komfortabler (siehe z.B. die unten beschriebene Konditionierungsuhr).

Bei der visuellen Stimulation mit spezifischen optischen ersten Reizen 21 werden beispielsweise Transmissionsbrillen verwendet, welche das Gesichtsfeld z.B. zeitweise bzw. partiell abdunkeln, was bei der Verrichtung alltäglicher Tätigkeiten und insbesondere beim Führen eines Fahrzeugs erheblich behindernd bzw. gefährdend sein kann. Ein angenehmer unspezifischer vibratorischer zweiter Reiz 22 ist dahingegen deutlich komfortabler und beispielsweise auch angenehmer als eine stundenlange Applikation von spezifischen Tonsequenzen. Sofern die spezifischen ersten Reize 21 mittels mehrerer Vibrationsaktuatoren appliziert werden, kann das Tragen der Aktuatoren - je nach deren örtlicher Platzierung - gegebenenfalls schwierig sein und selbst bei alltäglichen Verrichtungen sogar störend sein.

Durch den gesteigerten Komfort bei der Durchführung der Therapie kann die Bereitschaft des Patienten zur Durchführung der Therapie (Compliance) und somit der therapeutische Erfolg insgesamt erhöht werden.

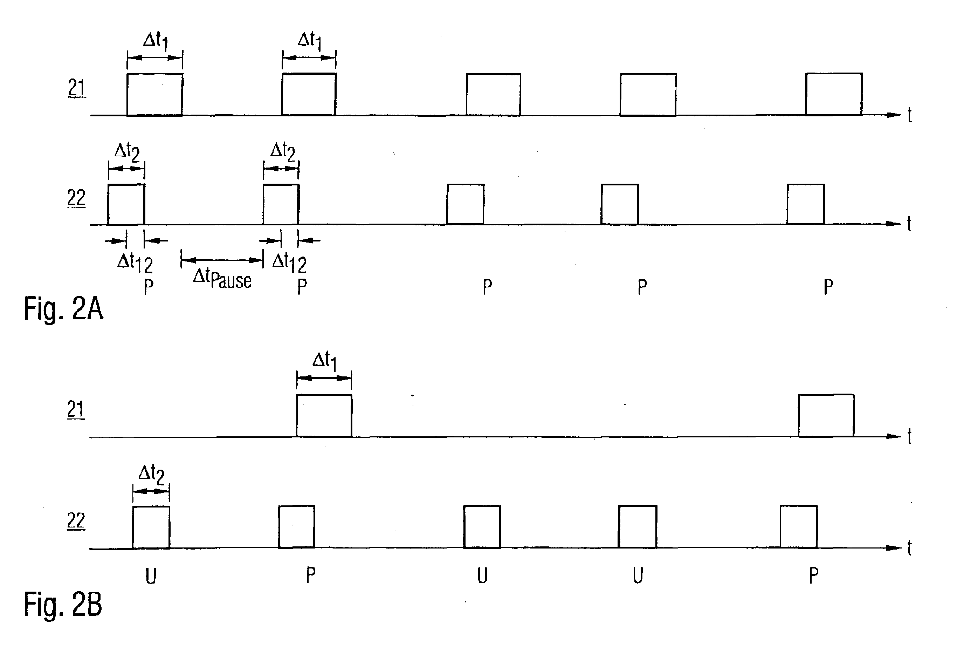

In den Fig. 2A und 2B sind beispielhaft die Unterschiede bei der Applikation der ersten und zweiten Reize 21, 22 in dem ersten und dem zweiten Betriebsmodus graphisch dargestellt. In Fig. 2A sind untereinander erste Zeitabschnitte $\Delta t_1$ und zweite Zeitabschnitte $\Delta t_2$ gegen die Zeit t aufgetragen, während derer die ersten Reize 21 bzw. die zweiten Reize 22 in dem ersten Betriebsmodus erzeugt und dem Patienten verabreicht werden. Die Zeitabschnitte $\Delta t_1$ und $\Delta t_2$ sind jeweils durch Rechtecke dargestellt. Aus Fig. 2A geht hervor, dass in dem ersten Betriebsmodus die Erzeugung und Applikation der unspezifischen zweiten Reize 22 an die Erzeugung und Applikation der spezifischen ersten Reize 21 gekoppelt ist. Die Zeitabschnitte $\Delta t_1$ und $\Delta t_2$ treten in der Lernphase paarweise auf. Durch die gepaarte Applikation der ersten und zweiten Reize 21, 22 wird das Gehirn 29 und/oder Rückenmark 29 des Patienten konditioniert, d.h. nach der Lernphase (z.B. schon nach zwei oder mehr gepaarten Zeitabschnitten $\Delta t_1$ und $\Delta t_2$) ruft auch ein unspezifischer zweiter Reiz 22, der ohne einen zusätzlichen spezifischen ersten Reiz 21 appliziert wird, eine therapeutische Wirkung wie ein spezifischer erster Reiz 21 hervor. Vor dieser Lernphase hätte ein unspezifischer zweiter Reiz 22 keine therapeutische Wirkung hervorgerufen.

Die Dauer der Zeitabschnitte $\Delta t_1$, in denen die spezifischen ersten Reize 21 appliziert werden, beträgt z.B. zwischen 30 Minuten und 6 Stunden, kann aber auch außerhalb dieses Bereichs liegen. Die Dauer der Zeitabschnitte $\Delta t_2$, in denen die unspezifischen zweiten Reize 22 appliziert werden, beträgt z.B. zwischen 10 Minuten und 6 Stunden, kann aber auch außerhalb dieses Bereichs liegen. Beispielsweise überlappen im ersten Betriebsmodus die Zeitabschnitte $\Delta t_1$ mit den jeweils zugehörigen Zeitabschnitten $\Delta t_2$. Dieser Überlapp $\Delta t_{12}$ beträgt z.B. mindestens 10% oder 20% oder 30% oder 40% oder 50% oder 60% oder 70% oder 80% oder mindestens 90% oder gar 100% des jeweiligen Zeitabschnitts $\Delta t_2$. Bei einander zugeordneten Zeitabschnitten $\Delta t_1$ und $\Delta t_2$ kann wie in Fig. 2A dargestellt der Zeitabschnitt $\Delta t_2$ zuerst beginnen, es ist alternativ aber auch möglich, dass mit dem Zeitabschnitt $\Delta t_1$ begonnen wird. Zwischen aufeinander folgenden Paaren von ersten und zweiten Reizen 21, 22 werden Pausen eingehalten, deren Länge $\Delta t_{Pause}$ z.B. zwischen 3 Stunden und 24 Stunden betragen kann. Sowohl die Längen der Zeitabschnitte $\Delta t_1$ und $\Delta t_2$ als auch die Überlappzeiträume $\Delta t_{12}$ sowie die Stimulationspausen $\Delta t_{Pause}$ können während einer Stimulationsphase variiert werden. Die Dauer der Lernphase, d.h. die Dauer, in der die Vorrichtung im ersten Betriebsmodus betrieben wird, kann vorgegeben sein und kann beispielsweise eine vorgegebene Anzahl von gepaarten Zeitabschnitten $\Delta t_1$ und $\Delta t_2$ umfassen.

Im Folgenden werden Beispiele für die Applikation der ersten und zweiten Reize 21, 22 während der Lernphase beschrieben. Gemäß einem Beispiel können während eines Zeitabschnitts $\Delta t_1$ von 6 Stunden und eines Zeitabschnitts $\Delta t_2$ von 6,25 Stunden erste Reize 21 bzw. zweite Reize 22 appliziert werden, wobei der Zeitabschnitt $\Delta t_2$ 15 Minuten vor dem Zeitabschnitt $\Delta t_1$ beginnt und beide Zeitabschnitte $\Delta t_1$ und $\Delta t_2$ somit gleichzeitig enden. Nach einer Pause $\Delta t_{Pause}$ von z.B. 6 Stunden könnte dieser Vorgang wiederholt werden. Um ein zügiges Erlernen bzw. Konditionieren des Nervensystems zu erzielen, könnte die Anzahl der Lernereignisse, d.h. der gepaarten Verabreichung von ersten und zweiten Reizen 21, 22, gegenüber dem vorstehenden Beispiel noch weiter erhöht werden. So könnten die Zeitabschnitte $\Delta t_1$ und $\Delta t_2$ z.B. auf 3 bzw. 3,125 Stunden reduziert werden, wobei der Zeitabschnitt $\Delta t_2$ 7,5 Minuten vor dem Zeitabschnitt $\Delta t_1$ beginnt. Nach einer Pause $\Delta t_{Pause}$ von z.B. 3 Stunden könnte die gekoppelte Stimulation erneut durchgeführt werden.

Ein Lerneffekt kann sich eventuell schon nach zwei Applikationen von miteinander gekoppelten ersten und zweiten Reizen 21, 22 einstellen. Um die Konditionierung des Nervensystems möglichst robust zu gestalten und die Konditionierung in der eigentlichen Stimulationsphase möglichst lange ausnutzen zu können, können z.B. 10 bis 50 Paarapplikationen in der Lernphase, d.h. dem ersten Betriebsmodus, durchgeführt werden.

Während der Lernphase muss nicht notwendigerweise jeder Zeitabschnitt $\Delta t_2$ einem der Zeitabschnitte $\Delta t_1$ zugeordnet sein. Beispielsweise kann nach einer bestimmten Anzahl von miteinander gekoppelten Zeitabschnitten $\Delta t_1$ und $\Delta t_2$ ein Zeitabschnitt $\Delta t_1$ oder $\Delta t_2$ eingefügt werden, der nicht an einen zugehörigen Zeitabschnitt $\Delta t_2$ bzw. $\Delta t_1$ gekoppelt ist und währenddessen lediglich erste Reize 21 bzw. zweite Reize 22 erzeugt und appliziert werden. Beispielsweise können im ersten Betriebsmodus mindestens 50% oder 60% oder 70% oder 80% oder 90% oder gar 100% der Zeitabschnitte $\Delta t_2$ an einen zugehörigen Zeitabschnitt $\Delta t_1$ gekoppelt sein. Ferner können im ersten Betriebsmodus mindestens 50% oder 60% oder 70% oder 80% oder 90% oder gar 100% der Zeitabschnitte $\Delta t_1$ an einen zugehörigen Zeitabschnitt $\Delta t_2$ gekoppelt sein.

Anschließend an die im ersten Betriebsmodus durchgeführte Lernphase erfolgt die eigentliche Stimulationsphase. Dazu schaltet die Steuereinheit 10 in den zweiten Betriebsmodus. Beispielhaft sind in Fig. 2B untereinander die Zeitabschnitte $\Delta t_1$ und $\Delta t_2$ gegen die Zeit t aufgetragen, während derer die ersten Reize 21 bzw. die zweite Reize 22 im zweiten Betriebsmodus erzeugt und appliziert werden.

In der eigentlichen Stimulationsphase wird der Umstand ausgenutzt, dass unspezifische zweite Reize 22 aufgrund der in der Lernphase erzielten Konditionierung des Nervensystems des Patienten auch therapeutische Wirkung haben. Hierzu werden - anders als in der Lernphase - nicht hauptsächlich Paare, bestehend aus ersten und zweiten Reizen 21 und 22, appliziert, vielmehr werden auch immer wieder während eines Zeitabschnitts $\Delta t_2$ nur zweite Reize 22 appliziert, die nicht an die Applikation eines ersten Reizes 21 gekoppelt sind. Beispielsweise ist im zweiten Betriebsmodus mindestens 10% oder 20% oder 30% oder 40% oder 50% oder 60% oder 70% oder 80% oder 90% der Zeitabschnitte $\Delta t_2$ kein Zeitabschnitt $\Delta t_1$ zugeordnet, d.h. insgesamt ist die Zahl der Zeitabschnitte $\Delta t_1$ im zweiten Betriebsmodus kleiner als die Zahl der zweiten Zeitabschnitte $\Delta t_2$. Vereinzelt können im zweiten Betriebsmodus gemäß einer Ausführung auch Zeitabschnitte $\Delta t_1$ eingefügt werden, die nicht an einen Zeitabschnitt $\Delta t_2$ gekoppelt sind. Gemäß einer weiteren Ausführung kann z.B. auch vorgesehen sein, dass im zweiten Betriebsmodus sämtlichen Zeitabschnitten $\Delta t_2$ kein Zeitabschnitt $\Delta t_1$ zugeordnet ist und dass keine ersten Reize 21 appliziert werden.

Die Paare "P" bestehend aus spezifischen und unspezifischen Reizen 21, 22 und die allein applizierten unspezifischen Reize "U" können im zweiten Betriebsmodus z.B. in periodischen Abfolgen verabreicht werden, z.B. in folgender Reihenfolge: P-P-U-U-U-P-P-U-U-U-P-P-U-U-U-... Das zeitliche Muster, nach dem die unspezifischen zweiten Reize 22 alleine auftreten, kann aber auch deterministisch oder stochastisch oder gemischt deterministisch-stochastisch sein, z.B. kann folgende Reihenfolge gewählt werden: P-P-U-U-U-P-U-U-U-U-U-P-P-U-U-U-P-P-U-U-P-P-U-U-U-U-U-P-P-U-U-U-...

Bei der Applikation von Paaren "P" von ersten und zweiten Reizen 21, 22 sind die ersten und zweiten Reize 21, 22 gemäß einer Ausführungsform von unterschiedlicher Modalität, d.h. beispielsweise sind die ersten Reize 21 akustische Reize und die zweiten Reize 22 vibratorische Reize. Gemäß einer weiteren Ausführungsform haben die als Paar "P" applizierten ersten und zweiten Reize 21, 22 die gleiche Modalität.

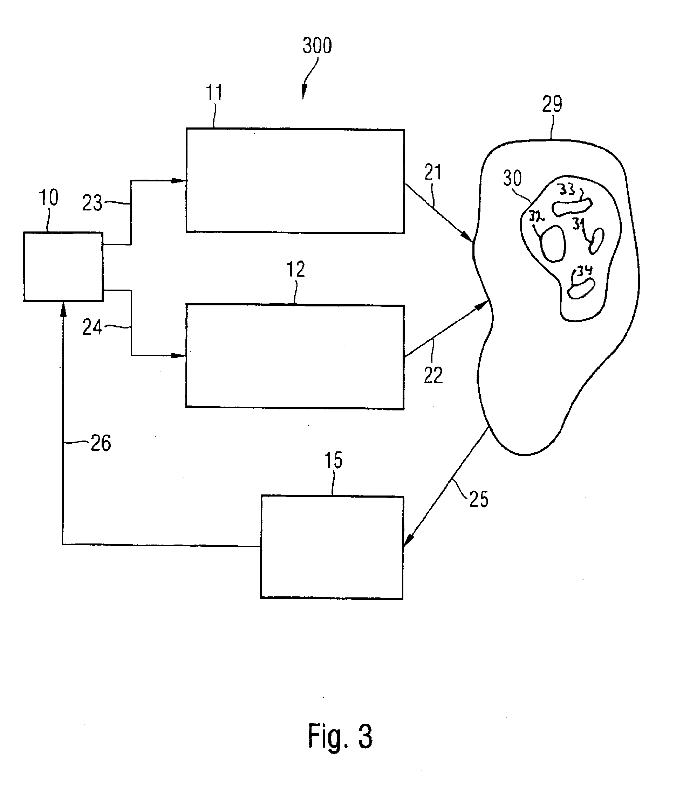

Der mittels der Vorrichtung 100 erzielte Stimulationseffekt kann beispielsweise mit Hilfe einer Messeinheit kontrolliert werden. Eine Vorrichtung 300, die eine solche Messeinheit 15 enthält, ist schematisch in Fig. 3 dargestellt. Die übrigen Komponenten der Vorrichtung 300 sind identisch zu denen der in Fig. 1 gezeigten Vorrichtung 100. Die Messeinheit 15 nimmt ein oder mehrere am Patienten gemessene Messsignale 25 auf, wandelt diese gegebenenfalls in elektrische Signale 26 um und führt diese der Steuereinheit 10 zu. Insbesondere kann mittels der Messeinheit 15 die neuronale Aktivität in dem stimulierten Zielgebiet, d.h. z.B. die neuronale Aktivität der in Fig. 3 schematisch dargestellten Neuronenpopulation 30, oder einem mit der Neuronenpopulation 30 verbundenen Gebiet gemessen werden.

Die Messeinheit 15 kann in Form eines oder mehrerer Sensoren in den Körper des Patienten implantiert sein.

Als invasive Sensoren können beispielsweise Tiefenhirnelektroden, sub- oder epidurale Hirnelektroden, subkutane EEG-Elektroden und sub- oder epidurale Rückenmarkselektroden dienen. Des Weiteren können an peripheren Nerven zu befestigende Elektroden als Sensoren eingesetzt werden.

Die Messsignale 25 können dauerhaft oder in den Pausen zwischen der Verabreichung der spezifischen ersten Reize 21, aber insbesondere auch während bzw. nach der ausschließlichen Verabreichung der unspezifischen zweiten Reize 22 aufgenommen werden. Sofern die neuronale Aktivität der Zielpopulation 30 gemessen wird, kann die Amplitude der krankhaften Schwingungen in typischen Frequenzbereichen der lokalen Feldpotentiale ermittelt werden, also z.B. bei akinetischen Parkinsonpatienten die integrale Leistung im Beta-Frequenzbereich zwischen 10 und 30 Hz. Bei einer effektiven Stimulation sinkt diese Amplitude ab. Lässt die Stimulationswirkung der allein applizierten unspezifischen zweiten Reize 22 im zweiten Betriebsmodus nach und übersteigt die gemessene Amplitude einen vorgegebenen Schwellwert, so kann die nächste Lernphase im ersten Betriebsmodus erfolgen. Danach kann wieder die eigentliche Stimulation im zweiten Betriebsmodus durchgeführt werden.

Der Schwellwert kann vom Arzt individuell für den jeweiligen Patienten eingestellt werden. Alternativ können typische Werte als Voreinstellung für den Schwellwert gewählt werden, z.B. der Mittelwert der Amplitude plus zweimal die Standardabweichung in Bereichen des Frequenzspektrums ohne Frequenzpeaks und oberhalb von z.B. 70 Hz.

Als Alternative zu den invasiven Sensoren oder auch zusätzlich dazu können auch ein oder mehrere nicht-invasive Sensoren eingesetzt werden. Der Vorteil der Verwendung von ausschließlich nicht-invasiven Sensoren ist, dass in diesem Fall kein einziges Bestandteil der Vorrichtung implantiert werden muss. Nicht-invasive Sensoren sind z.B. Elektroenzephalographie (EEG)-Elektroden, Magnetenzephalographie (MEG)-Sensoren und Elektromyographie (EMG)-Elektroden. Ferner kann z.B. über ein Akzelerometer die krankhaft oszillatorische Aktivität im Tremor-Frequenzbereich oder die Bewegungsarmut (im Sinne einer Verminderung der Gesamtbewegungen) gemessen werden. Wird ein vorgegebener Wert der Tremoraktivität überschritten bzw. ein kritischer Wert der mittleren stündlichen Aktivität (außerhalb der Nachtzeiten) unterschritten, so beginnt z.B. die nächste Lernphase im ersten Betriebsmodus.

Gemäß einer Ausführungsform werden zwei Schwellwerte für die Steuerung der beiden Betriebsmodi verwendet. Beispielsweise können zwei Schwellwerte $A_L$ und $A_S$ vorgegeben werden, mit denen die von der Messeinheit 15 z.B. gemessene Amplitude eines Symptoms verglichen wird. Der Schwellwert $A_L$ kann größer als der Schwellwert $A_S$ sein und den gröberen der beiden Schwellwerte darstellen. Übersteigt die Amplitude des Symptoms den Wert $A_L$, so wird von dem zweiten Betriebsmodus in den ersten Betriebsmodus geschaltet und eine erneute Lernphase durchgeführt.

Falls die Amplitude des Symptoms den feineren Schwellwert $A_S$ während des zweiten Betriebsmodus übersteigt, so wird nicht in den ersten Betriebsmodus geschaltet, sondern die Vorrichtung 300 verbleibt in der eigentlichen Stimulationsphase, es werden jedoch vermehrt Paare "P" aus spezifischen ersten Reizen 21 und unspezifischen zweiten Reizen 22 appliziert. Dazu kann beispielsweise eine nur aus unspezifischen Reizen "U" bestehende Teilfolge (-U-U-U-U-U-) übersprungen werden und es wird zu dem in der Sequenz nächsten Abschnitt gesprungen, der Paare "P" aus spezifischen und unspezifischen Reizen 21, 22 aufweist. Sofern in dem zweiten Betriebsmodus beispielsweise vorgegeben ist, dass ein bestimmter Prozentsatz der zweiten Reize 22 zusammen mit ersten Reizen 21 appliziert wird, so kann dieser Prozentsatz der Paare "P" beim Überschreiten des Schwellwerts $A_S$ um eine bestimmte Prozentzahl erhöht werden. Als Beispiel sei angenommen, dass im zweiten Betriebsmodus 30% der zweiten Reize 22 als Paare "P" zusammen mit ersten Reize 21 appliziert werden. Beim Überschreiten des Schwellwerts $A_S$ kann dieser Prozentsatz beispielsweise um 20% auf 50% erhöht werden. Sobald die gemessene Amplitude des Symptoms danach wieder einen weiteren vorgegebenen Schwellwert unterschreitet, kann wieder zu den im zweiten Betriebsmodus beispielhaft vorgesehenen 30% zurückgekehrt werden.

Als Messwert, dessen Amplitude mit den Schwellwerten $A_L$ und $A_S$ verglichen wird, kann z.B. die von einem invasiven Sensor gemessene Betaband-Aktivität der Neuronenpopulation 30 herangezogen werden. Bei einem nicht-invasiven Sensors kann beispielsweise die von einem Akzelerometer gemessene mittlere Amplitude der Tremoraktivität als Messwert verwendet werden.

Ferner kann die von dem Akzelerometer gemessene Bewegung des Patienten als Vergleichswert herangezogen werden. In diesem Fall ist der gröbere Schwellwert $A_L$ jedoch kleiner als der Schwellwert $A_S$. Sofern die mittlere Amplitude der Bewegung des Patienten den feineren Schwellwert $A_S$ unterschreitet, werden im zweiten Betriebsmodus vermehrt Paare "P" aus spezifischen ersten Reizen 21 und unspezifischen zweiten Reizen 22 appliziert. Unterschreitet die mittlere Amplitude der Bewegung den Wert $A_L$, so wird von dem zweiten Betriebsmodus in den ersten Betriebsmodus geschaltet und eine erneute Lernphase durchgeführt. Diese Behandlung kann insbesondere bei akinetischen Parkinsonpatienten eingesetzt werden.

Der Übergang vom zweiten in den ersten Betriebsmodus kann auch vom Patienten durch ein externes Patienten-Programmiergerät gesteuert werden. D.h. der Patient hat die Möglichkeit, an einem kleinen, handlichen externen Gerät eine Taste zu drücken, wenn er sich nicht ausreichend therapiert fühlt, wenn also z.B. sein Tremor oder seine Unbeweglichkeit zu stark sind. Nach einem vordefinierten Modus schaltet dann die Steuereinheit 10 vom zweiten Betriebsmodus in den ersten Betriebsmodus, also in eine erneute Lernphase. Der vordefinierte Modus bedeutet hier, dass dieses Umschalten in den ersten Betriebsmodus z.B. schon durch den ersten Tastendruck des Patienten ausgelöst wird. Die Vorrichtung 100 bzw. 300 kann aber auch vom Arzt so eingestellt werden, dass erst nach einer kleineren Anzahl von derartigen Tastendrucken pro vorgegebenem Zeitintervall, z.B. nach 3 Tastendrucken pro halber Stunde, das Umschalten in den ersten Betriebsmodus erfolgt. Ferner können auch bei dieser Ausführungsform zwei Schwellwerte $A_L$ und $A_S$ eingesetzt werden. Falls die Anzahl der Tastendrucke des Patienten innerhalb eines vorgegebenen Zeitintervalls den feineren Schwellwert $A_S$ während des zweiten Betriebsmodus übersteigt, so werden vermehrt Paare "P" aus spezifischen ersten Reizen 21 und unspezifischen zweiten Reizen 22 appliziert. Übersteigt die Anzahl der Tastendrucke den Schwellwert $A_S$, wird in die Lernphase geschaltet.

Zur Therapiekontrolle registriert die Vorrichtung 100 bzw. 300 die Anzahl und die Zeitpunkte der Tastendrucke. Diese Information kann vom Arzt mittels eines für den Arzt bestimmten externen Programmiergeräts ausgelesen werden.

Weiterhin kann vorgesehen sein, dass nach einer vorgegebenen Zeitdauer vom zweiten Betriebsmodus wieder in den ersten Betriebsmodus, also in die Lernphase, gewechselt wird. Für diesen Umschaltmodus ist nicht notwendigerweise eine Therapiekontrolle mit Hilfe der Messeinheit 15 erforderlich, d.h. dieser Umschaltmodus kann sowohl in der Vorrichtung 100 als auch in der Vorrichtung 300 implementiert sein.

Zur Erzeugung der unspezifischen zweiten Reize 22 kann die zweite Stimulationseinheit 12 z.B. einen Lautsprecher, eine Lichtquelle (bzw. Bildquelle), einen Vibrator und/oder ein Thermoelement enthalten. Generell sollen die zweiten Reize 22 stark genug sein, damit sie vom Patienten bewusst wahrgenommen werden. Sie sollen aber beispielsweise weder als unangenehm stark noch störend oder gar ablenkend empfunden werden. Als akustische zweite Reize 22 kommen beispielsweise ein Summton, ein Brummton oder eine Melodie infrage, die während der Zeitabschnitte $\Delta t_2$ von dem Lautsprecher erzeugt werden. Sofern optische Signale als zweite Reize 22 eingesetzt werden sollen, können diese z.B. abstrakte oder gegenständliche Muster sein, die während der Zeitabschnitte $\Delta t_2$ entweder statisch sind oder sich zeitlich verändern, z.B. eine Blüte, die sich im Wind bewegt, ein Fisch, der im Wasser schwimmt, ein Vogel, der fliegt, eine Sonne, die aufgeht, usw. Taktile bzw. vibratorische zweite Reize 22 können Vibrationen mit für den Patienten wahrnehmbaren Frequenzen sein, die während der Zeitabschnitte $\Delta t_2$ von einem mechanischen Vibrator erzeugt werden. Wahrnehmbare Vibrationsreize können Frequenzen im Bereich von 10 bis 160 Hz oder auch darüber aufweisen, während taktile Reize deutlich geringere Frequenzen haben, die z.B. kleiner als 1 Hz sind. Es können auch Mischformen von taktilen und vibratorischen Reizen eingesetzt werden. Die taktilen bzw. vibratorischen zweiten Reize 22 können z.B. von dem Patienten selbst als angenehm ausgewählt werden. Mittels des Vibrators kann ferner während der Zeitabschnitte $\Delta t_2$ auf die Haut des Patienten eine leichte, angenehm massierende Wirkung ausgeübt werden. Als thermische zweite Reize 22 können Wärmereize oder auch Kältereize verwendet werden. Obwohl Kältereize eine bessere zeitliche Auflösung haben (was aber bei der unspezifischen Reizung nicht notwendig ist), sind Wärmereize bevorzugt, da Kältereize vom Patienten (außer im Hochsommer) eher nicht als angenehm empfunden werden.

Die unspezifischen zweiten Reize 22 können vom Anfang bis zum Ende eines jeweiligen Zeitabschnitt $\Delta t_2$ dem Patienten kontinuierlich verabreicht werden. Alternativ können auch Applikationspausen während der Zeitabschnitte $\Delta t_2$ eingehalten werden, beispielsweise können die zweiten Reize 22 in bestimmten Zeitintervallen mit dazwischen liegenden Applikationspausen während der Zeitabschnitte $\Delta t_2$ verabreicht werden. Diese Zeitmuster können auch variiert werden, z.B. stochastisch oder deterministisch oder gemischt stochastischdeterministisch. Es kann vorgesehen sein, dass während mindestens 60% oder 70% oder 80% oder 90% der Zeitdauer eines jeweiligen Zeitabschnitts $\Delta t_2$ die zweiten Reize 22 appliziert werden.

Als spezifische erste Reize 21 werden optische, akustische, taktile, vibratorische und/oder thermische Reize verwendet, die eine desynchronisierende Wirkung haben oder zumindest eine Senkung der Koinzidenzrate der kranken Neuronen bewirken. Weiter unten wird beschrieben, dass es möglich ist, mittels der Stimulationseinheit 11 unterschiedliche Bereiche der Gehirns 29 oder Rückenmarks 29 separat zu stimulieren', indem die applizierten ersten Reize 21 über Nervenleitungen an unterschiedliche Zielgebiete, die im Gehirn 29 und/oder Rückenmark 29 liegen, weitergeleitet werden. Die Zielgebiete können während des Stimulationszeitraums $\Delta t_1$ mit eventuell unterschiedlichen und/oder zeitversetzten ersten Reizen 21 stimuliert werden.

Gemäß einer Ausgestaltung werden der Neuronenpopulation 30, die eine krankhaft synchrone und oszillatorische Aktivität aufweist, erste Reize 21 verabreicht, welche in der Neuronenpopulation 30 ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Neuronenpopulation 30 mittels einer gezielten Stimulation kontrolliert. Da es ferner möglich ist, die krankhafte Neuronenpopulation 30 an unterschiedlichen Stellen zu stimulieren, kann die Phase der neuronalen Aktivität der krankhaften Neuronenpopulation 30 an den unterschiedlichen Stimulationsstellen zu unterschiedlichen Zeitpunkten zurückgesetzt werden. Im Ergebnis wird dadurch die krankhafte Neuronenpopulation 30, deren Neuronen zuvor synchron und mit gleicher Frequenz und Phase aktiv waren, in mehrere Subpopulationen aufgespalten, die in Fig. 3 schematisch dargestellt sind und mit den Bezugszeichen 31, 32, 33 und 34 gekennzeichnet sind (beispielhaft sind hier vier Subpopulationen dargestellt). Innerhalb einer der Subpopulationen 31 bis 34 sind die Neuronen nach einem Zurücksetzen der Phase weiterhin synchron und feuern auch weiterhin mit derselben pathologischen Frequenz, aber jede der Subpopulationen 31 bis 34 weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen 31 bis 34 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 30 nähert sich schnell einem Zustand kompletter Desynchronisation, in welchem die Neuronen unkorreliert feuern. Der gewünschte Zustand, das heißt die komplette Desynchronisation, ist somit nach der Applikation der ersten Reize 21 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 30 in Subpopulationen 31 bis 34 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die elektrische Stimulation mit der Vorrichtung 100 bzw. 300 eine Neuorganisation der Konnektivität der gestörten neuronalen Netzwerke erzielt werden, sodass lang anhaltende therapeutische Effekte bewirkt werden können. Der erzielte synaptische Umbau ist von großer Bedeutung für die wirksame Behandlung neurologischer oder psychiatrischer Erkrankungen.

Stimulationseinheiten zur Erzeugung unspezifischer Reize:

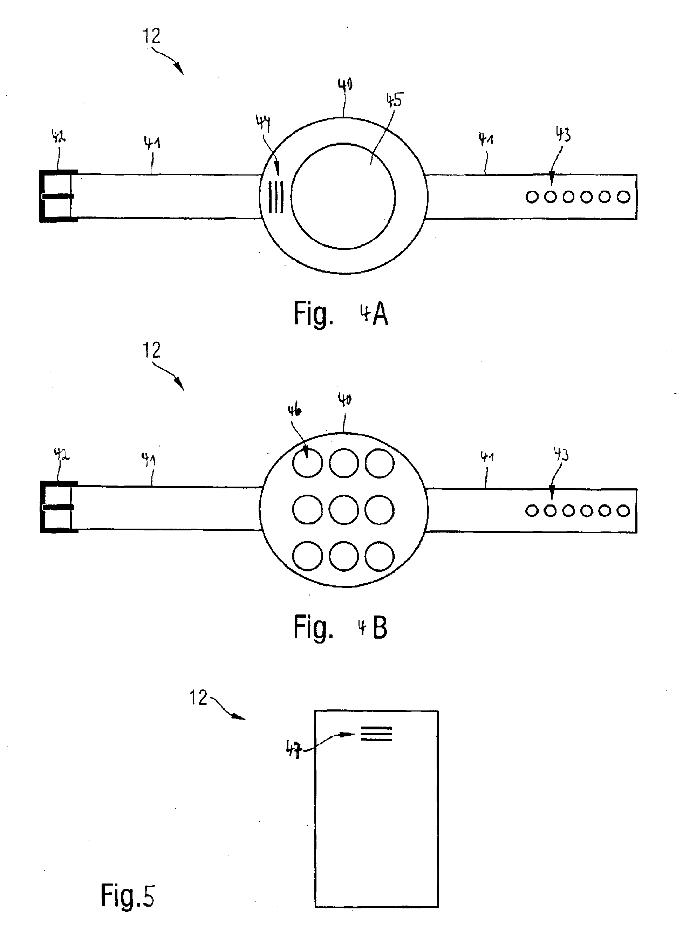

In den Fig. 4A, 4B und 5 sind Ausführungsbeispiele der nicht-invasiven zweiten Stimulationseinheit 12 zur Erzeugung der unspezifischen zweiten Reize 22 dargestellt. In dem in den Fig. 4A und 4B gezeigten Ausführungsbeispiel ist die zweite Stimulationseinheit 12 als sogenannte "Konditionierungsuhr" ausgeführt, die für den Patienten bequem zu tragen ist. In Fig. 4A ist die Vorderansicht, in Fig. 4B die Rückansicht der Konditionierungsuhr 12 gezeigt. Die Konditionierungsuhr 12 besteht aus einem Mittelteil 40, Armbändern 41, einem Verschlussteil 42 und zugehörigen Löchern 43. Alternativ kann auch ein Klettverschluss oder jeder andere gleichwertige Verschluss verwendet werden. Das Mittelteil 40 enthält einen Lautsprecher 44 zur Generierung von unspezifischen akustischen Reizen 22, z.B. einer Melodie oder einem angenehmen Summton, sowie ein Display 45 zur Generierung eines angenehmen, nicht blendenden unspezifischen optischen Reizes 22, z.B. einer sich im Wind bewegenden Blume oder einem abstrakten Lichtmuster mit hellen und warmen Farben. Ferner kann die Konditionierungsuhr 12 mit einem oder mehreren Vibratoren 46 ausgestattet sein, die unspezifische taktile und/oder vibratorische Reize 22 generieren. Zur Erzeugung taktiler Reize 22 können die Vibratoren z.B. mit Frequenzen von kleiner 1 Hz betrieben werden. Insbesondere können die beweglichen Teile der Vibratoren 46 in diesem Fall so ausgerichtet sein, dass sie Druckreize besser realisieren können, d.h. die Hauptbewegungsrichtung der Vibratoren 46 sollte in die Haut hinein gerichtet sein. Ferner könnten die taktilen Reize 22 auch durch Druckaktuatoren oder sich langsam relativ zur Haut bewegende Elemente, die z.B. in die Konditionierungsuhr integriert sein können, erzeugt werden. Sofern mittels der Vibratoren 46 vibratorische Reize 22 erzeugt werden sollen, können Vibrationsfrequenzen im Bereich von 10 bis 160 Hz oder darüber eingesetzt werden. In diesem Fall können die beweglichen Teile der Vibratoren 46 eine ausgeprägte Bewegungsrichtung im Wesentlichen parallel zur Hautoberfläche haben. Bewegungen senkrecht zur Hautoberfläche sind ebenfalls möglich. Die Vibratoren 46 können auch so betrieben werden, dass sie gleichzeitig taktile und vibratorische Reize 22 erzeugen.

Gemäß einer Ausführungsform ist auf der Rückseite der Konditionierungsuhr 12 ein Thermostimulator angeordnet, mit dem thermische zweite Reize 22 der Haut des Patienten verabreicht werden können.

Die Konditionierungsuhr 12 kann auch so ausgestaltet sein, dass sie nur einen unspezifischen Reiz 22 einer Sinnesmodalität, z.B. nur einen optischen Reiz, generiert. Die Stromversorgung der Konditionierungsuhr 12 erfolgt durch eine Batterie und/oder Solarzellen und/oder ein mechanisches Schwungrad im Inneren der Konditionierungsuhr 12.

Zur Kontrolle des Stimulationseffekts kann die Konditionierungsuhr 12 zusätzlich ein Akzelerometer beinhalten, mit dem sich die krankhafte oszillatorische Aktivität, z.B. von krankhaftem Tremor, oder aber das mittlere Aktivitätsniveau des Patienten messen lässt. Das mittlere Aktivitätsniveau des Patienten spiegelt die Verlangsamung bzw. Verarmung der Bewegungen bzw. der Bewegungsunfähigkeit des Patienten wider (d.h. die Brady-, Hypo- und Akinese).

Ein weiteres Ausführungsbeispiel der nicht-invasiven zweiten Stimulationseinheit 12 ist Fig. 5 schematisch dargestellt. Dabei handelt es sich um einen beispielsweise handyförmigen Stimulator, der z.B. in der Hemdtasche oder Hosentasche des Patienten getragen werden kann und der mittels eines Lautsprechers 47 unspezifische akustische Reize 22 generiert.

Ferner kann ein externes Programmiergerät für den Arzt vorgesehen sein, mit welchem die Parameter der Steuereinheit 10, der spezifischen Stimulationseinheit 11 und/oder der unspezifischen, physiologischen Stimulationseinheit 12 eingestellt werden können. Darüber hinaus kann dem Patienten ebenfalls ein externes Programmiergerät zur Verfügung gestellt werden, mit welchem der Patient die Stimulationsgeräte ausstellen kann bzw. Parameter der Stimulationseinheiten 11 und 12 in engen, vom Arzt vorgegebenen Grenzen modifizieren kann. Ferner kann das für den Patienten bestimmte Programmiergerät die weiter oben bereits beschriebene Funktionalität enthalten, mittels welcher der Patient selbstständig, z.B. durch das Betätigen einer Taste, ein Umschalten vom zweiten Betriebsmodus in den ersten Betriebsmodus, also die Lernphase, bewirken kann, wenn er sich nicht ausreichend therapiert fühlt, wenn also z.B. sein Tremor oder seine Unbeweglichkeit zu stark sind. Die Programmiergeräte können beispielsweise über Funkverbindungen mit den jeweiligen Komponenten des Stimulationsgeräts kommunizieren.

Stimulationseinheiten zur Erzeugung spezifischer optischer Reize:

Im Folgenden werden Ausgestaltungen der nicht-invasiven ersten Stimulationseinheit 11 zur Erzeugung optischer erster Reize 21 beschrieben. Derartige Stimulationseinheiten lassen sich auch der deutschen Patentanmeldung Nr. 10 2008 012 669.1 mit dem Titel "Vorrichtung und Verfahren zur visuellen Stimulation" entnehmen, die am 5. März 2008 beim Deutschen Patentund Markenamt hinterlegt worden ist. Der gesamte Offenbarungsgehalt der deutschen Patentanmeldung Nr. 10 2008 012 669.1 wird hiermit in die Offenbarung der vorliegenden Anmeldung aufgenommen.

Im Folgenden wird nur auf die Erzeugung der optischen ersten Reize 21 eingegangen. Es versteht sich, dass diese spezifischen ersten Reize 21 in Kombination mit den unspezifischen zweiten Reizen 22, wie sie oben z.B. im Zusammenhang mit den Fig. 1 bis 5 beschrieben wurden, appliziert werden.

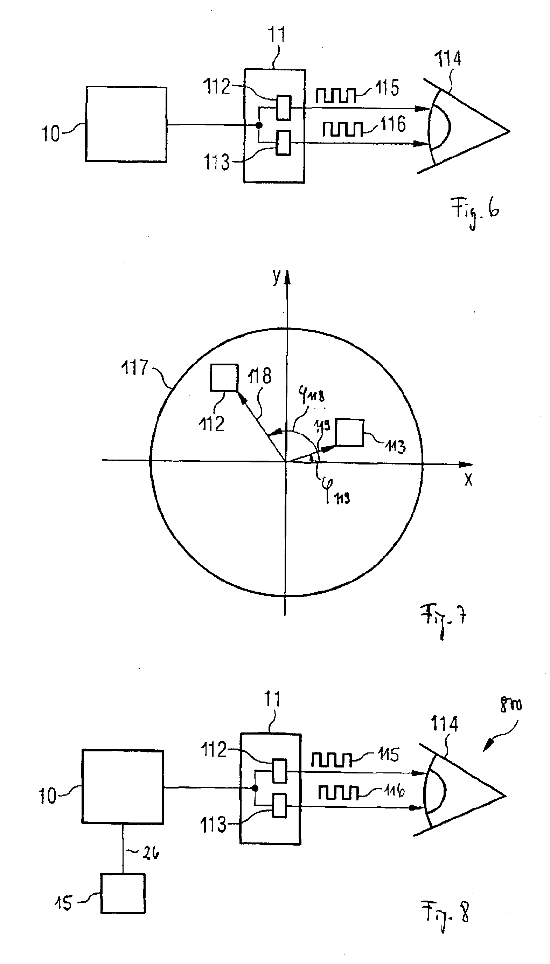

Fig. 6 zeigt schematisch eine Ausgestaltung der ersten Stimulationseinheit 11, die eine Mehrzahl von Stimulationselementen beinhaltet. In dem vorliegenden Ausführungsbeispiel weist die Stimulationseinheit 11 zwei Stimulationselemente 112 und 113 auf, die von der Steuereinheit 10 angesteuert werden. In Fig. 6 ist ferner ein Auge 114 eines Patienten dargestellt.

Während des Betriebs der ersten Stimulationseinheit 11 erzeugen die Stimulationselemente 112 und 113 optische erste Reize 115 bzw. 116, die vom Patienten über ein oder beide Augen 114 aufgenommen werden und über die Sehnerven an Neuronenpopulationen im Gehirn weitergeleitet werden. Die Steuereinheit 10 steuert die Stimulationselemente 112 und 113 dabei derart an, dass die optischen ersten Reize 115 und 116 beispielsweise zeitversetzt generiert werden.

Anstelle einer zeitversetzten Applikation der optischen ersten Reize 115 und 116 können diese auch mit unterschiedlichen Phasen oder unterschiedlichen Polaritäten appliziert werden. Ferner sind auch Mischformen denkbar, d.h. die optischen ersten Reize 115 und 116 können z.B. zeitversetzt sein und unterschiedliche Polaritäten aufweisen. Die erste Stimulationseinheit 11 kann so ausgestaltet sein, dass mit ihr beispielsweise nur eine der vorstehend genannten Stimulationsvarianten ausgeführt werden kann, oder die erste Stimulationseinheit 11 kann alternativ so ausgestaltet sein, dass mit ihr mehrere der genannten Stimulationsvarianten ausgeführt werden können.

Den optischen ersten Reizen 115 und 116 kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die optischen ersten Reize 115 und 116 können je nach Ausgestaltung der ersten Stimulationseinheit 11 als Leuchtstärkenmodulation natürlicher optischer Reize, z.B. mittels einer homogenen oder segmentierten Transmissionsbrille, als zusätzlich zu einem natürlichen optischen Reiz auftretender, modulierter optischer Reiz, z.B. mittels einer partiell durchsichtigen Lichtbrille, oder als künstlicher optischer Helligkeitsreiz, z.B. mittels einer undurchsichtigen Lichtbrille, verabreicht werden. Falls der Patient die optischen ersten Reize 115, 116 über beide Augen 114 aufnimmt, können die jeweiligen optischen ersten Reize 115, 116 beider Augen 114 korreliert bzw. koordiniert werden.

Die von den Stimulationselementen 112, 113 erzeugten optischen ersten Reize 115, 116 sind derart ausgestaltet, dass sie, wenn sie von der Retina aufgenommen werden und über den Sehnerv zu einer Neuronenpopulation mit einer krankhaft synchronen und oszillatorischen Aktivität geleitet werden, in der Neuronenpopulation ein Zurücksetzen der Phase der neuronalen Aktivität der stimulierten Neuronen bewirken.

In Fig. 7 ist schematisch das Gesichtsfeld 117 eines Patienten dargestellt. Als Gesichtsfeld wird der Raum bezeichnet, der mit einem Auge ohne Augenbewegungen überblickt wird. In Fig. 7 ist das Gesichtsfeld 117 zur Vereinfachung kreisförmig dargestellt. Typischerweise hat das Gesichtsfeld eine eher gewölbte ovale Form. Die genaue Größe und Form des Gesichtsfelds unterliegt dabei individuellen Schwankungen und ist zudem altersabhängig.

Punkte im Gesichtsfeld 117 lassen sich beispielsweise mit Hilfe ihrer Polarkoordinaten beschreiben. In Fig. 7 sind die räumlichen Lagen der Stimulationselemente 112 und 113 im Gesichtsfeld 117 beispielhaft dargestellt. Zur Veranschaulichung ist jeweils ein Eckpunkt der Stimulationselemente 112 und 113 mit einem Vektor 118 bzw. 119 gekennzeichnet. Die Vektoren 118 und 119 lassen sich im Polarkoordinatensystem über ihren Betrag und den Winkel $\varphi_{118}$ bzw. $\varphi_{119}$, den sie mit der x-Achse einschließen beschreiben.

Unterschiedliche Stellen im Gesichtsfeld 117 werden über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Dies bedeutet, dass mit den an unterschiedlichen räumlichen Orten angeordneten Stimulationselementen 112 und 113 jeweils unterschiedliche Neuronen stimuliert werden können. Folglich können die Stimulationselemente 112 und 113 sowie evtl. weitere Stimulationselemente räumlich so im Gesichtsfeld 117 des Patienten angeordnet sein, dass die von der Retina aufgenommenen optischen Reize an unterschiedliche Zielgebiete im Gehirn weitergeleitet werden. Es können demnach unterschiedliche Subpopulationen einer krankhaften Neuronenpopulation mit den Stimulationselementen 112 und 113 gezielt stimuliert werden, und es kann ein zeitversetztes Zurücksetzen der Phasen dieser Subpopulation durchgeführt werden, wie oben im Zusammenhang mit Fig. 3 beschrieben worden ist.

Die Zuordnung der Bereiche des Gesichtsfelds zu entsprechenden Bereichen des Gehirns ist beispielsweise in dem Artikel "Visual Field Maps in Human Cortex" von B. A. Wandell, S. O. Dumoulin und A. A. Brewer, erschienen in Neuron 56, Oktober 2007, Seiten 366 bis 383, beschrieben.

Die erste Stimulationseinheit 11 kann beispielsweise in einem sogenannten "open loop"-Modus betrieben werden, bei welchem die Steuereinheit 10 die Stimulationseinheit 11 derart ansteuert, dass die Stimulationselemente 112, 113 vorgegebene optische erste Reize 115, 116 erzeugen. Des Weiteren kann die erste Stimulationseinheit 11 zusammen mit der Steuereinheit auch zu einem in Fig. 8 schematisch dargestellten "closed loop"-System weitergebildet werden. Bei dieser Ausführung ist zusätzlich noch eine Messeinheit 15 vorgesehen, welche am Patienten aufgenommene Messsignale bereitstellt und diese an die Steuereinheit 10 weiterleitet. Bei der Messeinheit 15 kann es sich um nicht-invasive oder invasive Sensoren handeln (vgl. obige Beschreibung im Zusammenhang mit Fig. 3).

Hinsichtlich des Zusammenwirkens der Steuereinheit 10 mit der Messeinheit 15 sind verschiedene Ausgestaltungen denkbar. Beispielsweise kann - wie oben beschrieben wurde - anhand der Messsignale zwischen dem ersten Betriebsmodus, der Lernphase, und dem zweiten Betriebsmodus, der eigentlichen Stimulationsphase, gewechselt werden. Ferner können von der Steuereinheit 10 anhand der Ausprägung der krankhaften Merkmale Parameter der optischen ersten Reize 115, 116, wie beispielsweise die Stärke (Amplitude) der Reize oder die Frequenz der Stimulation oder die Pausen zwischen den Stimulationssequenzen, eingestellt werden.

Des Weiteren kann vorgesehen sein, dass die von der Messeinheit 15 aufgenommenen Messsignale direkt oder gegebenenfalls nach einem oder mehreren Verarbeitungsschritten in optische erste Reize umgesetzt werden und von der ersten Stimulationseinheit 11 appliziert werden. Beispielsweise können die Messsignale verstärkt und gegebenenfalls nach mathematischer Verrechnung (z.B. nach Mischung der Messsignale) mit einer Zeitverzögerung und linearen und/oder nichtlinearen Verrechnungsschritten als Steuersignale in die Steuereingänge der Stimulationselemente 112, 113 eingespeist werden. Der Verrechnungsmodus wird hierbei so gewählt, dass der krankhaften neuronalen Aktivität entgegengewirkt wird und die Stimulationssignale mit abnehmender krankhafter neuronaler Aktivität ebenfalls verschwinden oder zumindest deutlich in ihrer Stärke reduziert werden.

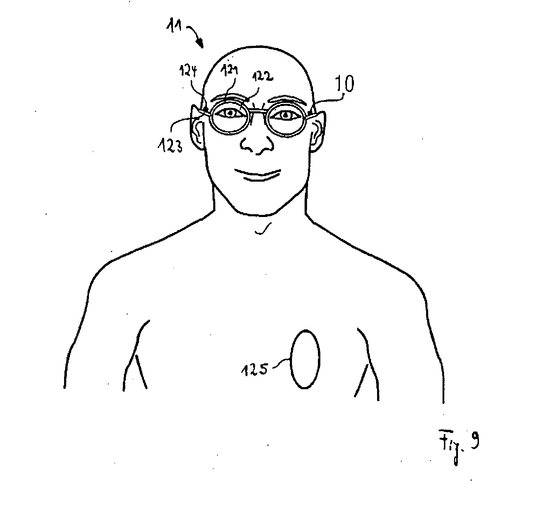

In Fig. 9 ist schematisch eine Ausgestaltung der ersten Stimulationseinheit 11 als Transmissionsbrille gezeigt, die aus den folgenden Komponenten besteht: (i) zwei Einfassungsteilen 121 mit je einem transmissionsmodulierten Brillenglas 122 (für jedes Auge einzeln), (ii) zwei Ohrbügeln 123, mit welchen die Brille hinter dem Ohr des Patienten mechanisch gehalten wird, und (iii) der Steuereinheit 10, welche die Transmission der transmissionsmodulierten Gläser 122 der Brille steuert. Anstelle einer Transmissionsbrille könnte auch eine der weiter unten beschriebenen Brillen, wie z.B. eine partiell durchsichtige oder undurchsichtige Lichtbrille, als Stimulationsbrille verwendet werden. Eine Batterie oder ein Akku zur Stromversorgung der elektrischen Bauelemente können in der Steuereinheit 10 oder auch baulich getrennt von der Steuereinheit 10 in oder an der Brille untergebracht sein. Die Brille kann vom Patienten mittels einer Bedieneinheit 124 (z.B. Anschaltknopf und/oder Drehregler) angeschaltet werden. Mit dem Drehregler kann z.B. die maximale Stimulationsstärke eingestellt werden. Zusätzlich zu den vorstehend genannten Komponenten kann ein Steuermedium 125 vorgesehen sein, welches beispielsweise telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Ferner kann auch ein weiteres, z.B. vom Arzt zu bedienendes Steuermedium (nicht dargestellt) bereitgestellt werden, welches telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Des Weiteren können ein oder mehrere Sensoren, z.B. EEG-Elektroden oder ein Akzelerometer, zur Registrierung und/oder Dokumentation des Stimulationserfolgs und zur Untersuchung durch den Arzt vorgesehen sein.

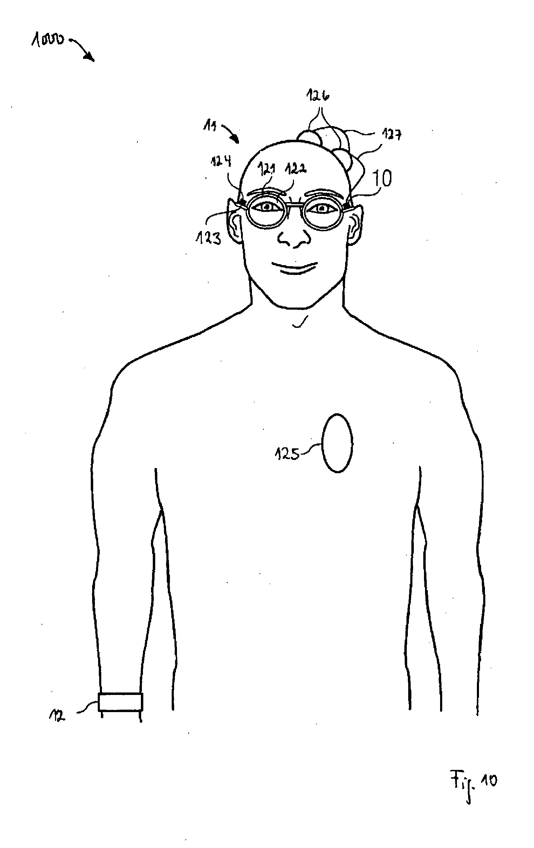

Fig. 10 zeigt schematisch eine Vorrichtung 1000, die eine wie in Fig. 9 ausgestaltete erste Stimulationseinheit 11, eine aus EEG-Elektroden 126 bestehende Messeinheit sowie eine zweite Stimulationseinheit 12 zur Applikation der zweiten, unspezifischen Reize aufweist. Sämtliche Einheiten der Vorrichtung 1000, d.h. die erste und zweite Stimulationseinheit 11, 12 sowie die Messeinheit, sind nicht-invasive Einheiten, die nicht mittels einer Operation im Körper des Patienten implantiert werden müssen. Die EEG-Elektroden 126 sind epikutan, d.h. auf der Haut des Patienten befestigt und über Verbindungskabel 127 mit der Steuereinheit 10 verbunden. Die Steuereinheit 10 verwendet die von den EEG-Elektroden 126 gelieferten Messsignale z.B. zur Einstellung des Betriebsmodus. Die Steuereinheit 10 kann beispielsweise die mittels der EEG-Elektroden 126 gemessene Potentialdifferenz auch verstärken und dieses Signal nach einer optionalen linearen oder nichtlinearen Verrechnung zur Ansteuerung der transmissionsmodulierten Gläser 122 der Transmissionsbrille verwenden. Als Alternative zu den Verbindungskabeln 127 können die EEG-Elektroden 126 auch schnurlos, d.h. telemetrisch mit der Steuereinheit 10 verbunden sein. Dies hat den Vorteil, dass der Patient nicht durch Verbindungskabel behindert wird und z.B. an Hindernissen hängen bleiben kann. Als zweite Stimulationseinheit 12 weist die Vorrichtung 1000 die in Fig. 4A und 4B gezeigte Konditionierungsuhr auf. Die zweiten, unspezifischen Reize können alternativ auch mittels einer anders ausgestalteten zweiten Stimulationseinheit 12 generiert werden.

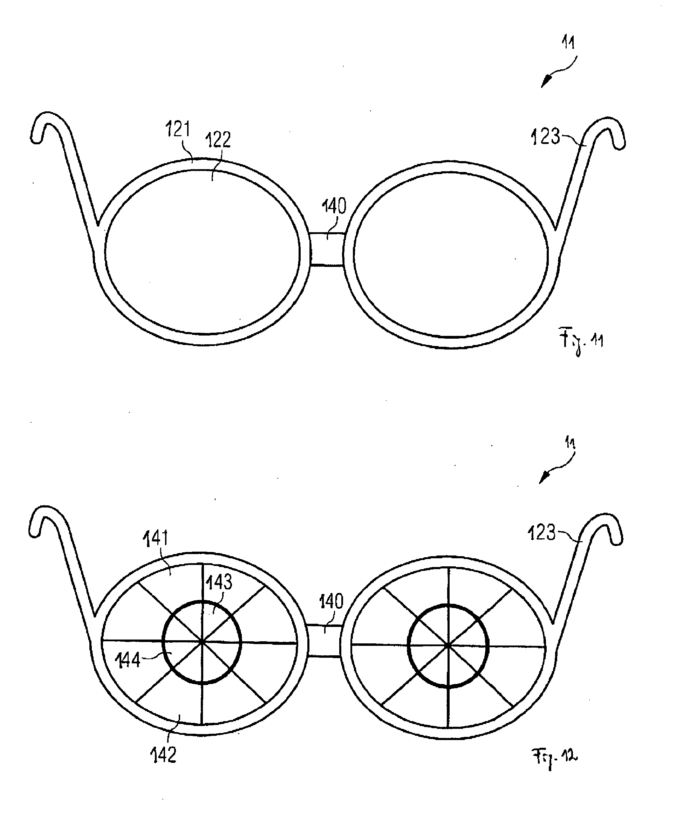

In Fig. 11 ist schematisch eine als erste Stimulationseinheit ausgestaltete Transmissionsbrille 11 mit homogenen Transmissionsgläsern 122 dargestellt. Die Transmissionsbrille 11 umfasst ferner Ohrbügel 123 zur mechanischen Befestigung am Patientenkopf, einen Steg 140, welcher die beiden Transmissionsgläser 122 verbindet, und Einfassungsteile 121, in welche die Transmissionsgläser 122 eingefasst sind. Die Transmissionsgläser 122 sind homogen, d.h. nicht in unterschiedliche Segmente unterteilt. Die Transmission des rechten und des linken Transmissionsglases 122 können separat geregelt werden, d.h. die Transmissionsgläser 122 können als Stimulationselemente 112 und 113 im Sinne der in Fig. 6 dargestellten Ausgestaltung verwendet werden. Mittels der Transmissionsbrille 11 können beide Augen des Patienten mit jeweils unterschiedlichen optischen ersten Reizen stimuliert werden.

In Fig. 12 ist eine Transmissionsbrille 11 mit segmentierten Transmissionsgläsern dargestellt. Die Transmissionsgläser sind jeweils in unterschiedliche Segmente unterteilt, deren Transmission getrennt gesteuert werden kann. Die Segmentierung kann beispielsweise radial und/oder zirkular sein (beides ist in Fig. 12 gezeigt). Die in Fig. 12 gezeigte Ausführung einer segmentierten Transmissionsbrille 11 ist lediglich beispielhaft zu verstehen. Die Anzahl der Segmente sowie die geometrischen Formen der einzelnen Segmente können anders gewählt werden.

Die Segmente der Transmissionsbrille 11 entsprechen den in Fig. 6 gezeigten Stimulationselementen. In Fig. 12 sind beispielhaft vier der Segmente mit den Bezugszeichen 141, 142, 143 und 144 gekennzeichnet.

Anhand der Segmente 141 bis 144 soll nachfolgend beispielhaft erläutert werden, wie durch zeitversetztes Zurücksetzen der Phasen von Subpopulationen einer krankhaft synchronen und oszillatorischen Neuronenpopulation eine Desynchronisation der gesamten Neuronenpopulation erzielt werden kann. Die Segmente 141 bis 144 sind so ausgewählt worden, dass die von ihnen erzeugten optischen ersten Reize jeweils vorzugsweise von einem bestimmten Teil der Netzhaut des Patienten aufgenommen werden, von wo aus die Reize zu bestimmten Bereichen des Gehirns weitergeleitet werden, sodass die oben beschriebene Aufspaltung einer krankhaften Neuronenpopulation in Subpopulationen ermöglicht wird. Damit Subpopulationen mit unterschiedlichen Phasen gebildet werden, können die optischen ersten Reize von den Segmenten 141 bis 144 beispielsweise zeitversetzt erzeugt werden. Gleichbedeutend mit dem zeitversetzten Erzeugen der Reize ist ein phasenversetztes Erzeugen der Reize, welches im Ergebnis ebenfalls zu einem zeitversetzten Zurücksetzen der Phasen der unterschiedlichen Subpopulationen führt.

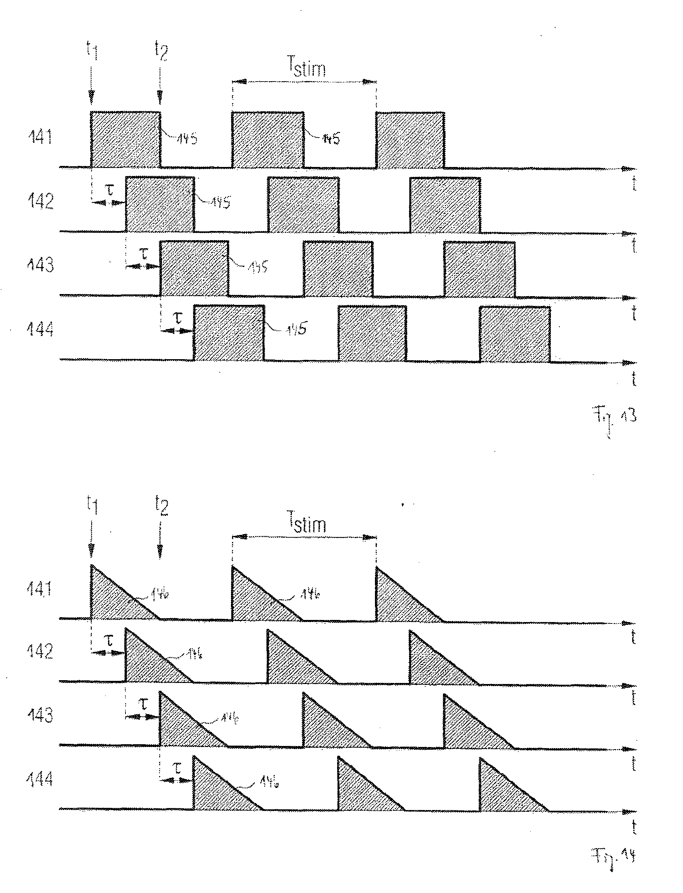

Ein für die oben beschriebenen Zwecke geeignetes Stimulationsverfahren, das beispielsweise mit der vorstehend beschriebenen Transmissionsbrillen 11 durchgeführt werden kann, ist in Fig. 13 schematisch dargestellt. In Fig. 13 sind untereinander die mittels der Segmente 141 bis 144 applizierten optischen ersten Reize 145 gegen die Zeit t aufgetragen. Bei der in Fig. 13 gezeigten Ausführungsform wird davon ausgegangen, dass nur die Segmente 141 bis 144 der Transmissionsbrille 11 optische erste Reize 145 erzeugen, d.h. nur die Transmission dieser Segmente wird von der Steuereinheit 10 moduliert. Selbstverständlich ist dies nur beispielhaft zu verstehen. Bei alternativen Ausgestaltungen können anstelle der Segmente 141 bis 144 andere Segmente zum Generieren der optischen Reize herangezogen werden. Es ist möglich, wie in Fig. 13 nur eine Auswahl der Segmente der Transmissionsbrille 11 zur Stimulation zu verwenden oder auch sämtliche Segmente.

Bei dem in Fig. 13 dargestellten Verfahren appliziert jedes der Segmente 141 bis 144 periodisch den optischen ersten Reiz 145. Pro Segment 141 bis 144 wird der Reiz 145 in dem vorliegenden Beispiel dreimal appliziert. Alternativ könnte der Reiz 145 pro Sequenz beispielsweise auch ein- bis fünfzehnmal wiederholt werden. Die Frequenz $f_{stim}$ = $1/T_{stim}$, mit welcher die Reize 145 pro Segment 141 bis 144 wiederholt werden, kann im Bereich von 1 bis 30 Hz und insbesondere im Bereich von 5 bis 20 Hz liegen, kann aber auch kleinere oder größere Werte annehmen. Derartige Sequenzen von optischen Reizen sind geeignet, die neuronale Phase einer stimulierten krankhaften Subpopulation von Neuronen zurückzusetzen.

Die Frequenz $f_{stim}$ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz, kann aber auch außerhalb dieses Bereichs liegen. Hierbei ist zu beachten, dass die Frequenz, mit welcher die krankhaften Neuronen synchron feuern, üblicherweise nicht konstant ist, sondern durchaus Variationen aufweisen kann und darüber hinaus bei jedem Patienten individuelle Abweichungen zeigt.

Zur Ermittlung der Frequenz $f_{stim}$ kann beispielsweise die mittlere Peakfrequenz der krankhaften rhythmischen Aktivität des Patienten bestimmt werden. Diese Peakfrequenz kann dann als Stimulationsfrequenz $f_{stim}$ verwendet werden oder auch variiert werden, beispielsweise in einem Bereich von $f_{stim}$ - 3 Hz bis $f_{stim}$ + 3 Hz. Alternativ kann aber auch ohne vorherige Messung eine Frequenz $f_{stim}$ im Bereich von 1 bis 30 Hz gewählt werden und diese beispielsweise während der Stimulation variiert werden, bis die Frequenz $f_{stim}$ gefunden wird, mit der sich die besten Stimulationserfolge erzielen lassen. Als weitere Alternative kann für die Stimulationsfrequenz $f_{stim}$ ein für die jeweilige Krankheit bekannter Literaturwert herangezogen werden. Eventuell kann dieser Wert noch variiert werden, bis beispielsweise optimale Stimulationsergebnisse erzielt werden.

Die Struktur eines einzelnen optischen ersten Reizes 145 soll nachfolgend anhand des ersten von dem Segment 141 generierten Reizes 145 erläutert werden. Hier wird zum Zeitpunkt $t_1$ das Segment 141 von der Steuereinheit 10 derart angesteuert, dass die Transmission, d.h. die Lichtdurchlässigkeit des Segments 141 minimal wird. Zum Zeitpunkt $t_2$ schaltet die Steuereinheit 10 die Transmission des Segments 141 auf den maximalen Wert. In anderen Worten bedeutet dies, dass das Segment 141 weniger transparent wird, wenn stimuliert wird. Dementsprechend nimmt der Patient eine verringerte Helligkeit des Umgebungslichts im Bereich des Segments 141 während der Stimulation wahr.

Alternativ ist es auch möglich, die Transmission des Segments 141 zum Zeitpunkt $t_1$ maximal zu schalten und zum Zeitpunkt $t_2$ minimal, sodass das Segment 141 während der Stimulation stärker transparent wird.

Grundsätzlich ist es denkbar, als maximale Transmission 100% zu wählen, d.h. in diesem Fall wird das Umgebungslicht durch das jeweilige Segment überhaupt nicht abgeschwächt. Eine derart hohe Transmission lässt sich jedoch aufgrund technischer Beschränkungen häufig nicht erreichen, sodass kleinere Transmissionswerte für die maximale Transmission im Bereich von 60% bis 100% gewählt werden können. Die minimale Transmission kann einen Wert in dem Bereich von 0% bis 30% annehmen. Es können aber auch noch Stimulationserfolge mit Transmissionswerten, die außerhalb der angegebenen Bereiche liegen, erzielt werden.

Die Dauer eines optischen ersten Reizes 145, d.h. die Zeitspanne zwischen den Zeitpunkten $t_1$ und $t_2$, kann beispielsweise $T_{stim}/2$ betragen. In diesem Fall sind die Zeitspanne, während der stimuliert wird, und die nachfolgende Stimulationspause gleich lang. Es ist aber auch möglich andere Stimulationsdauern zu wählen, beispielsweise im Bereich von $T_{stim}/2 - T_{stim}/10$ bis $T_{stim}/2 + T_{stim}/10$. Auch andere Stimulationsdauern sind möglich und können beispielsweise experimentell bestimmt werden.

Gemäß der in Fig. 13 gezeigten Ausgestaltung erfolgt die Verabreichung der optischen ersten Reize 145 über die einzelnen Segmente 141 bis 144 der Transmissionsbrille 11 mit einer zeitlichen Verzögerung zwischen den einzelnen Segmenten 141 bis 144. Beispielsweise kann der Beginn zeitlich aufeinander folgender und von unterschiedlichen Segmenten 141 bis 144 applizierten Reizen 145 um eine Zeit $\tau$ verschoben sein.

Im Fall von N Stimulationselementen bzw. Segmenten, die zur Stimulation eingesetzt werden, kann die zeitliche Verzögerung $\tau$ zwischen jeweils zwei aufeinander folgenden Reizen 145 beispielsweise im Bereich eines N-tels der Periode $T_{stim} = 1/f_{stim}$ liegen. In dem in Fig. 13 gezeigten Ausführungsbeispiel (N = 4) beträgt die zeitliche Verzögerung $\tau$ dementsprechend $T_{stim}/4$. Von der Vorgabe, dass die zeitliche Verzögerung $\tau$ zwischen jeweils zwei aufeinander folgenden Reizen 145 $T_{stim}/N$ beträgt, kann bis zu einem gewissen Grad abgewichen werden. Beispielsweise kann von dem Wert $T_{stim}/N$ für die zeitliche Verzögerung $\tau$ um bis zu $\pm 10\%$, $\pm 20\%$ oder $\pm 30\%$ abgewichen werden. Bei derartigen Abweichung wurden noch Stimulationserfolge erzielt, d.h. es konnte noch ein desynchronisierender Effekt beobachtet werden.

Die in Fig. 13 dargestellte Rechteckform der Einzelpulse 145 stellt eine ideale Form dar. Je nach der Güte der die Einzelpulse 145 erzeugenden Elektronik und der Transmissionsgläser 122 wird von der idealen Rechteckform abgewichen. Es können aber auch - z.B. je nach Grunderkrankung des Patienten sowie individueller psycho-physischer Beschaffenheit, z.B. Blendempfindlichkeit - Reize mit weniger scharfen Flanken, also glatteren Verläufen verwendet werden.

Anstelle von rechteckförmigen Reize 145 kann die Steuereinheit 10 beispielsweise auch anders ausgestaltete optische erste Reize erzeugen, wie sie beispielhaft in den Fig. 14 bis 16 dargestellt sind. In Fig. 14 sind dreieckförmige optische erste Reize 146 gezeigt. Zum Zeitpunkt $t_1$ wird beispielsweise auf minimale Transmission geschaltet und bis zum Zeitpunkt $t_2$ steigt die Transmission kontinuierlich auf den maximalen Wert an. Alternativ kann vorgesehen sein, dass die Transmission zu Beginn des Reizes 146 maximal ist und anschließend auf den minimalen Wert fällt.

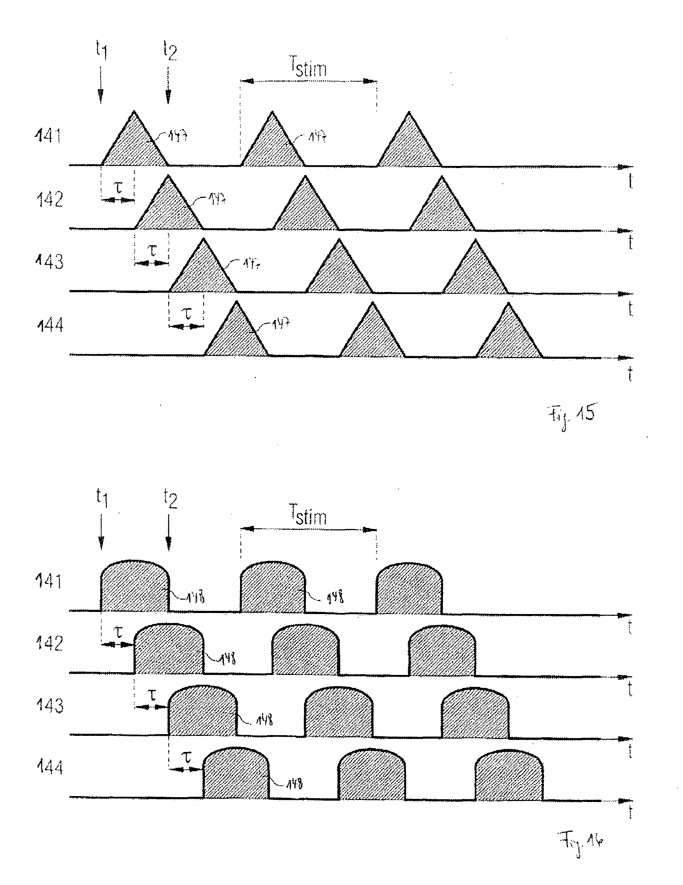

In Fig. 15 sind dreieckförmige optische erste Reize 147 mit einer ansteigenden und einer abfallenden Flanke gezeigt. Beginnend mit dem Zeitpunkt $t_1$ wird hier die Transmission beispielsweise erhöht und nach Erreichen des Maximums bis zum Zeitpunkt $t_2$ wieder erniedrigt.

Ferner kann vorgesehen sein, dass die ansteigenden und abfallenden Flanken der Reize (z.B. exponentiell) "abgerundet" sind. Dies ist in Fig. 16 anhand abgerundeter rechteckförmiger optischer erster Reize 148 gezeigt. Darüber hinaus können die Reize auch durch eine einfache Sinusform ersetzt werden.

Die oben beschriebenen Signalformen und deren Parameter sind nur beispielhaft zu verstehen. Es ist durchaus möglich, von den oben angegebenen Signalformen und deren Parametern abzuweichen.

Von dem in den Fig. 13 bis 16 gezeigten streng periodischen Stimulationsmuster kann auf unterschiedliche Art und Weise abgewichen werden. Beispielsweise braucht die zeitliche Verzögerung $\tau$ zwischen zwei aufeinander folgenden Reizen 145, 146, 147 bzw. 148 nicht notwendigerweise stets gleich groß zu sein. Es kann vorgesehen sein, dass die zeitlichen Abstände zwischen den einzelnen Reizen 145, 146, 147 bzw. 148 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

Des Weiteren können während der Applikation der Reize 145, 146, 147 bzw. 148 Pausen vorgesehen werden, während derer keine Stimulation erfolgt. Die Pausen können beliebig lang gewählt werden und insbesondere ein ganzzahliges Vielfaches der Periode $T_{stim}$ betragen. Die Pausen können nach einer beliebigen Anzahl von Stimulationen eingehalten werden. Z.B. kann eine Stimulation während N aufeinander folgender Perioden der Länge $T_{stim}$ durchgeführt werden und anschließend eine Stimulationspause während M Perioden der Länge $T_{stim}$ eingehalten werden, wobei N und M kleine ganze Zahlen sind, z.B. im Bereich von 1 bis 15. Dieses Schema kann entweder periodisch fortgesetzt werden oder stochastisch und/oder deterministisch, z.B. chaotisch, modifiziert werden.

Eine weitere Möglichkeit, von dem in den Fig. 13 bis 16 gezeigten streng periodischen Stimulationsmuster abzuweichen, besteht darin, die zeitlichen Abstände zwischen aufeinander folgenden Reizen 145, 146, 147 bzw. 148 pro Segment 141 bis 144 stochastisch oder deterministisch oder gemischt stochastisch-deterministisch zu variieren.

Des Weiteren kann pro Periode $T_{stim}$ (oder in anderen Zeitschritten) die Reihenfolge, in welcher die Segmente 141 bis 144 die Reize 145, 146, 147 bzw. 148 applizieren, variiert werden. Diese Variation kann stochastisch oder deterministisch oder gemischt stochastisch-deterministisch erfolgen.

Außerdem kann bei dem Stimulationsmuster, bei dem N Stimulationsperioden von M Perioden Pause gefolgt werden und als Zyklus wiederholt werden, innerhalb N zusammengehöriger Stimulationsperioden dieselbe Reihenfolge der Segmente 141 bis 144 gewählt werden, welche aber zwischen unterschiedlichen Blöcken mit N Stimulationsperioden variiert wird. Diese Variation kann stochastisch oder deterministisch oder gemischt stochastisch-deterministisch erfolgen.

Ferner kann pro Periode $T_{stim}$ (oder in einem anderen Zeitintervall) nur eine bestimmte Anzahl von den Segmenten 141 bis 144 zur Stimulation herangezogen werden und die an der Stimulation beteiligten Segmente können in jedem Zeitintervall variiert werden. Auch diese Variation kann stochastisch oder deterministisch oder gemischt stochastisch-deterministisch erfolgen.

Anstelle der in den Fig. 13 bis 16 gezeigten pulsförmigen und gegeneinander zeitverschobenen Reize 145 bis 148 können auch optische Reize mit anderen Signalformen eingesetzt werden. Beispielsweise kann jedes der Segmente 141 bis 144 ein (z.B. kontinuierliches) Sinussignal erzeugen, wobei die Phasen der von unterschiedlichen Segmenten 141 bis 144 erzeugten Sinussignale gegeneinander verschoben sind. Die mittlere Frequenz der Sinussignale kann dabei gleich sein. Die Phasenverschiebungen zwischen den einzelnen Sinussignalen können entweder vorgegeben sein, z.B. kann die Phasenverschiebung zwischen jeweils zwei von N Stimulationssignalen $2\pi/N$ betragen, was einem Zeitversatz von $T_{stim}/N$ entspricht, oder die Phasenverschiebungen können z.B. chaotisch und/oder stochastisch variiert werden. Ferner können die optischen Reize unterschiedliche Polaritäten aufweisen. Im Falle eines Sinussignals als optischer Reiz kann beispielsweise das Sinussignal von zwei Segmenten zeitgleich, aber mit umgekehrter Polarität appliziert werden (entspricht einer Phasenverschiebung von $\pi$).

Des Weiteren ist es möglich, dass jedes der Segmente 141 bis 144 ein Sinussignal mit jeweils unterschiedlicher Frequenz appliziert. Beispielsweise kann eines der Segmente ein Sinussignal mit 5 Hz und die anderen drei Segmente können Sinussignale mit 4 Hz, 3 Hz bzw. 2 Hz applizieren (d.h. im Falle einer Transmissionsbrille ändert sich die Transmission des jeweiligen Segments 141 bis 144 mit der entsprechenden Frequenz). Anstelle von Sinussignalen können auch andere (oszillierende) Signalformen, z.B. Rechtecksignale, mit der entsprechenden Grundfrequenz verwendet werden. Die Signale brauchen nicht zeitversetzt appliziert werden, sondern die Segmente 141 bis 144 können die optischen Reize beispielsweise auch gleichzeitig erzeugen. Die optischen Reize können kontinuierlich über einen längeren Zeitraum hinweg appliziert werden, es können aber auch Pausen während der Applikation eingehalten werden.

Die Applikation von optischen Reizen mit unterschiedlichen Frequenzen führt nicht notwendigerweise zu einem raschen Zurücksetzen der Phase der neuronalen Aktivität in den jeweiligen stimulierten Subpopulationen, jedoch wird durch die Stimulation mit diesen Signalen den jeweils stimulierten Subpopulationen über einen gewissen Zeitraum hinweg eine bestimmte, von der jeweiligen Stimulationsfrequenz abhängige Phase aufgezwungen. Letztlich führt auch dies zu einer Desynchronisation der gesamten Neuronenpopulation.

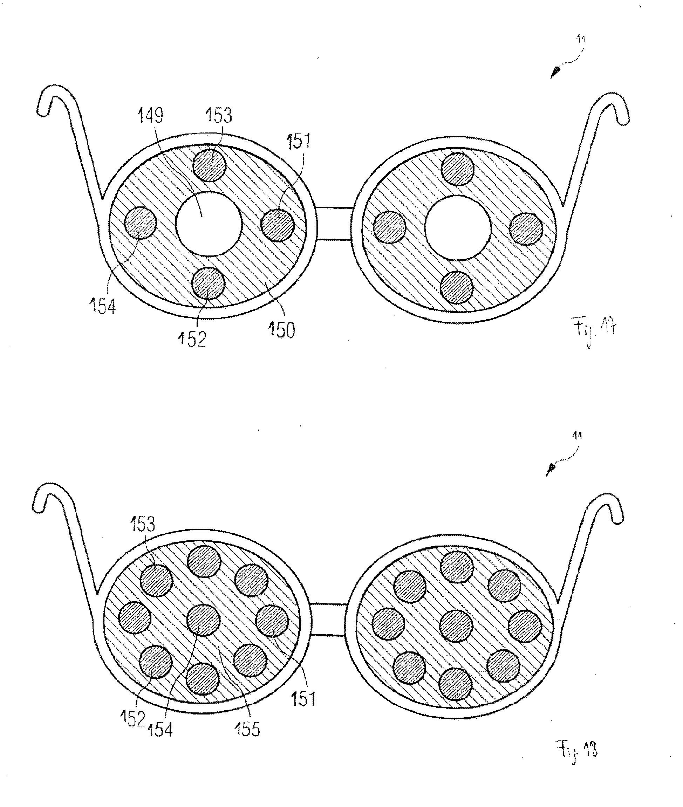

In Fig. 17 ist als weitere Ausführungsform der ersten Stimulationseinheit eine partiell durchsichtige Lichtbrille 11 schematisch dargestellt. Bei der partiell durchsichtigen Lichtbrille 11 wird kein Glas verwendet, dessen Transmission variiert werden kann. Vielmehr ist nur ein Teil 149 jedes der Brillengläser durchsichtig, während der übrige Teil 150 der Brillengläser undurchsichtig ist. An mindestens einem Ort ist pro Brillenglas eine Lichtquelle angeordnet. Die Lichtquelle kann z.B. eine Leuchtdiode oder ein Glasfaserkabel sein, das z.B. das Licht einer an anderer Stelle befestigten Leuchtdiode oder eines anderen Leuchtmittels zu dieser Stelle am Brillenglas weiterleitet. Die in Fig. 17 gezeigte Lichtbrille 11 verfügt pro Brillenglas über vier Lichtquellen 151, 152, 153 und 154. Die Lichtbrille 11 kann aber auch über jede andere Anzahl von Lichtquellen verfügen, die in einer beliebigen Geometrie angeordnet sein können. Ferner kann auch der durchsichtige Teil 149 anders ausgestaltet sein als in Fig. 17 dargestellt.

Der Patient kann nur durch den durchsichtigen Teil 149 der Brillengläser schauen. Wenn dieser Teil im Vergleich zum gesamten Brillenglas klein ist, wird der Patient gezwungen, seine Augen relativ zur Brille konstant positioniert zu halten. Die Lichtquellen 151 bis 154 reizen nur die Netzhaut des Patienten, während sie einen Betrachter auf der anderen Seite der Brille nicht visuell stimulieren. Die unterschiedlichen Lichtquellen 151 bis 154 reizen beispielsweise bestimmte Teilgebiete der Retina des Patienten. Der Zwischenraum zwischen Brillenrand und Gesicht kann lichtdicht abgeschlossen sein (nicht dargestellt).

In Fig. 18 ist als weitere Ausführungsform der ersten Stimulationseinheit eine undurchsichtige Lichtbrille 11 schematisch dargestellt. Bei der undurchsichtigen Lichtbrille 11 ist das Brillenglas 155 vollständig undurchsichtig. An mindestens einem Ort jedes der Brillengläser 155 ist eine Lichtquelle angebracht. Die Lichtquellen können genauso wie bei der partiell durchsichtigen Lichtbrille ausgestaltet sein, also z.B. als Leuchtdioden oder Glasfaserkabel. Bei dem in Fig. 18 gezeigten Beispiel weist jedes der Brillengläser neun Lichtquellen auf. Vier dieser Lichtquellen sind mit den Bezugszeichen 151 bis 154 versehen. Die Lichtbrille 11 kann aber auch jede andere Anzahl von Lichtquellen aufweisen, die in einer beliebigen Art angeordnet sein können.

Der Patient kann nicht durch die Brillengläser schauen, sondern er wird ausschließlich durch die Lichtquellen visuell gereizt. Die Lichtquellen reizen - wie bei der partiell durchlässigen Lichtbrille - nur die Netzhaut des Patienten. Die unterschiedlichen Lichtquellen reizen bestimmte Teilgebiete der Retina des Patienten. Der Zwischenraum zwischen Brillenrand und Gesicht kann lichtdicht abgeschlossen sein (nicht dargestellt).

Die undurchsichtige Lichtbrille 11 kann ein Fixation-Target enthalten, welches der Patient angenehm (z.B. ohne Blendungseffekte) fixieren kann. Durch die Instruktion, während der Therapie das Fixation-Target zu fixieren, wird verhindert, dass der Patient mit Augenfolgebewegungen den unterschiedlichen, aufleuchtenden Lichtquellen folgt. In letzterem Falle würde vor allem der zentrale Teil der Retina, die Fovea, gereizt, während mit einem Fixation-Target die unterschiedlichen Teile der Retina gereizt werden können.

Ein Stimulationsverfahren, das beispielsweise mit den in den Fig. 17 und 18 gezeigten Lichtbrillen 11 durchgeführt werden kann, ist in Fig. 19 schematisch dargestellt. In Fig. 19 sind untereinander die von den Lichtquellen 151 bis 154 der Lichtbrille 11 applizierten optischen ersten Reize 156 gegen die Zeit t aufgetragen.

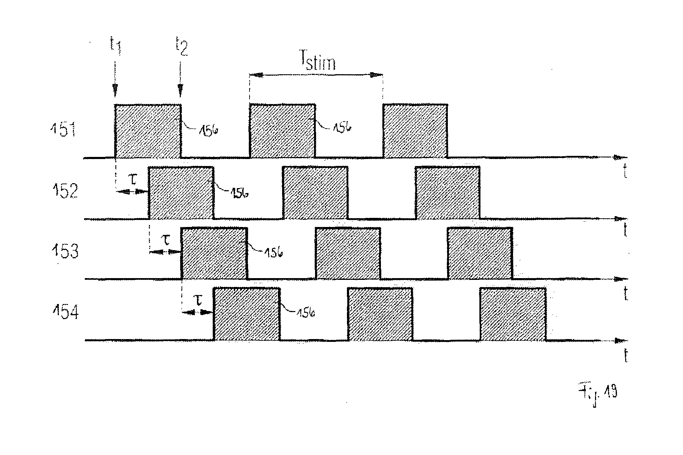

Das in Fig. 19 dargestellte Verfahren entspricht im Wesentlichen dem in Fig. 13 gezeigten Verfahren für die Transmissionsbrille. Bei dem in Fig. 19 dargestellten Verfahren appliziert jede der Lichtquellen 151 bis 154 periodisch den Reiz 156. Die Frequenz $f_{stim} = 1/T_{stim}$, mit welcher die Reize 156 pro Lichtquelle 151 bis 154 wiederholt werden, kann im Bereich von 1 bis 30 Hz und insbesondere im Bereich von 5 bis 20 Hz liegen, kann aber auch kleinere oder größere Werte annehmen.

Zur einfacheren Darstellung ist in Fig. 19 das Stimulationsverfahren nur für vier Lichtquellen 151 bis 154 dargestellt. Dieses Verfahren kann jedoch in entsprechender Weise auf eine beliebige Anzahl von Lichtquellen erweitert werden.

Bei der Erzeugung der Reize 156 mittels Lichtquellen wird typischerweise die betreffende Lichtquelle zum Zeitpunkt $t_1$ eingeschaltet und zum Zeitpunkt $t_2$ ausgeschaltet. Die maximale Amplitude (Helligkeit) der einzelnen Lichtreize liegt beispielsweise in einem Bereich von 1 bis 20 cd/m$^2$. Während der Stimulation, d.h. während der Zeitspanne zwischen $t_1$ und $t_2$, können auch kleinere Helligkeitswerte verwendet werden.

Alle im Zusammenhang mit den Fig. 13 bis 16 beschriebenen Ausgestaltungen können in entsprechender Weise auch auf die Stimulation mittels der in den Fig. 17 und 18 dargestellten Lichtbrillen 11 übertragen werden.

Stimulationseinheiten zur Erzeugung spezifischer akustischer Reize:

Im Folgenden werden Ausgestaltungen der nicht-invasiven ersten Stimulationseinheit 11 zur Erzeugung akustischer erster Reize beschrieben. Derartige Stimulationseinheiten lassen sich auch der deutschen Patentanmeldung Nr. 10 2008 015 259.5 mit dem Titel "Vorrichtung und Verfahren zur auditorischen Stimulation" entnehmen, die am 20. März 2008 beim Deutschen Patent- und Markenamt hinterlegt worden ist. Der gesamte Offenbarungsgehalt der deutschen Patentanmeldung Nr. 10 2008 015 259.5 wird hiermit in die Offenbarung der vorliegenden Anmeldung aufgenommen.

Im Folgenden wird nur auf die Erzeugung der akustischen ersten Reize eingegangen. Es versteht sich, dass diese spezifischen ersten Reize in Kombination mit den unspezifischen zweiten Reizen, wie sie oben z.B. im Zusammenhang mit den Fig. 1 bis 5 beschrieben wurden, appliziert werden.

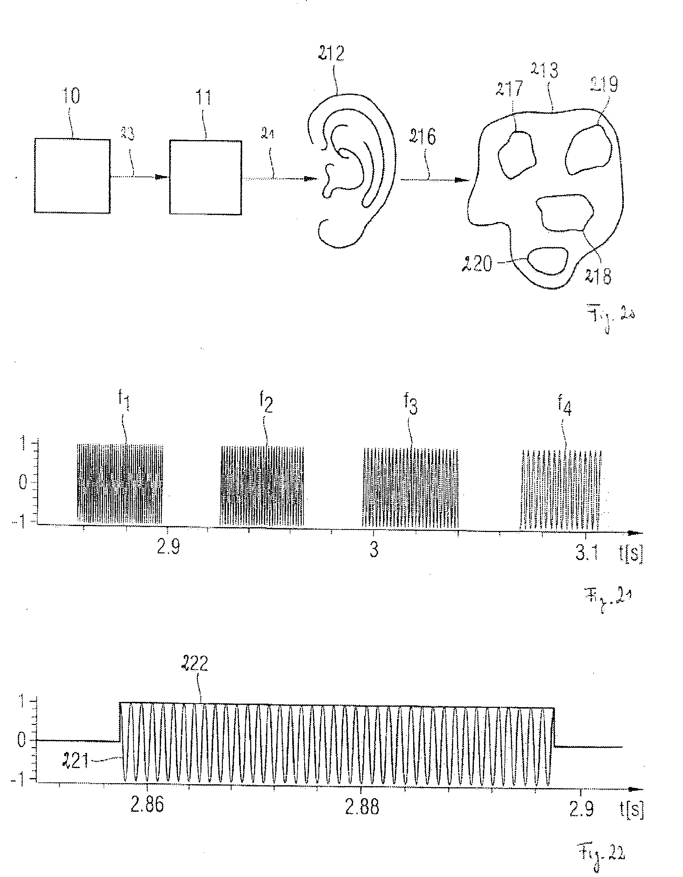

Fig. 20 zeigt schematisch eine Ausgestaltung der ersten Stimulationseinheit 11 zur Erzeugung akustischer erster Reize 21. Die erste Stimulationseinheit 11 wird von der Steuereinheit 10 mit Steuersignalen 23 angesteuert. In Fig. 20 sind ferner ein Ohr 212 eines Patienten sowie der auditorische Cortex 213 im Gehirn des Patienten schematisch dargestellt.

Das Frequenzspektrum der akustischen ersten Reize 21 kann ganz oder teilweise im für den Menschen hörbaren Bereich liegen. Die akustischen ersten Reize 21 werden von dem Patienten über ein oder beide Ohren 212 aufgenommen und über den oder die Hörnerven 216 an Neuronenpopulationen im Gehirn weitergeleitet. Die akustischen ersten Reize 21 sind derart ausgestaltet, dass sie Neuronenpopulationen im auditorischen Cortex 213 stimulieren. Im Frequenzspektrum der akustischen ersten Reize 21 sind zumindest eine erste Frequenz $f_1$ und eine zweite Frequenz $f_2$ vorhanden. Die akustischen ersten Reize 21 können ferner noch weitere Frequenzen oder Frequenzgemische enthalten, in dem in Fig. 20 gezeigten Ausführungsbeispiel sind dies eine dritte Frequenz $f_3$ und eine vierte Frequenz $f_4$.

Die von der ersten Stimulationseinheit 11 erzeugten akustischen ersten Reize 21 werden im Innenohr in Nervenimpulse umgesetzt und über den Hörnerv 216 zu dem auditorischen Cortex 213 weitergeleitet. Durch die tonotope Anordnung des auditorischen Cortex 213 wird bei der akustischen Stimulation des Innenohres mit einer bestimmten Frequenz ein bestimmter Teil des auditorischen Cortex 213 aktiviert. Die tonotope Anordnung des auditorischen Cortex ist z.B. in den folgenden Artikeln beschrieben: "Tonotopic organization of the human auditory cortex as detected by BOLD-FMRI" von D. Bilecen, K. Scheffler, N. Schmid, K. Tschopp und J. Seelig (erschienen in Hearing Research 126, 1998, Seiten 19 bis 27), "Representation of lateralization and tonotopy in primary versus secondary human auditory cortex" von D. R. M. Langers, W. H. Backes und P. van Dijk (erschienen in NeuroImage 34, 2007, Seiten 264 bis 273) und "Reorganization of auditory cortex in tinnitus" von W. Mühlnickel, T. Elbert, E. Taub und H. Flor (erschienen in Proc. Natl. Acad. Sci. USA 95, 1998, Seiten 10340 bis 10343).

In dem Beispiel gemäß Fig. 20 sind die akustischen ersten Reize 21 so ausgestaltet, dass mit ihnen eine Neuronenpopulation des auditorischen Cortex 213 mit einer krankhaft synchronen und oszillatorischen Aktivität stimuliert wird. Diese Neuronenpopulation lässt sich vor Beginn der Stimulation zumindest gedanklich in verschiedene Subpopulationen untergliedern, u.a. in die in Fig. 20 gezeigten Subpopulationen 217, 218, 219 und 220. Vor Beginn der Stimulation feuern die Neuronen aller Subpopulationen 217 bis 220 weitgehend synchron und im Mittel mit der gleichen pathologischen Frequenz. Aufgrund der tonotopen Organisation des auditorischen Cortex 213 werden mittels der ersten Frequenz $f_1$ die erste Subpopulation 217, mittels der zweiten Frequenz $f_2$ die zweite Subpopulation 218, mittels der dritten Frequenz $f_3$ die dritte Subpopulation 219 und mittels der vierten Frequenz $f_4$ die vierte Subpopulation 220 stimuliert. Die Stimulation mit den akustischen ersten Reizen 21 bewirkt in den jeweiligen Subpopulationen 217 bis 220 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Subpopulationen 217 bis 220 mittels einer gezielten Stimulation kontrolliert.

Aufgrund der tonotopen Anordnung des auditorischen Cortex 213 sowie der Mehrzahl von Frequenzen $f_1$ bis $f_4$, die in den akustischen ersten Reizen 21 enthalten sind, ist es möglich, die krankhafte Neuronenpopulation an den unterschiedlichen Stellen 217 bis 220 gezielt zu stimulieren. Dies ermöglicht es, die Phase der neuronalen Aktivität der krankhaften Neuronenpopulation an den unterschiedlichen Stimulationsstellen 217 bis 220 zu unterschiedlichen Zeitpunkten zurückzusetzen, indem die Frequenzen $f_1$ bis $f_4$ zu unterschiedlichen Zeitpunkten appliziert werden. Im Ergebnis wird dadurch die krankhafte Neuronenpopulation, deren Neuronen zuvor synchron und mit gleicher Frequenz und Phase aktiv waren, in die Subpopulationen 217 bis 220 aufgespalten. Innerhalb jeder der Subpopulationen 217 bis 220 sind die Neuronen weiterhin synchron und feuern auch weiterhin im Mittel mit derselben pathologischen Frequenz, aber jede der Subpopulationen 217 bis 220 weist bezüglich ihrer neuronalen Aktivität die Phase auf, die ihr durch den Stimulationsreiz mit der zugehörigen Frequenz $f_1$ bis $f_4$ aufgezwungen wurde.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d.h. die komplette Desynchronisation, ist somit nach der Applikation der akustischen ersten Reize 21 nicht sofort vorhanden, sondern stellt sich meist innerhalb weniger Perioden oder gar in weniger als einer Periode der pathologischen Aktivität ein.

Um den auditorischen Cortex 213 an unterschiedlichen Stellen, z.B. den in Fig. 20 gezeigten Stellen bzw. Subpopulationen 217 bis 220, fokal zu stimulieren, müssen reine Töne der zugehörigen Frequenzen $f_1$, $f_2$, $f_3$ und $f_4$ (mit geeigneter Einhüllender zur Vermeidung von Klickgeräuschen) verabreicht werden. Infolge der tonotopen Anordnung des auditorischen Cortex 213 werden unterschiedliche Teile des Gehirns durch die gleichzeitige Verabreichung der zugehörigen unterschiedlichen reinen Töne $f_1$ bis $f_4$, d.h. durch die Superposition verschiedener Sinusschwingungen stimuliert. Sollen die vier unterschiedlichen Orte 217 bis 220 z.B. zu unterschiedlichen Zeiten gereizt werden, werden die vier verschiedenen Frequenzen $f_1$ bis $f_4$ zu den jeweiligen Zeiten appliziert. Beispielhaft ist dies in Fig. 21 gezeigt. Hier werden Sinusschwingungen mit den Frequenzen $f_1$ = 1000 Hz, $f_2$ = 800 Hz, $f_3$ = 600 Hz und $f_4$ = 400 Hz sukzessive und pulsförmig appliziert, was zu einer sukzessiven fokalen Reizung an den vier verschiedenen Orten 217 bis 220 des auditorischen Cortex 213 führt. Die Stärke der durch die jeweilige Sinusschwingung erzeugten Reizung des jeweiligen Areals im auditorischen Cortex 213 entspricht der Amplitude der jeweiligen Sinusschwingung.

Die Generierung der in Fig. 21 gezeigten pulsförmigen Sinusschwingungen ist in Fig. 22 beispielhaft dargestellt. Dort wird eine Sinusschwingung 221 mit einer Rechteckfunktion 222, die beispielsweise die Werte 0 oder 1 annehmen kann, multipliziert. Zu den Zeitpunkten, zu denen die Rechteckfunktion 222 den Wert 0 hat, ist der zugehörige Reiz abgeschaltet und während der Zeit, in der die Rechteckfunktion 222 gleich 1 ist, ist der Reiz angeschaltet.

Anstelle der Rechteckfunktion 222 kann die Sinusschwingung 221 mit einer beliebigen anderen Funktion multipliziert werden. Im Ergebnis entspricht diese Multiplikation einer Amplitudenmodulation der Sinusschwingung 221. Um Klickgeräusche aufgrund eines scharfen Beginns und Endes der Töne zu vermeiden, kann statt der Rechteckfunktion 222 ein glatterer Verlauf gewählt werden, z.B. durch Multiplikation der Sinusschwingung 221 mit einer Sinus-Halbschwingung von geeigneter Dauer, z.B. der Dauer eines Reizes.

Anstelle der vorstehend beschriebenen Sinusschwingungen können auch oszillierende Signale mit einer anderen Signalform, wie z.B. Rechtecksignale, die mit der entsprechenden Grundfrequenz oszillieren, zur Generierung der akustischen ersten Reize 21 herangezogen werden.

Sofern statt einer fokalen Reizung eine weniger fokale Reizung durchgeführt werden soll, die größere Teile des auditorischen Cortex 213 aktiviert, so werden Frequenzgemische anstelle von einzelnen Frequenzen, beispielsweise pulsförmig appliziert. Mittels eines Frequenzgemisches in den Grenzen zwischen einer unteren Frequenz $f^{unten}$ und einer höheren Frequenz $f^{oben}$ werden all die Teile des auditorischen Cortex 213 gereizt, die durch die Frequenzen zwischen $f^{unten}$ und $f^{oben}$ aufgrund der tonotopen Anordnung stimuliert werden. Sollen z.B. vier unterschiedliche größere Bereiche des auditorischen Cortex 213 zu unterschiedlichen Zeiten stimuliert werden, so werden die vier zugehörigen Frequenzgemische mit den Grenzen $f_j^{unten}$ und $f_j^{oben}$ (j = 1, 2, 3, 4) zu den gewünschten Zeiten appliziert.

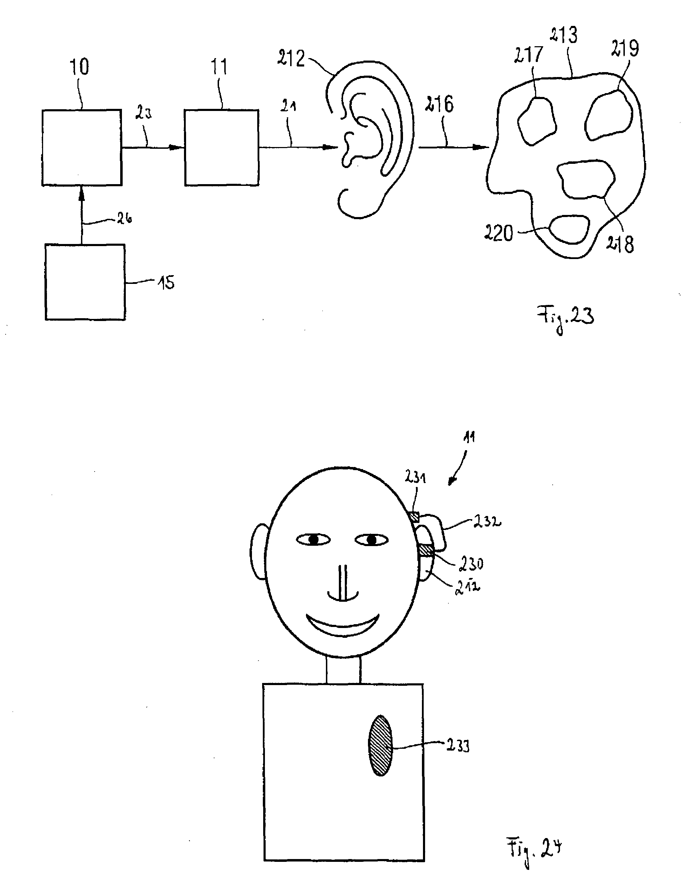

Die erste Stimulationseinheit 11 kann beispielsweise in einem sogenannten "open loop"-Modus betrieben werden, bei welchem die Steuereinheit 10 die erste Stimulationseinheit 11 derart ansteuert, dass diese vorgegebene akustische erste Reize 21 während einer bestimmten Stimulationszeit (z.B. während mehrerer Stunden) erzeugt. Des Weiteren kann die erste Stimulationseinheit 11 zusammen mit der Steuereinheit 10 auch zu einem in Fig. 23 schematisch dargestellten "closed loop"-System weitergebildet werden. Bei dieser Ausführung ist zusätzlich noch eine Messeinheit 15 vorgesehen, welche am Patienten aufgenommene Messsignale bereitstellt und diese an die Steuereinheit 10 weiterleitet. Bei der Messeinheit 15 kann es sich um nicht-invasive oder invasive Sensoren handeln (vgl. obige Beschreibung im Zusammenhang mit Fig. 3).

Hinsichtlich des Zusammenwirkens der Steuereinheit 10 mit der Messeinheit 15 sind verschiedene Ausgestaltungen denkbar.

Beispielsweise kann - wie oben beschrieben wurde - anhand der Messsignale zwischen dem ersten Betriebsmodus, der Lernphase, und dem zweiten Betriebsmodus, der eigentlichen Stimulationsphase, gewechselt werden. Ferner können von der Steuereinheit 10 anhand der Ausprägung der krankhaften Merkmale Parameter der akustischen ersten Reize 21, wie beispielsweise die Amplituden der jeweiligen Sinusschwingungen oder die Pausen zwischen Stimulationssequenzen, eingestellt werden.

Des Weiteren kann vorgesehen sein, dass die von der Messeinheit 15 aufgenommenen Messsignale direkt oder gegebenenfalls nach einem oder mehreren Verarbeitungsschritten in akustische erste Reize 21 umgesetzt werden und von der ersten Stimulationseinheit 11 appliziert werden. Beispielsweise können die Messsignale verstärkt und gegebenenfalls nach mathematischer Verrechnung (z.B. nach Mischung der Messsignale) mit einer Zeitverzögerung und linearen und/oder nichtlinearen Verrechnungsschritten als Steuersignale 23 in den Steuereingang der ersten Stimulationseinheit 11 eingespeist werden. Der Verrechnungsmodus wird hierbei so gewählt, dass der krankhaften neuronalen Aktivität entgegengewirkt wird und die akustischen ersten Reize 21 mit abnehmender krankhafter neuronaler Aktivität ebenfalls verschwinden oder zumindest deutlich in ihrer Stärke reduziert werden.

In Fig. 24 ist schematisch eine Ausgestaltung der ersten Stimulationseinheit 11 gezeigt, die einen Schallgenerator (Lautsprecher) verwendet, der in einen Ohrstöpsel 230 eingefasst ist. Der Ohrstöpsel 230 wird in den äußeren Gehörgang eines Ohrs 212 des Patienten eingefügt und mit oder ohne Bügel bzw. einer anderen geeigneten mechanischen Hilfe am Ohr 212 befestigt. Die Steuereinheit 10, welche den Schallgenerator ansteuert, sowie eine Batterie oder ein Akku zur Stromversorgung der elektrischen Bauelemente können in einer oder mehreren separaten Einheiten 231 untergebracht sein. Die Einheit 231 kann mittels einer mechanischen Halterung, z.B. einem Bügel, mit dem Ohrstöpsel 230 verbunden sein. Ein Verbindungskabel 232 verbindet den Ohrstöpsel 230 mit der Steuereinheit 10 bzw. der Batterie.

Alternativ kann statt des Ohrstöpsels 230 auch ein Kopfhörer verwendet werden, der die Steuereinheit 10 und die Batterie enthält. Die in Fig. 24 gezeigte Vorrichtung kann vom Patienten mittels einer Bedieneinheit (z.B. Anschaltknopf und/oder Drehregler) angeschaltet werden, die entweder an der Einheit 231 oder direkt am Ohrstöpsel 230 angebracht ist. Mit dem Drehregler kann z.B. die maximale Stimulationsstärke eingestellt werden. Zusätzlich zu den vorstehend genannten Komponenten kann ein Steuermedium 233 vorgesehen sein, welches beispielsweise telemetrisch (z.B. über Funk) oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Ferner kann auch ein weiteres, z.B. vom Arzt zu bedienendes Steuermedium (nicht dargestellt) bereitgestellt werden, welches telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

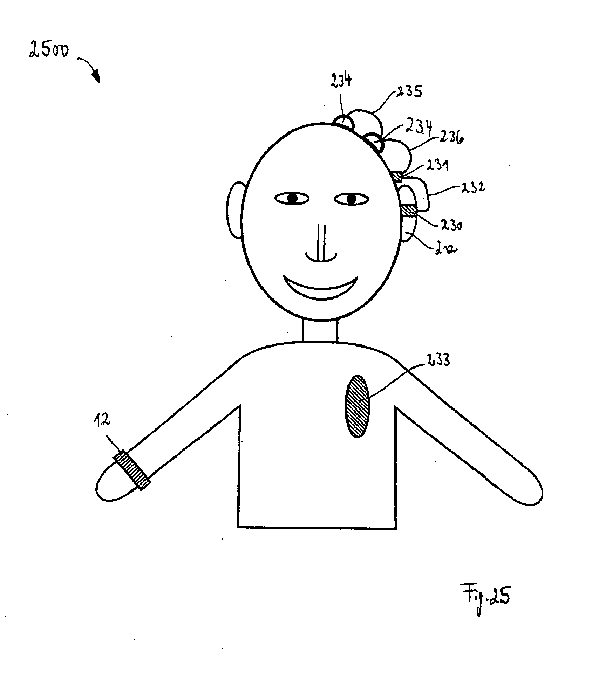

Fig. 25 zeigt schematisch eine Vorrichtung 2500, die eine wie in Fig. 24 ausgestaltete erste Stimulationseinheit 11, eine aus EEG-Elektroden 234 bestehende Messeinheit sowie eine zweite Stimulationseinheit 12 zur Applikation der zweiten, unspezifischen Reize aufweist. Die EEG-Elektroden 234 sind epikutan, d.h. auf der Haut des Patienten befestigt und über Verbindungskabel 235, 236 mit der Steuereinheit 10 verbunden. Die Steuereinheit 10 verwendet die von den EEG-Elektroden 234 gelieferten Messsignale z.B. zur Einstellung des Betriebsmodus. Die Steuereinheit 10 kann die mittels der EEG-Elektroden 234 gemessene Potentialdifferenz auch verstärken und dieses Signal nach einer optionalen linearen oder nicht-linearen Verrechnung zur Ansteuerung des Schallgenerators in dem Ohrstöpsel 230 verwenden. Als Alternative zu den Verbindungskabeln 235, 236 können die EEG-Elektroden 234 auch schnurlos, d.h. telemetrisch mit der Steuereinheit 10 verbunden sein. Dies hat den Vorteil, dass der Patient nicht durch Verbindungskabel behindert wird und z.B. an Hindernissen hängen bleiben kann. Als zweite Stimulationseinheit 12 weist die Vorrichtung 2500 die in Fig. 4A und 4B gezeigte Konditionierungsuhr auf. Die zweiten, unspezifischen Reize können alternativ auch mittels einer anders ausgestalteten zweiten Stimulationseinheit 12 generiert werden.

Anhand der oben bereits erwähnten vier Frequenzen $f_1$ bis $f_4$ soll nachfolgend beispielhaft erläutert werden, wie durch zeitversetztes Zurücksetzen der Phasen der neuronalen Aktivität von Subpopulationen einer krankhaft synchronen und oszillatorischen Neuronenpopulation eine Desynchronisation der gesamten Neuronenpopulation erzielt werden kann. Die vier Frequenzen $f_1$ bis $f_4$ sind lediglich beispielhaft zu verstehen, d.h. es kann eine beliebige andere Zahl von Frequenzen oder Frequenzgemischen zu Stimulationszwecken eingesetzt werden. Die Frequenzen $f_1$ bis $f_4$ sind so ausgewählt worden, dass mit ihnen jeweils bestimmte Bereiche 217 bis 220 des auditorischen Cortex 213 stimuliert werden. Dies ermöglicht die oben beschriebene Aufspaltung einer krankhaften Neuronenpopulation in Subpopulationen 217 bis 220. Damit die Subpopulationen 217 bis 220 nach der Stimulation unterschiedliche Phasen aufweisen, können die Frequenzen $f_1$ bis $f_4$ beispielsweise zeitversetzt appliziert werden.

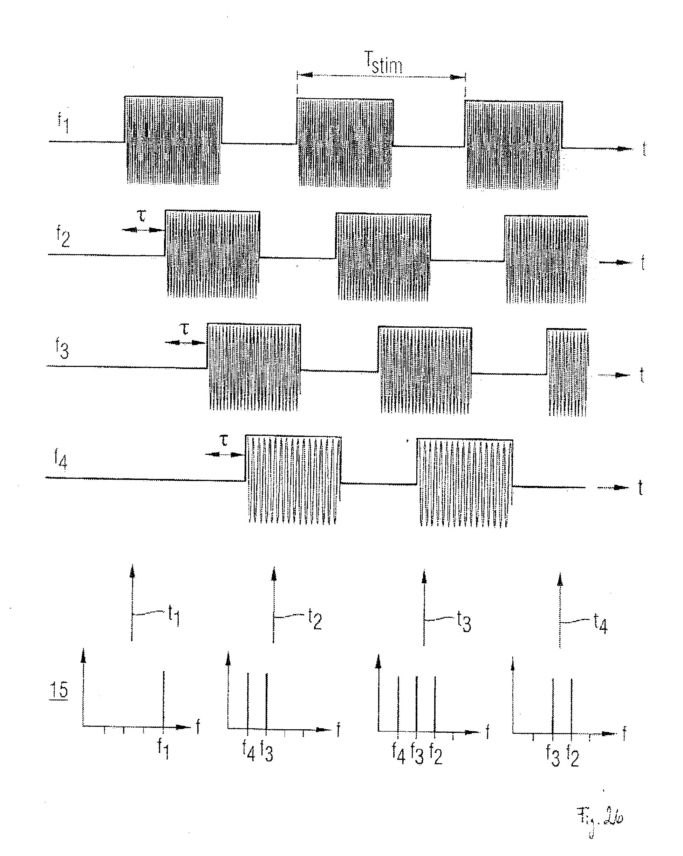

Ein für die oben beschriebenen Zwecke geeignetes Stimulationsverfahren ist in Fig. 26 schematisch dargestellt. In Fig. 26 sind in den oberen vier Zeilen untereinander vier Sinusschwingungen mit den Frequenzen $f_1$, $f_2$, $f_3$ bzw. $f_4$ gegen die Zeit t aufgetragen. Aus den dargestellten Sinusschwingungen werden die akustischen ersten Reize 21 gebildet. Zur Erzeugung von pulsförmigen Sinusschwingungen sind die vier Sinusschwingungen mit Rechteckfunktionen multipliziert worden. Wie oben bereits erläutert wurde, können statt der Rechteckfunktionen auch glattere Funktionen, wie z.B. Sinus-Halbschwingungen, verwendet werden, um Klickgeräusche zu vermeiden. Jeder Sinusschwingungspuls wiederholt sich periodisch mit einer Frequenz $f_{stim}$. Die Frequenz $f_{stim} = 1/T_{stim}$ kann im Bereich von 1 bis 30 Hz und insbesondere im Bereich von 5 bis 20 Hz liegen, kann aber auch kleinere oder größere Werte annehmen. Derartige Sequenzen von pulsförmigen Sinusschwingungen sind, wenn sie als akustische erste Reize 21 appliziert werden, geeignet, die neuronale Phase der jeweils stimulierten krankhaften Neuronen-Subpopulation 217, 218, 219 bzw. 220 zurückzusetzen. Der Phasenreset ergibt sich dabei nicht notwendigerweise bereits nach einem oder wenigen Pulsen, sondern es können eine gewisse Anzahl der in Fig. 26 gezeigten Sinusschwingungspulse erforderlich sein, um die neuronale Phase der jeweiligen Subpopulation 217, 218, 219 bzw. 220 zurückzusetzen.

Die Frequenz $f_{stim}$ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz, kann aber auch außerhalb dieses Bereichs liegen. Beim Tinnitus findet sich z.B. im Frequenzbereich von 1,5 bis 4 Hz übermäßig synchrone neuronale Aktivität. Hierbei ist zu beachten, dass die Frequenz, mit welcher die krankhaften Neuronen synchron feuern, üblicherweise nicht konstant ist, sondern durchaus Variationen aufweisen kann und darüber hinaus bei jedem Patienten individuelle Abweichungen zeigt.

Zur Ermittlung der Frequenz $f_{stim}$ kann beispielsweise die mittlere Peakfrequenz der krankhaften rhythmischen Aktivität des Patienten bestimmt werden. Diese Peakfrequenz kann dann als Stimulationsfrequenz $f_{stim}$ verwendet werden oder auch variiert werden, beispielsweise in einem Bereich von $f_{stim}$ - 3 Hz bis $f_{stim}$ + 3 Hz. Alternativ kann aber auch ohne vorherige Messung eine Frequenz $f_{stim}$ im Bereich von 1 bis 30 Hz gewählt werden und diese beispielsweise während der Stimulation variiert werden, bis die Frequenz $f_{stim}$ gefunden wird, mit der sich die besten Stimulationserfolge erzielen lassen. Als weitere Alternative kann für die Stimulationsfrequenz $f_{stim}$ ein für die jeweilige Krankheit bekannter Literaturwert herangezogen werden. Eventuell kann dieser Wert noch variiert werden, bis beispielsweise optimale Stimulationsergebnisse erzielt werden.

Die Dauer eines Sinusschwingungspulses, d.h. die Zeitspanne, in dem in der vorliegenden Ausgestaltung die Rechteckfunktion den Wert 1 annimmt, kann beispielsweise $T_{stim}/2$ betragen. In diesem Fall sind die Zeitspanne, während der die jeweilige Frequenz zur Stimulation beiträgt, und die nachfolgende Stimulationspause gleich lang. Es ist aber auch möglich andere Stimulationsdauern zu wählen, beispielsweise im Bereich von $T_{stim}/2 - T_{stim}/10$ bis $T_{stim}/2 + T_{stim}/10$. Die Stimulationsdauern können beispielsweise experimentell bestimmt werden.

Gemäß der in Fig. 26 gezeigten Ausgestaltung erfolgt die Verabreichung der einzelnen Frequenzen $f_1$ bis $f_4$ mit einer zeitlichen Verzögerung zwischen den einzelnen Frequenzen $f_1$ bis $f_4$. Beispielsweise kann der Beginn zeitlich aufeinander folgender und unterschiedliche Frequenzen aufweisender Pulse um eine Zeit $\tau$ verschoben sein.

Im Fall von N Frequenzen, die zur Stimulation eingesetzt werden, kann die zeitliche Verzögerung $\tau$ zwischen jeweils zwei aufeinander folgenden Pulsen beispielsweise im Bereich eines N-tels der Periode $T_{stim} = 1/f_{stim}$ liegen. In dem in Fig. 26 gezeigten Ausführungsbeispiel (N = 4) beträgt die zeitliche Verzögerung $\tau$ dementsprechend $T_{stim}/4$. Von der Vorgabe, dass die zeitliche Verzögerung $\tau$ zwischen jeweils zwei aufeinander folgenden Sinusschwingungspulsen $T_{stim}/N$ beträgt, kann bis zu einem gewissen Grad abgewichen werden. Beispielsweise kann von dem Wert $T_{stim}/N$ für die zeitliche Verzögerung $\tau$ um bis zu $\pm 5\%$, $\pm 10\%$, $\pm 20\%$ oder $\pm 30\%$ abgewichen werden. Bei derartigen Abweichung wurden noch Stimulationserfolge erzielt, d.h. es konnte noch ein desynchronisierender Effekt beobachtet werden.

Aus den periodischen Sinusschwingungspulsen mit den Frequenzen $f_1$ bis $f_4$ wird durch Superposition der akustische erste Reiz 21 gebildet. Die einzelnen Sinusschwingungspulse können dabei beispielsweise linear oder nichtlinear miteinander kombiniert werden. Dies bedeutet, dass die Sinusschwingungen der einzelnen Frequenzen $f_1$ bis $f_4$ nicht notwendigerweise mit den gleichen Amplituden zu dem akustischen ersten Reiz 21 kombiniert werden müssen. In der untersten Zeile von Fig. 26 ist beispielhaft das Frequenzspektrum des akustischen ersten Reizes 21 zu vier verschiedenen Zeitpunkten $t_1$, $t_2$, $t_3$ und $t_4$ dargestellt. Die dort gezeigten Frequenzspektren, insbesondere die Höhe und Form der Frequenzpeaks, sind lediglich beispielhaft zu verstehen und können auch völlig unterschiedliche Formen aufweisen. Im Einzelnen lassen sich den dargestellten Frequenzspektren die folgenden Aussagen entnehmen: Zum Zeitpunkt $t_1$ tritt lediglich die Frequenz $f_1$ in dem akustischen ernsten Reiz 21 auf. Zum Zeitpunkt $t_2$ sind dies die Frequenzen $f_3$ sowie $f_4$, zum Zeitpunkt $t_3$ die Frequenzen $f_2$ bis $f_4$ und zum Zeitpunkt $t_4$ die Frequenzen $f_2$ sowie $f_3$.

Gemäß einer alternativen Ausgestaltung werden statt der Frequenzen $f_1$ bis $f_4$ vier Frequenzgemische mit den Grenzen $f_j^{unten}$ und $f_j^{oben}$ (j = 1, 2, 3, 4) verwendet. In einem Frequenzgemisch j kann eine beliebige Anzahl von Frequenzen im Bereich von $f_j^{unten}$ bis $f_j^{oben}$ vorliegen.

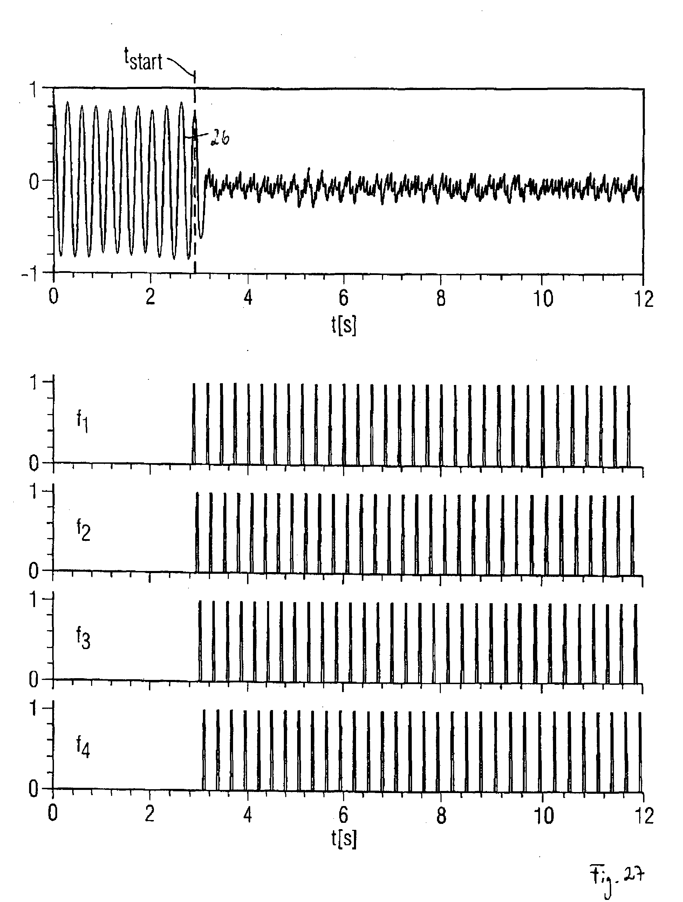

Gemäß einer weiteren alternativen Ausgestaltung werden anstelle der Rechteckfunktionen andere Funktionen zur Amplitudenmodulation der Sinusschwingungen eingesetzt werden, z.B. Sinushalbwellen, deren Frequenz kleiner als $f_1$ bis $f_4$ ist. Ferner ist es beispielsweise denkbar, dass dreieckförmige Pulse als Modulationsfunktionen eingesetzt werden. Ein solcher Puls kann eine sprungförmigen Onset (von 0 auf 1) aufweisen und danach einen Abfall auf 0, wobei der Abfall beispielsweise durch eine lineare oder exponentielle Funktion gegeben sein kann. Durch die Modulationsfunktion wird letztlich die Form der Einhüllenden der einzelnen Pulse bestimmt. In Fig. 27 ist die bereits in Fig. 26 gezeigte Stimulation über einen längeren Zeitraum hinweg dargestellt. Die einzelnen Sinusschwingungen mit den Frequenzen $f_1$ = 1000 Hz, $f_2$ = 800 Hz, $f_3$ = 600 Hz und $f_4$ = 400 Hz sind in Fig. 27 nicht gezeigt, sondern nur die jeweiligen rechteckförmigen Einhüllenden. Ferner ist in Fig. 27 ein beispielsweise von der Messeinheit 15 aufgenommenes Messsignal 26 dargestellt, das die neuronale Aktivität im auditorischen Cortex vor und während der Stimulation wiedergibt. Die Periode $T_{stim}$ beträgt vorliegend 1/(3,5 Hz) = 0,29 s.

Die Stimulation wird zum Zeitpunkt $t_{start}$ gestartet. Dem Messsignal 26, das in dem vorliegenden Beispiel bandpassgefiltert worden ist, ist zu entnehmen, dass die Neuronen im auditorischen Cortex vor Beginn der Stimulation eine synchrone und oszillatorische Aktivität aufweisen. Kurz nach Beginn der Stimulation wird die krankhaft synchrone neuronale Aktivität im Zielgebiet bereits unterdrückt.

Von dem in den Fig. 26 und 27 gezeigten streng periodischen Stimulationsmuster kann auf unterschiedliche Art und Weise abgewichen werden. Beispielsweise braucht die zeitliche Verzögerung $\tau$ zwischen zwei aufeinander folgenden Sinusschwingungspulsen nicht notwendigerweise stets gleich groß zu sein. Es kann vorgesehen sein, dass die zeitlichen Abstände zwischen den einzelnen Sinusschwingungspulsen unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

Des Weiteren können während der Applikation der akustischen ersten Reize 21 Pausen vorgesehen werden, während denen keine Stimulation erfolgt. Die Pausen können beliebig lang gewählt werden und insbesondere ein ganzzahliges Vielfaches der Periode $T_{stim}$ betragen. Die Pausen können nach einer beliebigen Anzahl von Stimulationen eingehalten werden. Z.B. kann eine Stimulation während N aufeinander folgender Perioden der Länge $T_{stim}$ durchgeführt werden und anschließend eine Stimulationspause während M Perioden der Länge $T_{stim}$ eingehalten werden, wobei N und M kleine ganze Zahlen sind, z.B. im Bereich von 1 bis 15. Dieses Schema kann entweder periodisch fortgesetzt werden oder stochastisch und/oder deterministisch, z.B. chaotisch, modifiziert werden.

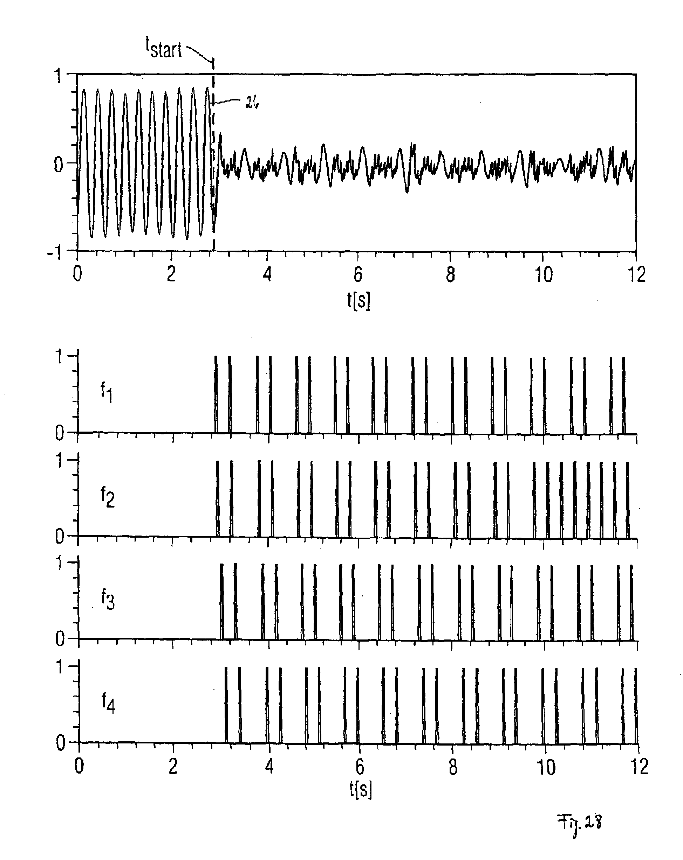

In Fig. 28 ist eine derartige Stimulation gezeigt. Hier gelten N = 2 und M = 1. Ansonsten entspricht die Stimulation der in Fig. 27 gezeigten Stimulation.

Eine weitere Möglichkeit, von dem in Fig. 26 gezeigten streng periodischen Stimulationsmuster abzuweichen, besteht darin, die zeitlichen Abstände zwischen aufeinander folgenden Pulsen einer Frequenz $f_j$ oder eines Frequenzgemisches mit den Grenzen $f_j^{unten}$ und $f_j^{oben}$ (j = 1, 2, 3, 4) stochastisch oder deterministisch oder gemischt stochastisch-deterministisch zu variieren.

Des Weiteren kann pro Periode $T_{stim}$ (oder in anderen Zeitschritten) die Reihenfolge, in welcher die beteiligten Frequenzen $f_j$ oder Frequenzgemische mit den Grenzen $f_j^{unten}$ und $f_j^{oben}$ appliziert werden, variiert werden. Diese Variation kann stochastisch oder deterministisch oder gemischt stochastisch-deterministisch erfolgen.

Ferner kann pro Periode $T_{stim}$ (oder in einem anderen Zeitintervall) nur eine bestimmte Anzahl der Frequenzen $f_j$ oder Frequenzgemische mit den Grenzen $f_j^{unten}$ und $f_j^{oben}$ appliziert werden und die an der Stimulation beteiligten Frequenzen $f_j$ oder Frequenzgemische mit den Grenzen $f_j^{unten}$ und $f_j^{oben}$ können in jedem Zeitintervall variiert werden. Auch diese Variation kann stochastisch oder deterministisch oder gemischt stochastisch-deterministisch erfolgen.

Die vorstehend beschriebenen Stimulationssignale bewirken, dass die Phase der neuronalen Aktivität der krankhaften Neuronenpopulation an den unterschiedlichen Stimulationsstellen zu unterschiedlichen Zeitpunkten zurückgesetzt wird. Dadurch wird die krankhafte Neuronenpopulation, deren Neuronen zuvor synchron und mit gleicher Frequenz und Phase aktiv waren, in mehrere Subpopulationen aufgespalten, was letztlich zu einer Desynchronisation führt.

Im Folgenden werden weitere Ausgestaltungen der "closed loop"-Stimulation beschrieben, die beispielsweise mittels der in Fig. 25 gezeigten Vorrichtung 2500 durchgeführt werden können. Wie bereits weiter oben beschrieben wurde, kann das von der Messeinheit 15 aufgenommene Messsignal 26 dazu verwendet werden, ein Steuersignal 23 zu generieren, mit dem die erste Stimulationseinheit 11 angesteuert wird. Dabei kann das Messsignal 26 entweder direkt oder gegebenenfalls nach einem oder mehreren Verarbeitungsschritten in die akustischen ersten Reize 21 umgesetzt werden und von der ersten Stimulationseinheit 11 appliziert werden. Der Verrechnungsmodus kann hierbei so gewählt werden, dass der krankhaften neuronalen Aktivität entgegengewirkt wird und die akustischen ersten Reize 21 mit abnehmender krankhafter neuronaler Aktivität ebenfalls verschwinden oder zumindest deutlich in ihrer Stärke reduziert werden.

Bevor das Messsignal 26 in den Steuereingang der ersten Stimulationseinheit 11 eingespeist wird, kann das Messsignal 26 linear oder nicht-linear verarbeitet werden. Beispielsweise kann das Messsignal 26 gefiltert und/oder verstärkt und/oder mit einer Zeitverzögerung beaufschlagt werden und/oder mit einem anderen Messsignal 26 gemischt werden. Ferner kann mit dem Messsignal 26 oder dem verarbeiteten Messsignal 26 die Amplitude einer Sinusschwingung mit einer Frequenz im hörbaren Bereich moduliert werden und die amplitudenmodulierte Sinusschwingung kann danach mittels des Schallgenerators als akustischer erster Reiz 21 oder als Teil davon appliziert werden.

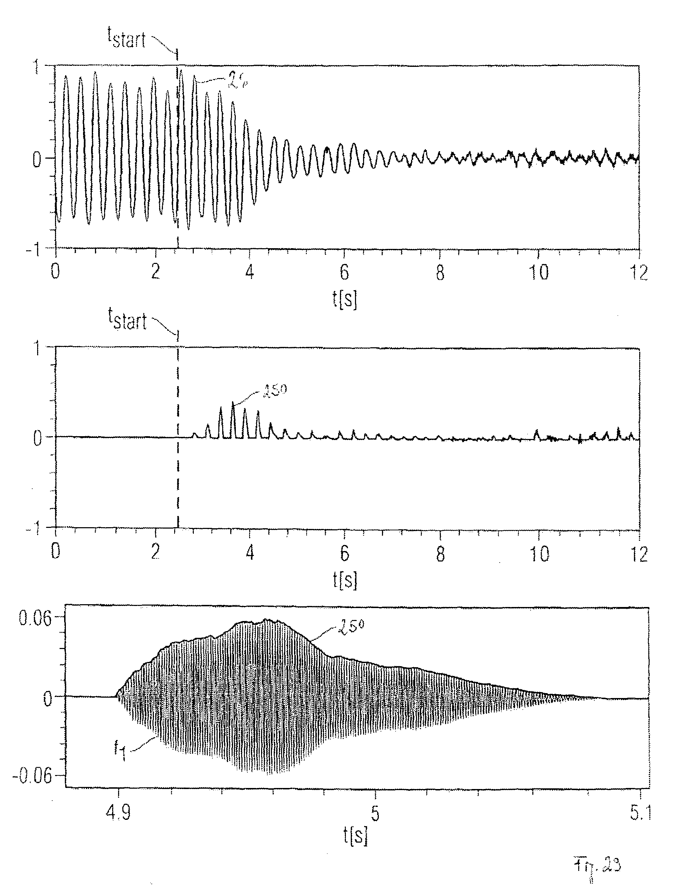

Zur Amplitudenmodulation einer Sinusschwingung oder einer anderen oszillierenden Schwingung muss nicht notwendigerweise das komplette Messsignal 26 herangezogen werden. Es kann z.B. vorgesehen sein, dass dazu nur ein Teil des Messsignals 26 oder des verarbeiteten Messsignals 26 verwendet wird, beispielsweise der Teil, der oberhalb oder unterhalb eines bestimmten Schwellwerts liegt. Eine derartige Amplitudenmodulation ist in Fig. 29 beispielhaft dargestellt. In dem obersten Graph von Fig. 29 ist das bandpassgefilterte Messsignal 26 gegen die Zeit t aufgetragen, ferner ist der Startzeitpunkt $t_{start}$ der Stimulation angegeben. In dem mittleren Graph ist das aus dem Messsignal 26 gewonnene Modulationssignal 250 dargestellt. Zur Generierung des Modulationssignals 250 ist das Messsignal 26 nicht-linear verarbeitet worden und alle negativen Werte des Messsignals 26 bzw. des verarbeiteten Messsignals 26 sind auf Null gesetzt worden. Ferner ist das Modulationssignal 250 gegenüber dem Messsignal 26 zeitverzögert worden. Anschließend ist das so gewonnene Halbwellensignal 250 mit einer Sinusschwingungen der Frequenz $f_1$ = 1000 Hz multipliziert worden. Das Modulationssignal 250 stellt die Einhüllende der Sinusschwingung dar, wie im untersten Graph von Fig. 29 für einen kleinen Zeitausschnitt gezeigt ist. Die so gewonnene amplitudenmodulierte Sinusschwingung ist anschließend in die erste Stimulationseinheit 11 rückgekoppelt worden, um von dem Schallgenerator in die akustischen ersten Reize 21 umgesetzt zu werden.

Anstelle einer Sinusschwingung mit einer einzigen Frequenz kann das Modulationssignal 250 auch mit einem beliebigen Gemisch von Sinusschwingungen (oder anderen Schwingungen) im hörbaren Frequenzbereich multipliziert werden, je nachdem, an welchen Stellen des auditorischen Cortex die Desynchronisation erfolgen soll.

Am Verlauf des in Fig. 29 dargestellten Messsignals 26 lässt sich ablesen, dass die akustische nicht-lineare zeitverzögerte Halbwellenstimulation zu einer robusten Unterdrückung der krankhaft synchronen neuronalen Aktivität führt. Der Wirkmechanismus dieser Stimulation unterscheidet sich jedoch von der Wirkungsweise des z.B. in Fig. 26 gezeigten Stimulationsverfahrens. Bei der in Fig. 29 dargestellten Stimulation wird nicht die Phase der neuronalen Aktivität in den jeweiligen stimulierten Subpopulationen zurückgesetzt, sondern die Synchronisation in der krankhaft aktiven Neuronenpopulation wird unterdrückt, indem der Sättigungsprozess der Synchronisation beeinflusst wird.

Im Folgenden wird anhand eines Beispiels erläutert, wie ein von der Messeinheit 15 gewonnenes Messsignal 26 einer nicht-linearen Prozessierung unterworfen werden kann, bevor es als Ansteuerungssignal der ersten Stimulationseinheit 11 verwendet wird.

Ausgangspunkt ist eine Gleichung für das Ansteuerungssignal S (t) :

$$S(t) = K \cdot \overline{Z}^2(t) \cdot \overline{Z}^*(t - \tau) \qquad (1)$$

In Gleichung (1) sind K ein Verstärkungsfaktor, der geeignet gewählt werden kann, und $\overline{Z}(t)$ eine mittlere Zustandsvariable des Messsignals 26. $\overline{Z}(t)$ ist eine komplexe Variable und kann folgendermaßen dargestellt werden:

$$\overline{Z}(t) = X(t) + iY(t), \qquad (2)$$

wobei X(t) z.B. dem neurologischen Messsignal 26 entsprechen kann. Da die betrachteten Frequenzen im Bereich von 10 Hz = 1/100ms = $1/T_\alpha$ liegen, kann der Imaginärteil Y(t) durch X(t - $\tau_\alpha$) angenähert werden, wobei beispielsweise $\tau_\alpha$ = $T_\alpha$/4 gilt. Damit ergibt sich:

$$S(t) = K \cdot \left[X(t) + iX(t - \tau_\alpha)\right]^2 \cdot \left[X(t - \tau) - iX(t - \tau - \tau_\alpha)\right]$$

(3)

Gleichung (3) kann folgendermaßen umgeformt werden:

$$
\begin{aligned}
S(t) = K \cdot \Big[ & X(t)^2 \cdot X(t - \tau) + i2X(t) \cdot X(t - \tau_\alpha) \cdot X(t - \tau) - X(t - \tau_\alpha) \cdot X(t - \tau) \\
& - iX(t - \tau - \tau_\alpha) \cdot X(t)^2 + 2X(t) \cdot X(t - \tau_\alpha) \cdot X(t - \tau - \tau_\alpha) \\
& + iX(t - \tau - \tau_\alpha) \cdot X(t - \tau_\alpha) \Big]
\end{aligned}
$$

(4)

Als Ansteuerungssignal für die Stimulationseinheit 11 wird der Realteil aus Gleichung (4) verwendet:

$$
\begin{aligned}
\mathrm{real}[S(t)] = K \cdot \Big[ & X(t)^2 \cdot X(t - \tau) - X(t - \tau_\alpha) \cdot X(t - \tau) \\
& + 2X(t) \cdot X(t - \tau_\alpha) \cdot X(t - \tau - \tau_\alpha) \Big]
\end{aligned}
$$

(5)

Mit dem rückgekoppelten und eventuell weiterverarbeiteten Messsignal 26 kann der auditorische Cortex ferner gezielt an verschiedenen Stellen stimuliert werden. Im Falle von den oben beschriebenen vier verschiedenen Frequenzen $f_1$ bis $f_4$ wird das eventuell weiterverarbeitete Messsignal 26 mit einer entsprechenden Zeitverzögerung beaufschlagt und mit den Frequenzen $f_1$ bis $f_4$ multipliziert. Sofern die Stimulation weniger fokal sein soll, sondern ausgedehnter erfolgen soll, werden statt der reinen Sinusschwingungen der Frequenzen $f_1$ bis $f_4$ vier verschiedene Frequenzgemische mit den Grenzen $f_j^{unten}$ und $f_j^{oben}$ (j = 1, 2, 3, 4) verwendet.

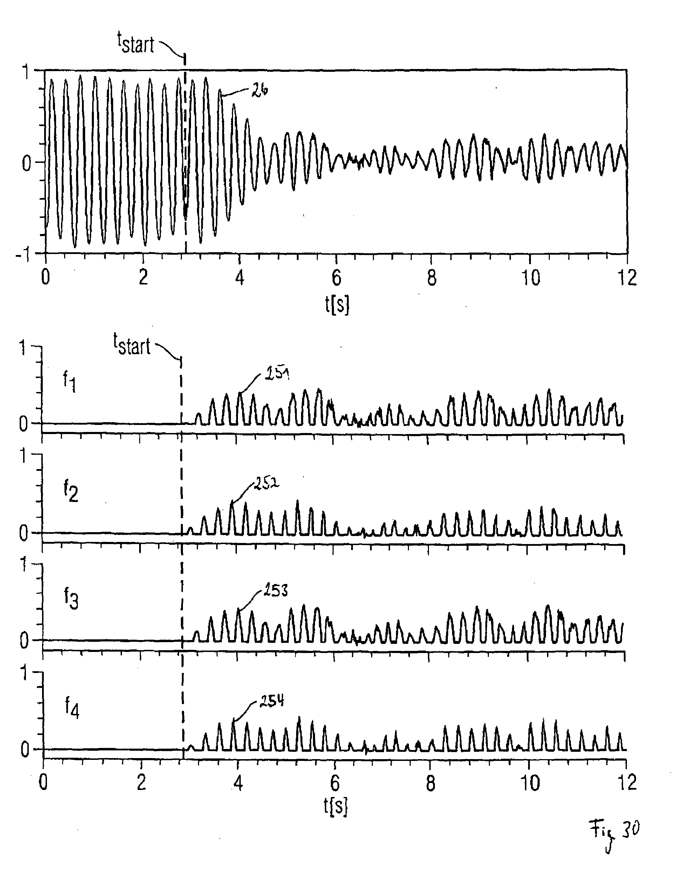

In Fig. 30 ist eine derartige Stimulation beispielhaft dargestellt. Aus dem bandpassgefilterten Messsignal 26 sind hier durch lineare Verarbeitungsschritte die Modulationssignale 251, 252, 253 und 254 gewonnen worden, mit denen Amplitudenmodulationen der Frequenzen $f_1$ bis $f_4$ durchgeführt worden sind. Durch Superposition der modulierten Sinusschwingungen ist das Steuersignal 23 erzeugt worden, welches von dem Schallgenerator 11 in die akustischen ersten Reize 21 umgesetzt worden ist.

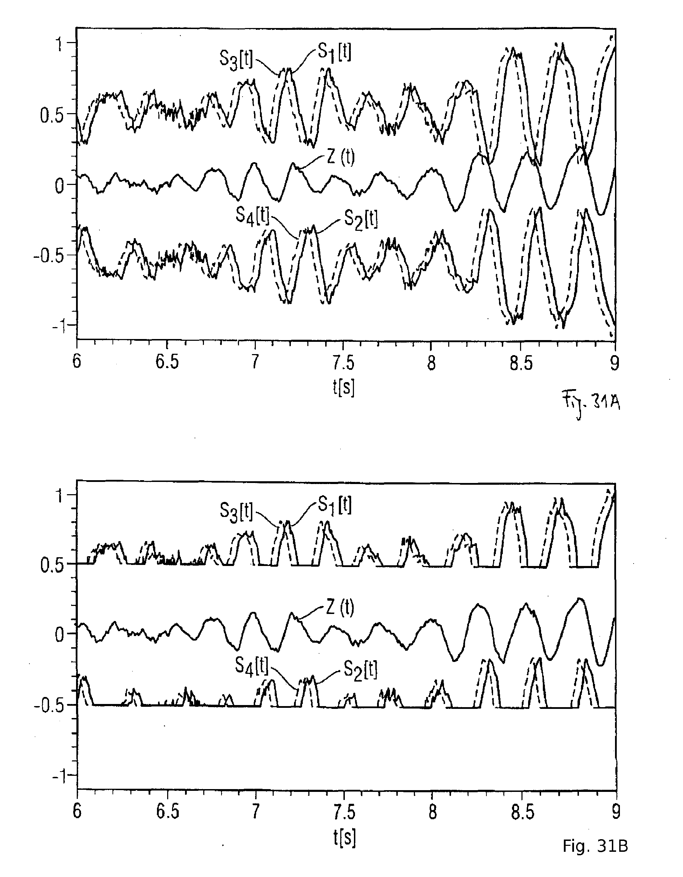

Im Folgenden wird anhand der Fig. 31A und 31B beispielhaft erläutert, wie aus dem Messsignal 26 die Modulationssignale 251 bis 254 gewonnen werden können. Dazu wird zunächst eine Verzögerungszeit $\tau$ festgelegt, die in dem vorliegenden Beispiel zu $\tau = T_{stim}/2$ gesetzt worden ist (andere Werte wie z.B. $\tau = T_{stim}$ oder $\tau = 3T_{stim}/2$ sind ebenfalls möglich). Die Frequenz $f_{stim} = 1/T_{stim}$ kann beispielsweise im Bereich der mittleren Frequenz des Messsignals 26 liegen, z.B. im Bereich von 1 bis 30 Hz, insbesondere im Bereich von 5 bis 20 Hz. Anhand der Verzögerungszeit $\tau$ können für jedes der Modulationssignale 251 bis 254 bestimmte Verzögerungszeiten $\tau_1$, $\tau_2$, $\tau_3$ und $\tau_4$ errechnet werden, beispielsweise anhand folgender Gleichung:

$$\tau_j = \tau \cdot \frac{11 - 2 \cdot (j - 1)}{8} \qquad \text{mit } j = 1, 2, 3, 4$$

(6)

Die Modulationssignale 251 bis 254 können beispielsweise aus dem Messsignal 26 gewonnen werden, indem das Messsignal 26 jeweils um die Verzögerungszeiten $\tau_1$, $\tau_2$, $\tau_3$ bzw. $\tau_4$ verzögert wird:

$$S_j(t) = K \cdot Z(t - \tau_j) \qquad (7)$$

In Gleichung (7) stehen $S_1(t)$, $S_2(t)$, $S_3(t)$ und $S_4(t)$ für die Modulationssignale 251 bis 254 und $Z(t)$ für das Messsignal 26. K ist ein Verstärkungsfaktor, der geeignet gewählt werden kann. Ferner können alle negativen Werte (oder alle Werte ober- oder unterhalb eines bestimmten Schwellwerts) der Modulationssignale $S_1(t)$ bis $S_4(t)$ auf Null gesetzt werden.

Gemäß einer in den Fig. 31A und 31B dargestellten Ausgestaltung werden die Modulationssignale $S_1(t)$ bis $S_4(t)$ nur aus den Verzögerungszeiten $\tau_1$ und $\tau_2$ errechnet, wobei die Modulationssignale $S_1(t)$ und $S_2(t)$ bzw. $S_3(t)$ und $S_4(t)$ jeweils unterschiedliche Polaritäten aufweisen:

$$S_1(t) = K \cdot Z(t - \tau_1) \qquad (8)$$

$$S_2(t) = -K \cdot Z(t - \tau_1) \qquad (9)$$

$$S_3(t) = K \cdot Z(t - \tau_2) \qquad (10)$$

$$S_4(t) = -K \cdot Z(t - \tau_2) \qquad (11)$$

Zur klareren Darstellung sind in den Fig. 31A und 31B die Modulationssignale $S_1(t)$ und $S_3(t)$ um den Wert 0,5 nach oben und die Modulationssignale $S_2(t)$ und $S_4(t)$ um den Wert 0,5 nach unten verschoben worden.

Wie in Fig. 31B gezeigt ist, können alle negativen Werte (oder alle Werte ober- oder unterhalb eines bestimmten Schwellwerts) der Modulationssignale $S_1(t)$ bis $S_4(t)$ auf Null gesetzt werden. Die Generierung der in Fig. 30 gezeigten Modulationssignale 251 bis 254 entspricht der in den Fig. 31A und 31B gezeigten Generierung der Modulationssignale $S_1(t)$ bis $S_4(t)$.

Stimulationseinheiten zur Erzeugung spezifischer taktiler, vibratorischer und/oder thermischer Reize:

Im Folgenden werden Ausgestaltungen der nicht-invasiven ersten Stimulationseinheit 11 zur Erzeugung taktiler, vibratorischer und/oder thermischer erster Reize 21 beschrieben. Derartige Stimulationseinheiten lassen sich auch der deutschen Patentanmeldung Nr. 10 2010 000 390.5 mit dem Titel "Vorrichtung und Verfahren zur Behandlung eines Patienten mit Vibrations-, Tast- und/oder Thermoreizen" entnehmen, die am 11. Februar 2010 beim Deutschen Patent- und Markenamt hinterlegt worden ist. Der gesamte Offenbarungsgehalt der deutschen Patentanmeldung Nr. 10 2010 000 390.5 wird hiermit in die Offenbarung der vorliegenden Anmeldung aufgenommen.

Im Folgenden wird nur auf die Erzeugung der taktilen, vibratorischen und thermischen ersten Reize 21 eingegangen. Es versteht sich, dass diese spezifischen ersten Reize 21 in Kombination mit den unspezifischen zweiten Reizen 22, wie sie oben z.B. im Zusammenhang mit den Fig. 1 bis 5 beschrieben wurden, appliziert werden.

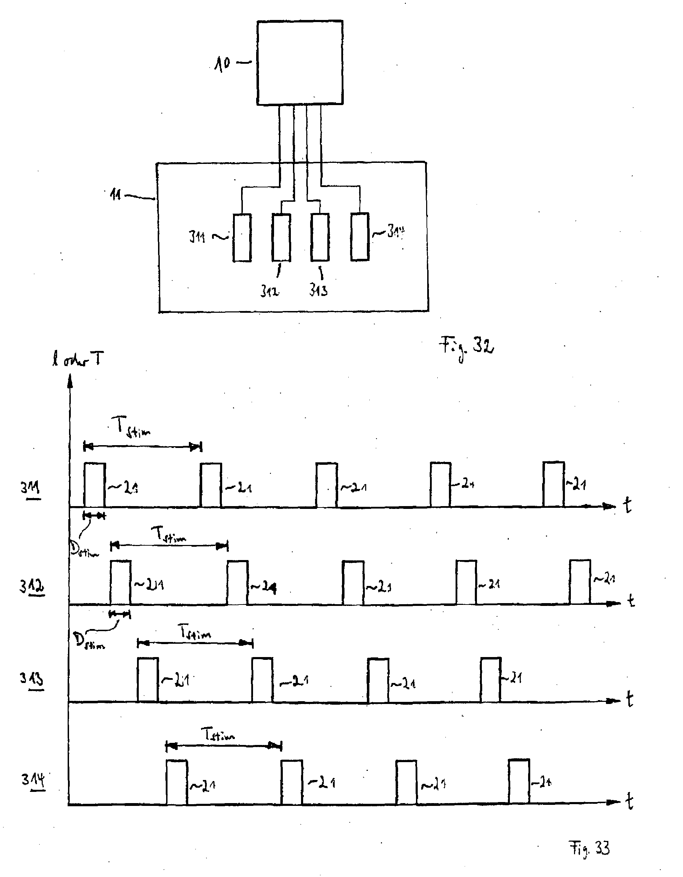

Fig. 32 zeigt schematisch eine Ausgestaltung der ersten Stimulationseinheit 11, die eine Mehrzahl von Stimulationselementen beinhaltet. In dem vorliegenden Ausführungsbeispiel weist die erste Stimulationseinheit 11 vier Stimulationselemente 311, 312, 313, 314 auf, die von der Steuereinheit 10 angesteuert werden. Die in Fig. 32 gezeigte Ausgestaltung ist lediglich beispielhaft zu verstehen. Alternativ zu dieser Ausgestaltung kann die erste Stimulationseinheit 11 eine beliebige Anzahl N (N = 2, 3, 4, 5, 6, 7, 8, 9, 10, 11, 12,...) von Stimulationselementen enthalten.

Die Stimulationselemente 311 bis 314 sind derart ausgestaltet, dass sie auf die Haut des Patienten aufgesetzt werden können. Je nach Erkrankung bzw. betroffenen Körperpartien werden die Stimulationselemente 311 bis 314 in einer geeigneten Anordnung auf der Haut des Patienten befestigt, beispielsweise am Arm, am Bein, an der Hand und/ oder am Fuß des Patienten. Taktile, vibratorische und thermische erste Reize 21 können je nach Krankheitsbild entweder einzeln oder in Kombination auf der Haut verabreicht werden.

Die Mehrzahl von Stimulationselementen 311 bis 314 ermöglicht es, unterschiedliche rezeptive Bereiche der Haut über die einzelnen Stimulationselemente 311 bis 314 zeitlich und räumlich koordiniert zu stimulieren. Die Stimulationselemente 311 bis 314 können so auf der Haut des Patienten angeordnet sein, dass die auf das Hautgewebe applizierten Reize über Nervenleitungen an unterschiedliche Zielgebiete, die z.B. im Rückenmark und/oder im Gehirn liegen, weitergeleitet werden. Folglich können verschiedene Zielgebiete im Rückenmark und/oder Hirn während desselben Stimulationszeitraums mit eventuell unterschiedlichen und/oder zeitversetzten Reizen stimuliert werden.

Ein Stimulationsverfahren, das mit der in Fig. 32 gezeigten ersten Stimulationseinheit 11 durchgeführt werden kann, ist in Fig. 33 schematisch dargestellt. In Fig. 33 sind untereinander die über die Stimulationselemente 311 bis 314 applizierten ersten Reize 21 gegen die Zeit t aufgetragen.

Bei dem in Fig. 33 dargestellten Verfahren appliziert jedes der Stimulationselemente 311 bis 314 den ersten Reiz 21 periodisch an den jeweiligen rezeptiven Bereich der Haut, auf dem das Stimulationselement 311 bis 314 angebracht ist. Die Frequenz $f_{stim}$ = $1/T_{stim}$ ($T_{stim}$ = Periodendauer), mit welcher die von jedem der Stimulationselemente 311 bis 314 erzeugten ersten Reize 21 wiederholt werden, kann im Bereich von 1 bis 60 Hz und insbesondere im Bereich von 30 bis 60 Hz oder im Bereich von 1 bis 30 Hz oder im Bereich 1 bis 20 Hz oder im Bereich von 5 bis 20 Hz liegen, kann aber auch kleinere oder größere Werte annehmen. Die Dauer $D_{stim}$ eines einzelnen ersten Reizes 21 kann insbesondere von der Art des Reizes abhängen. Die in Fig. 33 gezeigte Ordinate hängt ebenfalls von der Art der ersten Reize 21 ab. Bei einem Vibrations- oder Tastreiz kann beispielsweise die Auslenkung I eines Stimulationselements gegen die Zeit t aufgetragen werden, bei einem Thermoreiz kann eine Temperatur T dargestellt werden. Die über die verschiedenen Stimulationselemente 311 bis 314 applizierten ersten Reize 21 können identisch oder aber verschieden sein.

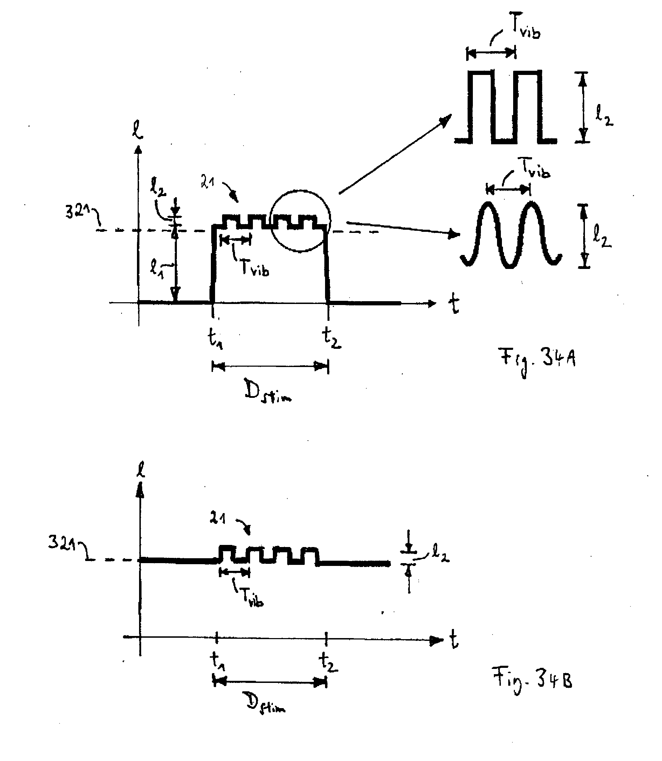

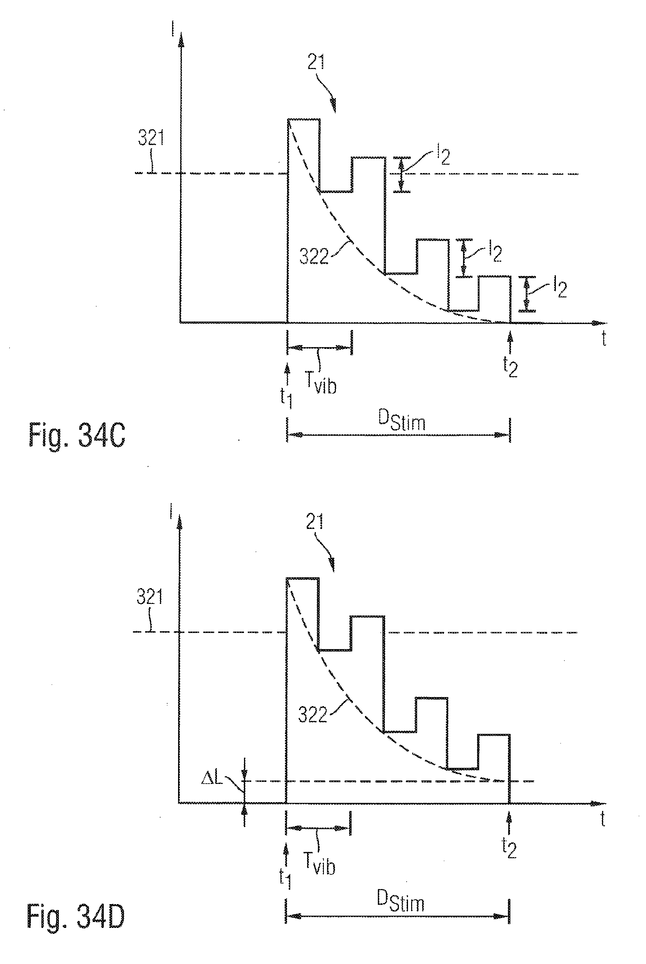

Verschiedene Ausgestaltungen einzelner vibratorischer erster Reize 21 sind in Fig. 34A, 34B, 34C und 34D dargestellt. Dort ist die Auslenkung I eines Stimulationselements gegen die Zeit t aufgetragen. In Fig. 34A wird das Stimulationselement zur Zeit $t_1$ aus seiner Ruheposition ausgelenkt und in die Haut des Patienten eingedrückt. Die Lage der Hautoberfläche ist durch eine gestrichelte Linie 321 dargestellt. Nachdem das Stimulationselement in Kontakt mit der Haut getreten ist, wird ein periodischer Vibrationsreiz mit einer Frequenz $f_{vib}$ = $1/T_{vib}$ im Bereich von 30 bis 300 Hz appliziert ($T_{vib}$ = Periodendauer des Vibrationsreizes). Bei einer Frequenz $f_{vib}$ von 300 Hz kann das Stimulationselement eine Kraft von etwa 2 N ausüben. Die Dauer $D_{stim}$ des Vibrationsreizes 20 kann im Bereich von 10 bis 500 ms liegen. Insbesondere liegt die Stimulationsdauer $D_{stim}$ im Bereich von

$$0 < D_{stim} < \frac{T_{stim}}{N}, \qquad\qquad (12)$$

wobei N die Anzahl der Stimulationselemente ist. Z.B. ergibt sich für $T_{stim}$ = 1 Hz und N = 4 ein Bereich von 10 bis 250 ms für die Stimulationsdauer $D_{stim}$. Es können aber auch zeitlich überlappende Stimuli verwendet werden.

Zur Zeit $t_2$ wird das Stimulationselement wieder in seine Ruheposition verbracht, wo es keinen Kontakt zur Haut hat. Wie in Fig. 34A gezeigt kann der vibratorische erste Reiz 21 ein rechteckförmiger oder sinusförmiger Reiz sein, er kann aber auch andere Formen haben. Die in Fig. 34A gezeigte Auslenkung $I_1$ zur Eindrückung des Stimulationselements in die Haut kann im Bereich von 0,5 bis 3 mm liegen. Die Auslenkung $I_2$ des Stimulationselements während der Vibration kann zwischen 0,1 und 0,5 mm betragen.

In Fig. 34B ist eine Variation des in Fig. 34A gezeigten vibratorischen ersten Reizes 21 dargestellt. Bei der in Fig. 34B gezeigten Ausgestaltung steht das Stimulationselement stets in Kontakt mit der Haut des Patienten. Während des Stimulationszeitraums $D_{stim}$ wird ein wie oben beschriebener vibratorischer erster Reiz 21 appliziert.

Eine weitere Variation des vibratorischen ersten Reizes 21 ist in Fig. 34C dargestellt. Im Unterschied zum vibratorischen ersten Reiz 21 aus Fig. 34A wird das Stimulationselement bereits während des Stimulationszeitraums $D_{stim}$ wieder zurückgefahren, so dass die Vibrationen mit zunehmender Zeitdauer weniger in die Haut eindrücken und sich das Stimulationselement schließlich vollständig von der Haut löst. Beispielsweise kann das Zurückfahren des Stimulationselements entlang einer linearen oder nicht-linearen, z.B. exponentiellen, Kurve 322 erfolgen, welcher die Vibrationen $f_{vib}$ des Stimulationselements überlagert sind. In dem in Fig. 34C gezeigten Beispiel reicht die abfallende Flanke eines jeden Pulses bis auf die Kurve 322 herunter. Der sich daran anschließende Puls hat eine fest vorgegebene Höhe $I_2$, d.h. die ansteigende Flanke eines jeden Pulses hat die Höhe $I_2$.

Eine Variation des vibratorischen ersten Reizes 21 aus Fig. 34C ist in Fig. 34D gezeigt. Dort geht die Kurve 322 nicht bis auf die Nulllinie (I = 0) zurück, sondern hat einen fest vorgegebenen Offset $\Delta L$ von der Nulllinie.

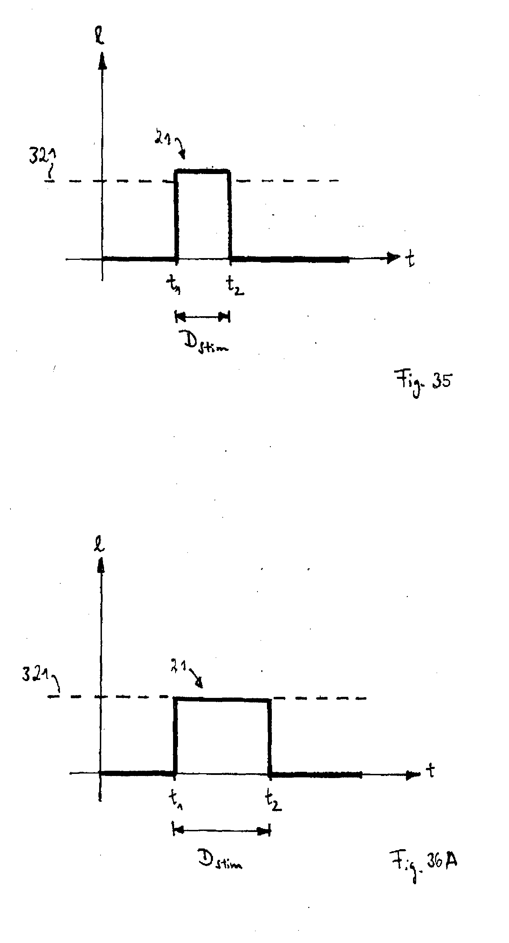

Eine Ausführungsform eines taktilen ersten Reizes 21 ist in Fig. 35 gezeigt. Das Stimulationselement wird zur Zeit $t_1$ in die Haut des Patienten eingedrückt, verweilt dort für die Stimulationsdauer $D_{stim}$ und wird zur Zeit $t_2$ wieder zurückgefahren. Die Stimulationsdauer $D_{stim}$ liegt bei einem taktilen ersten Reiz 21 im Bereich von 10 bis 500 ms. Insbesondere liegt die Stimulationsdauer $D_{stim}$ in dem oben in (12) angegebenen Bereich, es können aber auch zeitlich überlappende Stimuli verwendet werden.

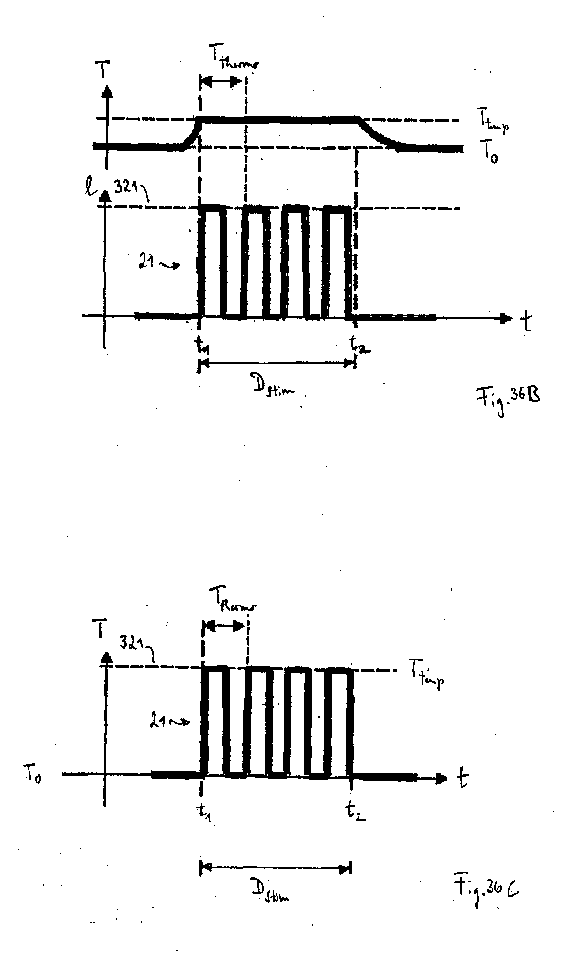

Verschiedene Ausführungsformen einzelner thermischer erster Reize 21 sind in Fig. 36A, 36B und 36C dargestellt. Bei den in Fig. 36A und 36B gezeigten Ausgestaltungen wird ein Stimulationselement auf eine Temperatur $T_{temp}$ erhitzt oder gekühlt. Wie in Fig. 36B gezeigt ist, kann die Temperatur $T_{temp}$ erst kurz vor der Applikation des thermischen ersten Reizes 21 erzeugt werden. In diesem Fall hat das Stimulationselement während der Stimulationspausen eine Temperatur $T_0$, welche z.B. der Raumtemperatur entspricht. Alternativ kann das Stimulationselement auf einer konstanten Temperatur $T_{temp}$ gehalten werden.

Bei der Ausgestaltung nach Fig. 36A wird das erhitzte oder gekühlte Stimulationselement zur Zeit $t_1$ auf die Haut des Patienten gebracht und verbleibt dort für die gesamte Stimulationsdauer $D_{stim}$. Im Unterschied dazu wird bei der Ausgestaltung nach Fig. 36B das Stimulationselement während der Stimulationsdauer $D_{stim}$ periodisch mit einer Frequenz $f_{thermo}$ zur Haut verbracht und wieder entfernt. Die Frequenz $f_{thermo} = 1/T_{thermo}$ kann im Bereich von 1 bis 10 Hz liegen ($T_{thermo}$ = Periodendauer des Thermoreizes).

Der in Fig. 36C gezeigte thermische erste Reiz 21 entspricht im Wesentlichen dem Thermoreiz 21 aus Fig. 36B. Der Unterschied ist, dass der Thermoreiz 21 aus Fig. 36C berührungslos erzeugt wird. Hier wird die Stimulationstemperatur $T_{temp}$ durch elektromagnetische Strahlung, beispielsweise Infrarotlicht, erzeugt. Ferner wird die elektromagnetische Strahlung periodisch mit der Frequenz $f_{thermo} = 1/T_{thermo}$ variiert (z.B. durch An- und Ausschalten eines Infrarotstrahlers).

Bei thermischen ersten Reizen 21 liegt die Stimulationsdauer $D_{stim}$ im Bereich von 10 bis 500 ms. Insbesondere liegt die Stimulationsdauer $D_{stim}$ in dem oben in (12) angegebenen Bereich, es können aber auch zeitlich überlappende Stimuli verwendet werden. Die Temperatur $T_{temp}$ kann von 22 bis 42 °C betragen. Die Temperatur $T_0$ ist in der Regel die Körpertemperatur des Patienten. Die Frequenz $f_{thermo}$ kann zwischen 1 und 10 Hz liegen, kann aber auch außerhalb dieses Bereichs liegen.

Es ist auch denkbar, dass ein einzelner erster Reiz 21 mehrere Reizarten umfasst. Beispielsweise kann der in Fig. 34A gezeigte vibratorische erste Reiz 21 gleichzeitig ein Thermoreiz sein, sofern das den Reiz ausübende Stimulationselement entsprechend erwärmt oder gekühlt ist. Ferner ist der vibratorische erste Reiz 21 aus Fig. 34A gleichzeitig ein Tastreiz (durch das Auftreffen des Stimulationselement auf die Haut werden Tastrezeptoren aktiviert).

Die von den Stimulationseinheiten 311 bis 314 applizierten ersten Reize 21 werden von in oder unter der Haut gelegenen Rezeptoren aufgenommen und an das Nervensystem weitergeleitet. Zu diesen Rezeptoren zählen beispielsweise Merkel-Zellen, Ruffini-Körperchen, Meissner-Körperchen und Haarfollikelrezeptoren, die insbesondere als Rezeptoren für die taktilen ersten Reize 21 wirken. Die vibratorischen ersten Reize 21 zielen vorwiegend auf die Tiefensensibilität ab. Die vibratorischen ersten Reize 21 können von in der Haut, den Muskeln, dem Subkutangewebe und/ oder den Sehnen des Patienten gelegenen Rezeptoren aufgenommen werden. Als Rezeptoren für die vibratorischen ersten Reize 21 seien beispielhaft die Vater-Pacini-Körperchen genannt, die Vibrationsempfindungen und Beschleunigungen vermitteln. Die thermischen ersten Reize 21 werden von den Thermorezeptoren der Haut aufgenommen. Dies sind Warmrezeptoren (auch Wärmerezeptoren, Warmsensoren oder Wärmesensoren genannt) und Kaltsensoren (auch Kältesensoren, Kaltrezeptoren oder Kälterezeptoren genannt). In der Haut des Menschen liegen die Kaltsensoren mehr oberflächlich, die Warmrezeptoren etwas tiefer.

Die von den Stimulationselementen 311 bis 314 generierten ersten Reize 21 sind derart ausgestaltet, dass sie, wenn sie von den entsprechenden Rezeptoren aufgenommen werden und über die Nervenleitungen zu einer Neuronenpopulation im Gehirn oder Rückenmark mit einer krankhaft synchronen und oszillatorischen Aktivität geleitet werden, in der Neuronenpopulation ein Zurücksetzen der Phase der neuronalen Aktivität der stimulierten Neuronen bewirken. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Neuronenpopulation mittels einer gezielten Stimulation kontrolliert.

Ferner ist es aufgrund der Mehrzahl von Stimulationselementen möglich, die krankhafte Neuronenpopulation an unterschiedlichen Stellen zu stimulieren. Die an unterschiedlichen Stellen der Haut applizierten ersten Reize 21 werden nämlich an unterschiedliche Stellen im Gehirn oder Rückenmark weitergeleitet. Dies ermöglicht es, die Phase der neuronalen Aktivität der krankhaften Neuronenpopulation an den unterschiedlichen Stimulationsstellen zu unterschiedlichen Zeitpunkten zurückzusetzen. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb einer Subpopulation sind die Neuronen weiterhin synchron und feuern auch weiterhin mit derselben pathologischen Frequenz, aber jede der Subpopulationen weist bezüglich ihrer neuronalen Aktivität die Phase auf, die ihr durch den Stimulationsreiz aufgezwungen wurde.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d.h. die komplette Desynchronisation, ist somit nach der Applikation der ersten Reize 21 nicht sofort vorhanden, sondern stellt sich meist innerhalb weniger Perioden oder gar in weniger als einer Periode der pathologischen Aktivität ein.

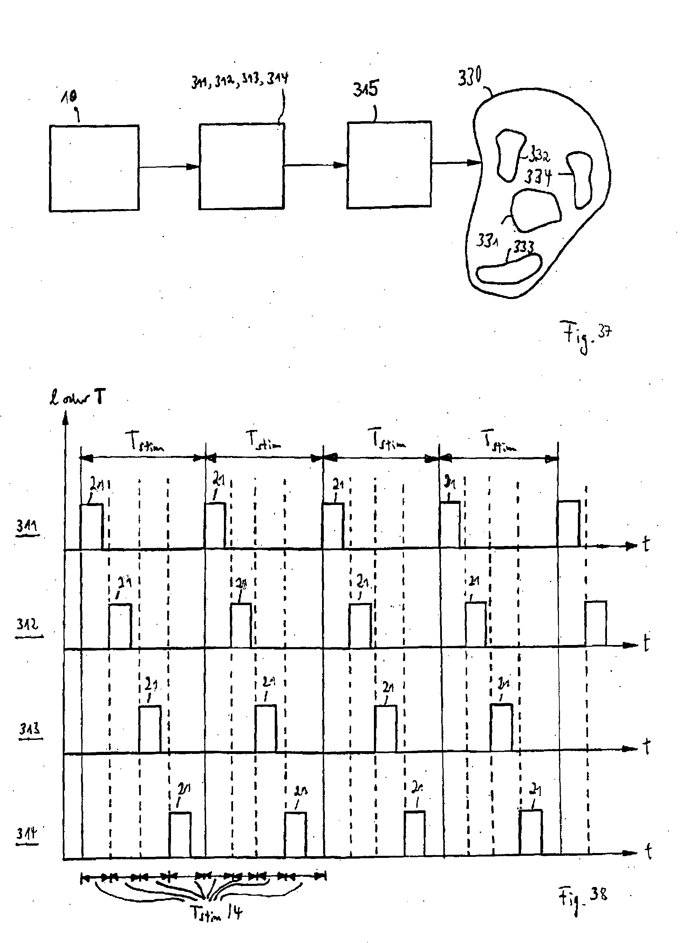

Schematisch ist die Stimulation mehrerer Subpopulationen einer krankhaft aktiven Neuronenpopulation 330 mit Hilfe der ersten Stimulationseinheit 11 in Fig. 37 dargestellt. Über die Stimulationselemente 311 bis 314 der ersten Stimulationseinheit 11 werden an unterschiedlichen Stellen der Haut 315 die jeweiligen Rezeptoren mit taktilen und/oder vibratorischen und/oder thermischen ersten Reizen 21 stimuliert. Die von den Stimulationselementen 311, 312, 313 und 314 applizierten ersten Reize 21 werden an unterschiedliche Subpopulationen 331, 332, 333 bzw. 334 der Neuronenpopulation 330 weitergeleitet (Reize von Stimulationselement 311 zu Subpopulation 331, Reize von Stimulationselement 312 zu Subpopulation 332, Reize von Stimulationselement 313 zu Subpopulation 333 und Reize von Stimulationselement 314 zu Subpopulation 334) und resetten die Phasen dieser Subpopulationen zu jeweils unterschiedlichen Zeitpunkten, wodurch eine Desynchronisation der gesamten Neuronenpopulation 330 erzielt wird.

Die gezielte Stimulation bestimmter Bereiche des Gehirns oder Rückenmarks wird durch die somatotope Zuordnung von Körperregionen zu diesen Bereichen ermöglicht. Beispielsweise können die Stimulationselemente 311 bis 314 am Fuß, Unterschenkel und Oberschenkel oder aber an der Hand, dem Unteram und Oberam des Patienten angebracht werden. Aufgrund der somatotopischen Gliederung der Nervenleitungsbahnen werden durch die an den jeweiligen Stellen applizierten Reize unterschiedliche Neuronen stimuliert. Die somatotope Zuordnung von Hautstellen zu Bereichen des Gehirns ist beispielsweise in A. Benninghoff et al.: "Lehrbuch der Anatomie des Menschen. Dargestellt unter Bevorzugung funktioneller Zusammenhänge. 3. Bd. Nervensystem, Haut und Sinnesorgane", Urban und Schwarzenberg, München 1964, beschrieben.

Um durch zeitversetztes Zurücksetzen der Phasen der Subpopulationen 331 bis 334 der krankhaft synchronen Neuronenpopulation 330 eine Desynchronisation der gesamten Neuronenpopulation 330 zu erzielen, kann auf verschiedene Art und Weise vorgegangen werden. Beispielsweise können die ersten Reize 21, die ein Zurücksetzen der Phase von Neuronen bewirken, zeitversetzt über die unterschiedlichen Stimulationselemente 311 bis 314 an die jeweiligen rezeptiven Felder der Haut abgegeben werden. Des Weiteren können die Reize z.B. phasenversetzt oder mit unterschiedlicher Polarität appliziert werden, so dass sie im Ergebnis auch zu einem zeitversetzten Zurücksetzen der Phasen der unterschiedlichen Subpopulationen 331 bis 334 führen.

Ein für die oben beschriebenen Zwecke geeignetes Stimulationsverfahren ist in Fig. 38 schematisch dargestellt. In Fig. 38 sind untereinander die über die Stimulationselemente 311 bis 314 applizierten ersten Reize 21 gegen die Zeit t aufgetragen. Als erste Reize 21 können beispielsweise die in den Fig. 34A bis 36C dargestellten Vibrations-, Tast- und Thermoreize verwendet werden. Das in Fig. 38 gezeigte Diagramm ist in sich periodisch wiederholende erste Zeitabschnitte der Länge $T_{stim}$ unterteilt. Die Frequenz $f_{stim} = 1/T_{stim}$, mit welcher die ersten Zeitabschnitte der Länge $T_{stim}$ wiederholt werden, kann im Bereich von 1 bis 60 Hz und insbesondere im Bereich von 30 bis 60 Hz oder im Bereich von 1 bis 30 Hz oder im Bereich 1 bis 20 Hz oder im Bereich von 5 bis 20 Hz liegen, kann aber auch kleinere oder größere Werte annehmen.

Die ersten Zeitabschnitte der Länge $T_{stim}$ sind ferner in zweite Zeitabschnitte der Länge $T_{stim}/4$ unterteilt. Bei einer Stimulation über N Stimulationseinheiten könnten die ersten Zeitabschnitte in N zweite Zeitabschnitte der Länge $T_{stim}/N$ unterteilt sein.

Gemäß einer Ausgestaltung generiert jedes der Stimulationselemente 311 bis 314 innerhalb eines ersten Zeitabschnitts nicht mehr als einen ersten Reiz 21. In aufeinander folgenden zweiten Zeitabschnitten können erste Reize 21 von unterschiedlichen Stimulationselementen 311 bis 314 generiert werden.

Bei der in Fig. 38 dargestellten Ausgestaltung appliziert jedes der Stimulationselemente 311 bis 314 einen ersten Reiz 21 streng periodisch mit der Frequenz $f_{stim}$. Die Verabreichung der ersten Reize 21 über unterschiedliche Stimulationselemente 311 bis 314 erfolgt mit einer zeitlichen Verzögerung zwischen den einzelnen Stimulationselementen 311 bis 314 um $T_{stim}/4$.

Im Fall von N Stimulationselementen kann die zeitliche Verzögerung zwischen jeweils zwei aufeinander folgenden ersten Reizen 21 beispielsweise im Bereich eines N-tels der Periode $1/f_{stim}$ liegen, d.h. $1/(N \times f_{stim}) = T_{stim}/N$, d.h. insbesondere vergeht zwischen den Startzeitpunkten von zwei aufeinander folgenden ersten Reizen 21 die Zeit $T_{stim}/N$.

Die Frequenz $f_{stim}$ kann beispielsweise im Bereich der mittleren Frequenz der krankhaft rhythmischen Aktivität des Ziel-Netzwerks liegen. Bei Erkrankungen, bei denen eine gesteigerte neuronale Synchronisation vorliegt, liegt die mittlere Frequenz typischerweise im Bereich von 1 bis 30 Hz, kann aber auch außerhalb dieses Bereichs liegen. Hierbei ist zu beachten, dass die Frequenz, mit welcher die betroffenen Neuronen synchron feuern, üblicherweise nicht konstant ist, sondern durchaus Variationen aufweisen kann und darüber hinaus bei jedem Patienten individuelle Abweichungen zeigt.

Von dem in Fig. 38 gezeigten streng periodischen Stimulationsmuster kann auf unterschiedliche Art und Weise abgewichen werden. Beispielsweise braucht die zeitliche Verzögerung $T_{stim}$ zwischen aufeinander folgenden und von derselbem Stimulationselement erzeugten ersten Reizen 21 nicht stets gleich groß sein, sondern kann im Bereich von $\pm$ 10% oder $\pm$ 5% oder $\pm$ 3% um $T_{stim}$ herum variieren. Ferner kann auch der Zeitabstand zwischen zwei aufeinander folgenden und von verschiedenen Stimulationselementen erzeugten ersten Reizen 21 im Bereich von $\pm$ 10% oder $\pm$ 5% oder $\pm$ 3% um $T_{stim}/N$ herum variieren. Es kann durchaus vorgesehen sein, dass die zeitlichen Abstände zwischen den einzelnen ersten Reizen 21 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

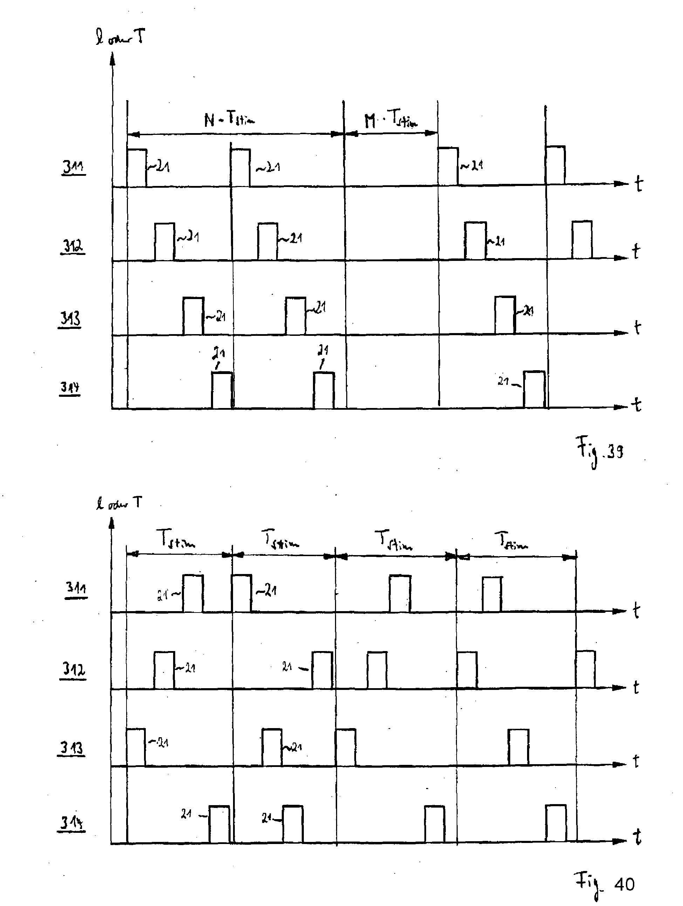

Des Weiteren können während der Applikation der ersten Reize 21 Pausen vorgesehen werden, während derer keine Stimulation erfolgt. Eine solche Pause ist beispielhaft in Fig. 39 gezeigt. Die Pausen können beliebig lang gewählt werden und insbesondere ein ganzzahliges Vielfaches der Periode $T_{stim}$ betragen. Ferner können die Pausen nach einer beliebigen Anzahl von Stimulationen eingehalten werden. Z.B. kann eine Stimulation während N aufeinander folgender Perioden der Länge $T_{stim}$ durchgeführt werden und anschließend eine Pause während M Perioden der Länge $T_{stim}$ ohne Stimulation eingehalten werden, wobei N und M kleine ganze Zahlen sind, z.B. im Bereich von 1 bis 10. Dieses Schema kann entweder periodisch fortgesetzt werden oder stochastisch und/oder deterministisch oder gemischt stochastisch-deterministisch modifiziert werden.

Eine weitere Möglichkeit, von dem in Fig. 38 gezeigten streng periodischen Stimulationsmuster abzuweichen, besteht darin, die zeitliche Abfolge der einzelnen ersten Reize 21 stochastisch oder deterministisch oder gemischt stochastisch-deterministisch zu variieren.

Des Weiteren kann pro Periode $T_{stim}$ (oder auch in anderen Zeitschritten) die Reihenfolge, in welcher die Stimulationselemente 311 bis 314 die ersten Reize 21 applizieren, variiert werden, wie dies beispielhaft in Fig. 40 gezeigt ist. Diese Randomisierung kann stochastisch oder deterministisch oder gemischt stochastisch-deterministisch erfolgen.

Die in Fig. 40 gezeigte Randomisierung kann mit der in Fig. 39 gezeigten Stimulationsform kombiniert werden. Beispielsweise kann in jedem der N aufeinander folgenden Stimulationszeitabschnitte der Länge $T_{stim}$ eine erneute Randomisierung durchgeführt werden oder aber es erfolgt nach jeder Pause der Länge M x $T_{stim}$ eine Randomisierung und innerhalb der darauf folgenden N Stimulationszeitabschnitte bleibt die Reihenfolge, in welcher die Stimulationselemente 311 bis 314 die ersten Reize 21 applizieren, konstant.

Ferner kann pro Periode $T_{stim}$ (oder in einem anderen Zeitintervall) nur eine bestimmte Anzahl von Stimulationselementen 311 bis 314 zur Stimulation herangezogen werden und die an der Stimulation beteiligten Stimulationselemente können in jedem Zeitintervall variiert werden. Auch diese Variation kann stochastisch oder deterministisch oder gemischt stochastisch-deterministisch erfolgen.

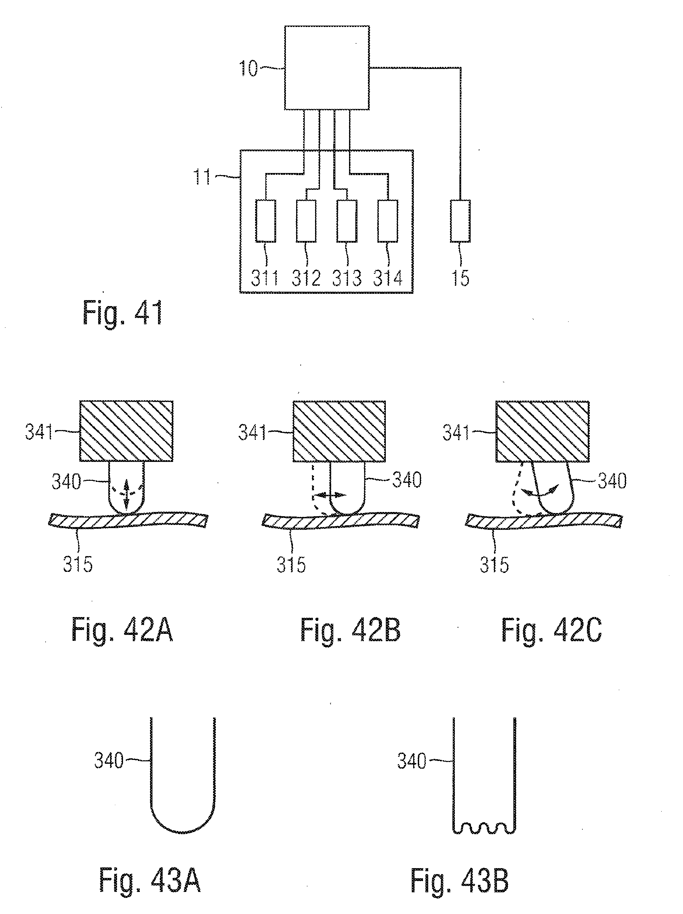

Die erste Stimulationseinheit 11 kann beispielsweise in einem "open loop"-Modus betrieben werden, bei welchem die Steuereinheit 10 die Stimulationselemente 311 bis 314 derart ansteuert, dass diese vorgegebene erste Reize 21 erzeugen, die an das Hautgewebe abgegeben werden. Des Weiteren kann die erste Stimulationseinheit 11 zusammen mit der Steuereinheit 10 auch zu einem in Fig. 41 schematisch dargestellten "closed loop"-System weitergebildet werden. Bei dieser Ausführung ist zusätzlich noch eine Messeinheit 15 vorgesehen, welche am Patienten aufgenommene Messsignale bereitstellt und diese an die Steuereinheit 10 weiterleitet. Bei der Messeinheit 15 kann es sich um nicht-invasive oder invasive Sensoren handeln (vgl. obige Beschreibung im Zusammenhang mit Fig. 3).

Hinsichtlich des Zusammenwirkens der Steuereinheit 10 mit der Messeinheit 15 sind verschiedene Ausgestaltungen denkbar.

Beispielsweise kann - wie oben beschrieben wurde - anhand der Messsignale zwischen dem ersten Betriebsmodus, der Lernphase, und dem zweiten Betriebsmodus, der eigentlichen Stimulationsphase, gewechselt werden. Ferner können von der Steuereinheit 10 anhand der Ausprägung der krankhaften Merkmale Parameter der ersten Reize 21, z.B. eine bestimmte Frequenz $f_{vib}$ oder Eindrücktiefe $l_2$ im Fall von Vibrationsreizen, eingestellt werden.

Des Weiteren kann vorgesehen sein, dass die von der Messeinheit 15 aufgenommenen Messsignale direkt oder gegebenenfalls nach einem oder mehreren Verarbeitungsschritten in taktile, vibratorische und/oder thermische erste Reize 21 umgesetzt werden und von der ersten Stimulationseinheit 11 appliziert werden. Beispielsweise können die Messsignale verstärkt und gegebenenfalls nach mathematischer Verrechnung (z.B. nach Mischung der Messsignale) mit einer Zeitverzögerung und linearen und/oder nichtlinearen Verrechnungsschritten als Steuersignale 23 in den Steuereingang der ersten Stimulationseinheit 11 eingespeist werden. Der Verrechnungsmodus wird hierbei so gewählt, dass der krankhaften neuronalen Aktivität entgegengewirkt wird und die taktilen, vibratorischen und/oder thermischen ersten Reize 21 mit abnehmender krankhafter neuronaler Aktivität ebenfalls verschwinden oder zumindest deutlich in ihrer Stärke reduziert werden.

Fig. 42A bis 42C zeigen schematisch verschiedene Möglichkeiten zur Realisierung eines Stimulationselements zur Erzeugung von taktilen und/oder vibratorischen ersten Reizen 21, wie sie in den Fig. 34A bis 35 gezeigt sind. Beispielsweise kann das Stimulationselement als Stab 340 (oder ein anderer Körper) ausgestaltet sein, mit dessen einem Ende die Haut 315 des Patienten stimuliert wird. Angetrieben wird das Stimulationselement 340 von einem elektromechanischen Wandler 341 (oder Aktor oder Aktuator), der elektrische Energie in eine Bewegung des Stimulationselements 340 umsetzt. Als elektromechanische Wandler 341 eignen sich beispielsweise Gleichstrommotoren, Schwingspulen (engl.: voice coil), piezoelektrische Wandler oder aus elektroaktiven Polymeren (EAP) aufgebaute Wandler, die beim Anlegen einer elektrischen Spannung ihre Form ändern.

Die elektromechanischen Wandler 341 können so ausgelegt sein, dass das Stimulationselement 340 senkrecht zur Hautoberfläche ausgelenkt wird (vgl. Fig. 42A) oder parallel dazu (vgl. Fig. 42B). Die Bewegung des Stimulationselements 340 kann aber auch auf beliebigen anderen Bahnen erfolgen. Als Beispiel dafür ist in Fig. 42C eine pendelförmige Auslenkung des Stimulationselements 340 dargestellt.

Das Ende des Stimulationselements 340, das in Berührung mit der Hautoberfläche kommt und letztlich die Reize erzeugt, kann beispielsweise im Wesentlichen die Form einer Halbkugel aufweisen (vgl. Fig. 43A) oder eine noppenartige Oberfläche haben (vgl. Fig. 43B) oder eine andere geeignete Form haben.

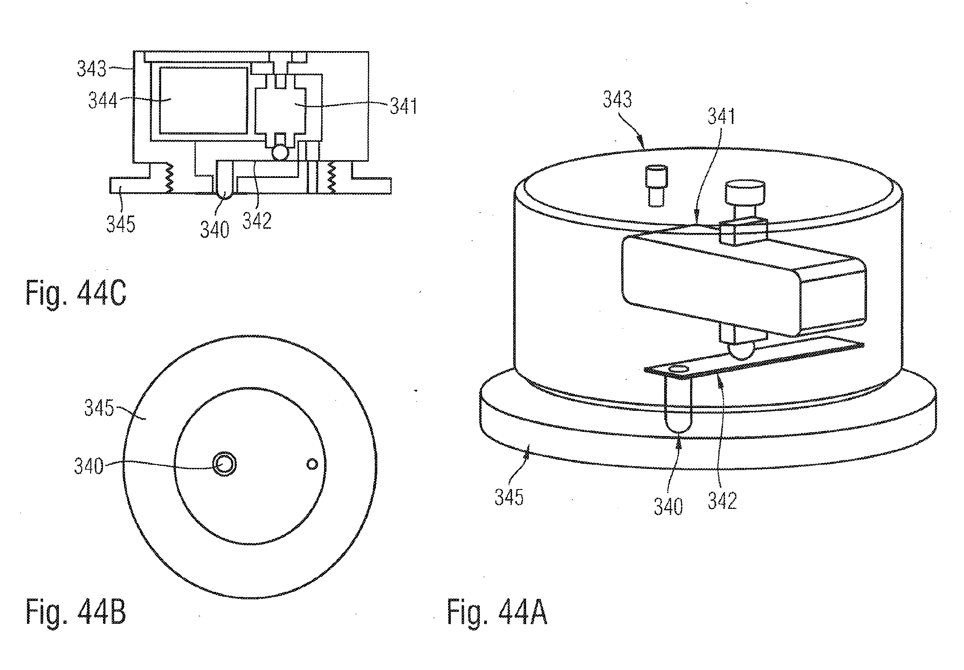

In den Fig. 44A bis 44C ist eine Ausgestaltung eines Stimulationselements zur Applikation von taktilen und/oder vibratorischen ersten Reizen 21 in Durchsicht (vgl. Fig. 44A), Draufsicht von unten (vgl. Fig. 44B) und im Querschnitt (vgl. Fig. 44C) dargestellt. Das vorliegende Stimulationselement enthält einen Piezoaktuator 341 als elektromechanischen Wandler. Da die Auslenkung des Piezoaktuators 341 für die beabsichtigten Zwecke nicht ausreichend ist, kann ein Mechanismus zur Verstärkung der Auslenkung des Piezoaktuators 341 vorgesehen sein. Beispielhaft ist hier ein Hebelarm 342 gezeigt, der die Bewegung des Piezoaktuators 341 verstärkt. Der Hebelarm ist vorliegend eine längliche Biegefeder 342, die mit ihrem einen Ende am Gehäuse 343 des Stimulationselements befestigt ist und an deren anderem Ende das Stimulationselement 340 angebracht ist. Der Piezoaktuator 341 drückt auf die Oberseite der Biegefeder 342 und das an der Unterseite der Biegefeder 342 angebrachte Stimulationselement 340 folgt der Auslenkung des Piezoaktuators 341 mit einer aufgrund der geometrischen Anordnung verstärkten Amplitude und appliziert die Vibrationsund/oder Tastreize auf die Haut des Patienten. Die Unterseite des Stimulationselements 340, die mit der Haut in Berührung kommt, kann verschiedene Geometrien und Abmessungen aufweisen. Beispielsweise kann das Stimulationselement 340 an seiner Unterseite flach, rund oder ungleichförmig sein.

In dem Gehäuse 343 des Stimulationselements, das den Piezoaktuator 341 und den Verstärkungsmechanismus beherbergt, kann ferner ein Raum 344 für Elektronik und Verbindungsanschlüsse vorgesehen sein. Außerdem ist an der Unterseite des Gehäuses 343 ein Verstellring 345 angebracht, der mit dem Gehäuse 343 über ein Gewinde verbunden ist und der eine Verstellung der Höhe ermöglicht, um die das Stimulationselement 340 in seiner Ruheposition von der Unterseite der Stimulationseinheit hervorsteht. Während des Betriebs sitzt das Stimulationselement mit seiner Unterseite auf der Haut des Patienten und ist beispielsweise mit einer geeigneten Manschette am Körper des Patienten befestigt. Zusätzlich oder alternativ zu der Manschette könnte das Stimulationselement noch mit einem ein- oder doppelseitigen medizinischen Klebeband an der Haut des Patienten befestigt sein. Das Gehäuse 343 schützt den Patienten vor möglichen Gefahren, wie z.B. elektrischer Spannung.

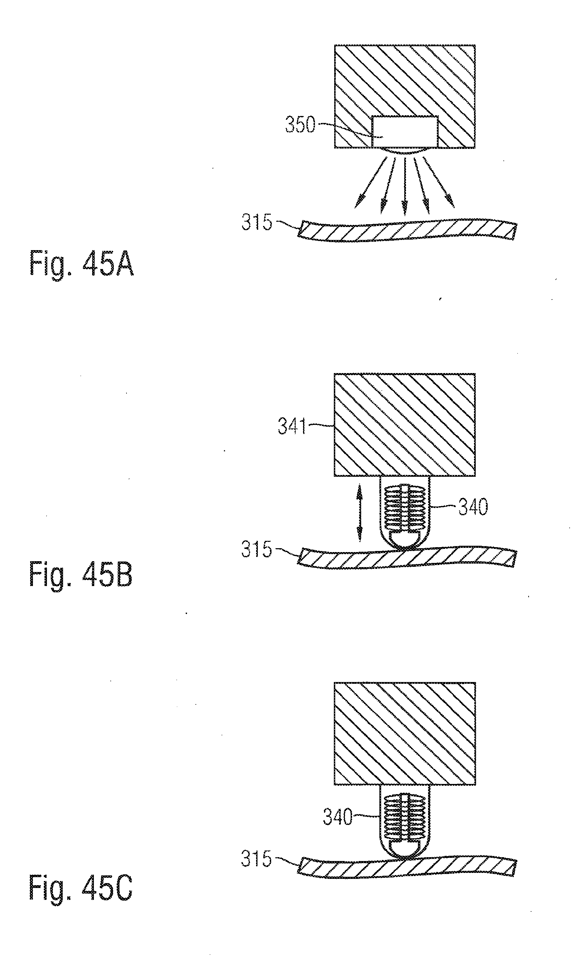

Fig. 45A bis 45C zeigen schematisch verschieden ausgestaltete Stimulationselemente zur Erzeugung von thermischen ersten Reizen 21, wie sie in den Fig. 36A bis 36C dargestellt sind. Die in Fig. 45A dargestellte Stimulationseinheit arbeitet kontaktlos und bewirkt durch das Licht einer Infrarot-LED 350 eine Erwärmung der Haut.

Stimulationselemente, die durch Berührung der Hautoberfläche Thermoreize applizieren, sind in den Fig. 45B und 45C gezeigt. Das in Fig. 45B gezeigte Stimulationselement enthält mit einem elektromechanischen Wandler 341 und einem stabförmigen Stimulationselement 340 im Wesentlichen die gleichen Bauelemente wie das Stimulationselement aus Fig. 42A. Zusätzlich weist das Stimulationselement aus Fig. 45B ein Heizund/oder Kühlelement auf (z.B. in Form einer Heizschleife), welches das Stimulationselement heizt oder kühlt. Die thermischen ersten Reize 21 werden durch die Bewegungen des Stimulationselements 340 erzeugt, bei welchen das Stimulationselement 340 wiederholt in Kontakt mit der Haut 315 kommt und wieder entfernt wird. Die Temperatur des Stimulationselements 340 kann während der gesamten Stimulation konstant sein.

Alternativ kann das beheizbare bzw. kühlbare Stimulationselement 340 wie in Fig. 45C gezeigt während des gesamten Stimulationszeitraums in Kontakt mit der Haut 315 des Patienten stehen. Die Thermoreize werden in diesem Fall durch eine zeitliche Variation der Temperatur des Stimulationselements 340 generiert. Ein elektromechanischer Wandler ist bei dieser Ausgestaltung nicht zwingend erforderlich.

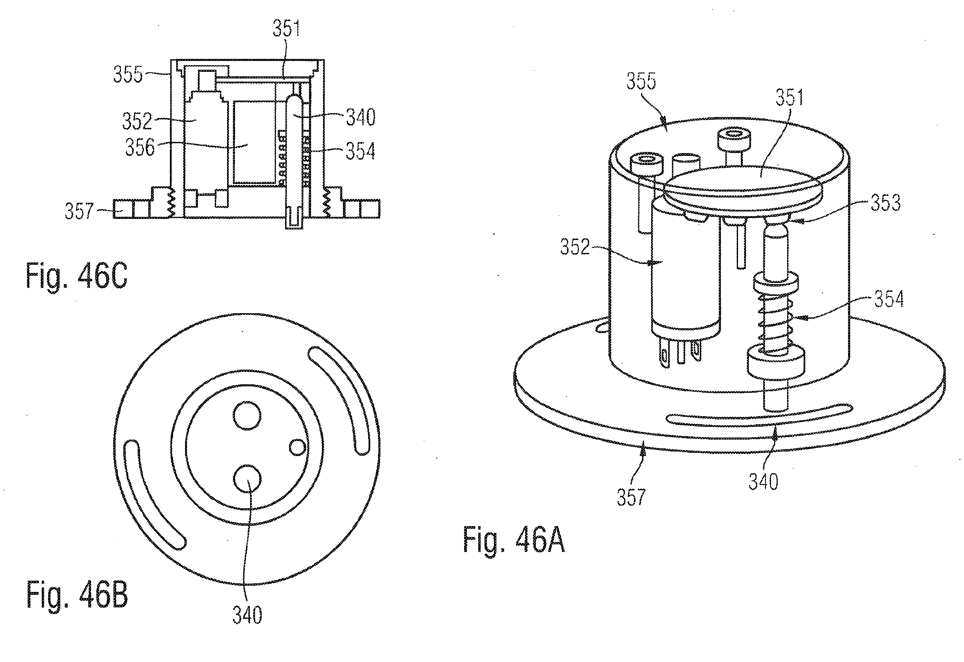

In den Fig. 46A bis 46C ist eine Ausgestaltung eines Stimulationselements zur Applikation von thermischen ersten Reizen 21 in Durchsicht (vgl. Fig. 46A), Draufsicht von unten (vgl. Fig. 46B) und im Querschnitt (vgl. Fig. 46C) dargestellt. Das Stimulationselement enthält ein stabförmiges Stimulationselement 340, dessen unteres Ende beheizbar und/oder kühlbar ist. An seinem oberen Ende wird das Stimulationselement 340 von einer Nockenscheibe 351 angetrieben. Während der Stimulation versetzt ein Gleichstrommotor 352 die Nockenscheibe 351 in Rotation. Durch die an der Unterseite der Nockenscheibe 351 angebrachten Nocken 353 wird das Stimulationselement 340 nach unten hin ausgelenkt. Eine Rückstellfeder 354 sorgt dafür, dass das Stimulationselement 340 anschließend wieder in seine Ausgangslage zurückkehrt. Durch diesen Mechanismus wird die Rotationsbewegung der Nockenscheibe 351 in eine lineare Bewegung des Stimulationselements 340 umgewandelt. Wie oben beschrieben kann das Stimulationselement 340 entweder für eine gewisse Zeit mit der Haut des Patienten in Kontakt stehen oder aber das Stimulationselement 340 wird durch eine Rotation der Nockenscheibe 351 zyklisch auf die Haut gebracht und wieder entfernt.

Die Bauteile des Stimulationselements können in ein Gehäuse 355 eingebracht sein. In dem Gehäuse 355 kann ein Raum 356 für Elektronik und Verbindungsanschlüsse vorgesehen sein. Außerdem kann an der Unterseite des Gehäuses 355 ein Verstellring 357 angebracht sein, der mit dem Gehäuse 355 über ein Gewinde verbunden ist und der eine Verstellung der Höhe ermöglicht, um die das Stimulationselement 340 in seiner Ruheposition von der Unterseite der Stimulationseinheit hervorsteht (das Stimulationselement 340 kann aufgrund des Verstellrings in seiner Ruheposition auch vollständig oberhalb der Unterseite des Verstellrings liegen). Während des Betriebs sitzt das Stimulationselement mit seiner Unterseite auf der Haut des Patienten und ist beispielsweise mit einer geeigneten Manschette am Körper des Patienten befestigt. Zusätzlich oder alternativ zu der Manschette könnte das Stimulationselement noch mit einem ein- oder doppelseitigen medizinischen Klebeband an der Haut des Patienten befestigt sein. Das Gehäuse 355 schützt den Patienten vor möglichen Gefahren, wie z.B. elektrischer Spannung.

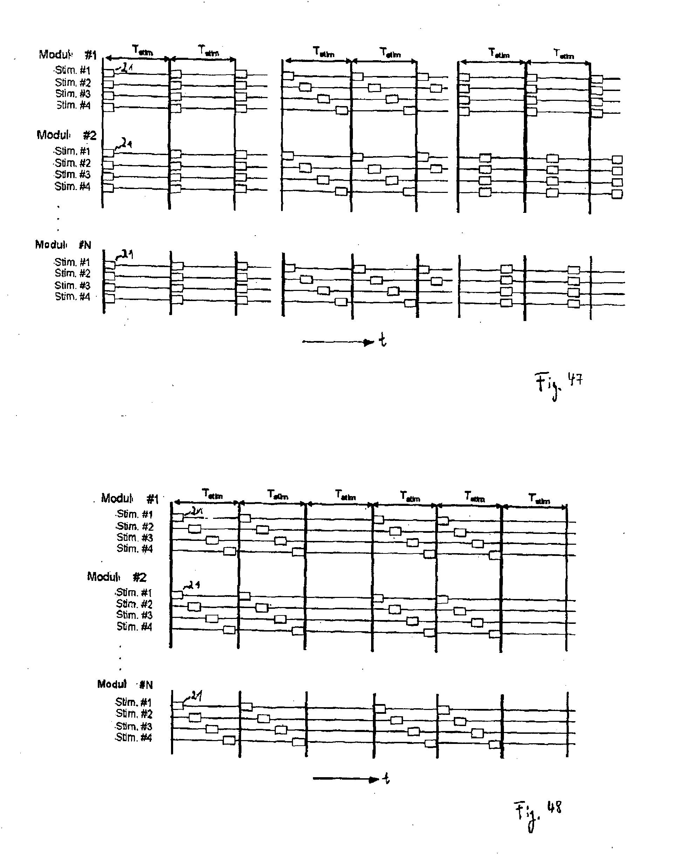

Die in dieser Anmeldung beschriebenen Stimulationselemente können einzeln am Patienten befestigt werden oder können auch zu mehreren in ein Modul integriert werden. Beispielsweise kann ein Modul eine Manschette mit mehreren daran befestigten Stimulationselementen umfassen. Die Manschette kann dann an einem Arm oder Bein des Patienten befestigt werden. Fig. 47 zeigt Stimulationsverfahren, wie sie mit insgesamt N Modulen, die jeweils z.B. vier Stimulationselemente enthalten, durchgeführt werden können. Bei dem in Fig. 47 ganz links dargestellten Stimulationsverfahren applizieren alle Stimulationselemente zu Beginn einer Stimulationsperiode $T_{stim}$ einen taktilen, vibratorischen oder thermischen ersten Reiz 21. Bei dem in der Mitte von Fig. 47 gezeigten Stimulationsverfahren sind die ersten Reize 21 der vier verschiedenen Stimulationselemente eines Moduls jeweils um $T_{stim}/4$ gegeneinander verschoben. In diesem Fall appliziert in jedem Zeitabschnitt der Länge $T_{stim}/4$ genau ein Stimulationselement jedes Moduls einen ersten Reiz 21. Bei dem in Fig. 47 ganz rechts dargestellten Stimulationsverfahren erzeugen die vier Stimulationselemente eines Moduls ihre ersten Reize 21 gleichzeitig, jedoch sind die ersten Reize 21 unterschiedlicher Module gegeneinander verschoben.

Bei allen in Fig. 47 gezeigten Stimulationsverfahren können auch beliebige Pausen während der Stimulation eingehalten werden. Typischerweise haben die Stimulationspausen die Länge einer oder mehrerer Stimulationsperioden $T_{stim}$. Beispielhaft ist dies in Fig. 48 gezeigt. Bei dem dort dargestellten Stimulationsverfahren wird eine Stimulation während zwei aufeinander folgender Stimulationsperioden $T_{stim}$ durchgeführt, danach wird während einer Stimulations-periode $T_{stim}$ eine Stimulationspause eingehalten. Dieses Muster wiederholt sich periodisch.

Des Weiteren kann den in den Fig. 47 und 48 gezeigten Stimulationsverfahren eine Randomisierung der Reihenfolge, in welcher die einzelnen Stimulationseinheiten die ersten Reize 21 generieren, hinzugefügt werden, wobei u.a. folgende Randomisierungen denkbar sind:

1. Randomisierung der Reizsequenzen für jede Stimulationsperiode $T_{stim}$ kohärent über alle Module, d.h. zu Beginn jeder Stimulationsperiode $T_{stim}$ wird eine Reihenfolge festgelegt, in der die Stimulationselemente die ersten Reize 21 generieren (z.B. die Reihenfolge Stim. #4, Stim. #2, Stim. #3, Stim. #1) und diese Reihenfolge gilt für alle Module.

2. Randomisierung der Reizsequenzen für einen Block von aufeinander folgenden Stimulationsperioden $T_{stim}$ ko-härent über alle Module, d.h. zu Beginn eines in Fig. 48 gezeigten Blocks von aufeinander folgenden Stimulations-perioden $T_{stim}$ (bzw. nach einer Stimulationspause) wird eine Reihenfolge festgelegt, in der die Stimulationselemente die ersten Reize 21 generieren (z.B. die Reihenfolge Stim. #4, Stim. #2, Stim. #3, Stim. #1) und diese Reihenfolge gilt für alle Module für den Stimulationsblock bis zur nächsten Pause.

3. Randomisierung der Reizsequenzen nicht kohärent über alle Module, sondern nur kohärent über eine Untergruppe aller Module variiert, d.h. nur für ein bestimmtes Modul (z.B. das Modul #2) wird eine Randomisierung gemäß den vorstehenden Ziffern 1. oder 2. durchgeführt, die übrigen Module verhalten sich wie in Fig. 47 gezeigt.

4. Randomisierung der Reizsequenzen nicht kohärent über alle Module, sondern kohärent über mehr als eine Untergruppe aller Module variiert, d.h. nur für zwei oder mehr Module (z.B. die Module #2 und #4) wird eine Randomisierung gemäß den vorstehenden Ziffern 1. oder 2. durchgeführt, die übrigen Module verhalten sich wie in Fig. 47 gezeigt.

5. Randomisierung der Reizsequenzen unkorreliert zwischen verschiedenen Modulen, d.h. für jede Stimulations-periode $T_{stim}$ oder für jeden Block von aufeinander folgenden Stimulationsperioden $T_{stim}$ zwischen zwei Pausen wird für jedes Modul unabhängig von den anderen Modulen eine Reihenfolge, in der die Stimulationselemente die ersten Reize 21 generieren, festgelegt.

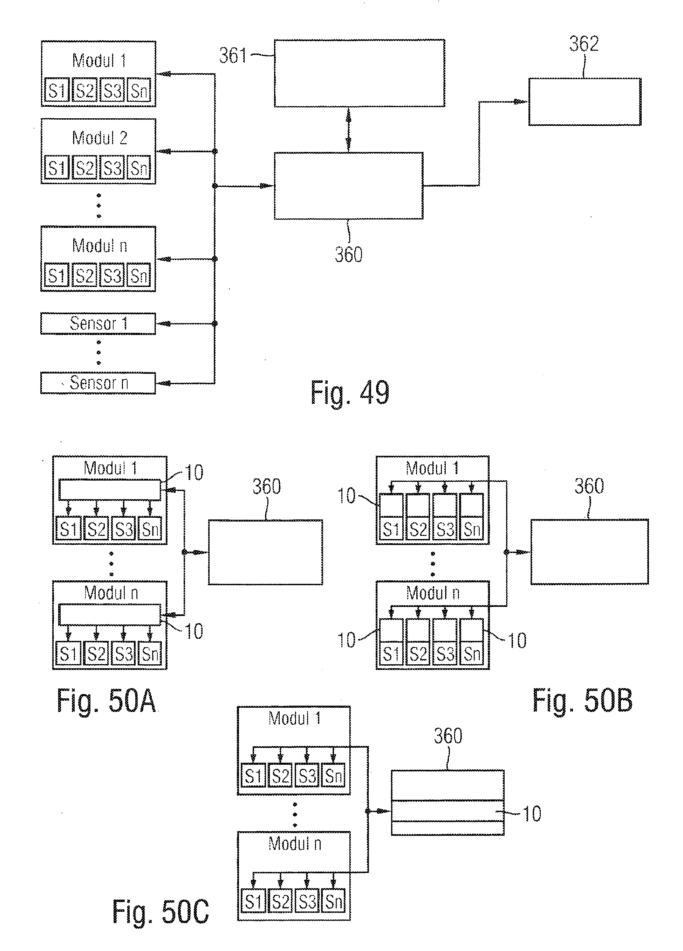

In Fig. 49 ist schematisch das Blockschaltbild einer Vorrichtung zur Erzeugung von taktilen, vibratorischen und/ oder thermischen ersten Reizen 21 dargestellt. Die Vorrichtung enthält n Module mit jeweils n Stimulationselementen sowie n Sensoren. Die Module und Sensoren stehen über Verbindungsleitungen oder über Funk (z.B. ein WPAN (Wireless Personal Area Network)-Netzwerk) mit einem Verbindungsmodul 360 in Verbindung, welches wiederum an einen Computer 361, z.B. ein Laptop, und externe Vorrichtungen 362 angeschlossen sein kann. Es müssen nicht notwendigerweise alle Module und Sensoren gleichzeitig zum Einsatz kommen, es kann je nach Stimulationsart auch nur eine Teilmenge davon eingesetzt werden. Die Module und/oder Sensoren können durch Batterien oder Akkus mit Strom versorgt werden, so dass sie unabhängig von einer zentralen Stromversorgung sind. Der Benutzer, beispielsweise ein Arzt, kann mittels einer geeigneten, auf dem Computer 361 abgespeicherten Software ein Stimulationsverfahren auswählen und die Parameter dieses Stimulationsverfahrens einstellen.

Die Steuerung der in die Module integrierten Stimulationseinheiten kann über den Computer 361 erfolgen. Als Alternative kann in jedes Modul eine Steuereinheit 10 integriert sein (vgl. Fig. 50A), die für die Ansteuerung der Stimulationselemente des jeweiligen Moduls zuständig ist. Dies ermöglicht einen weitgehend eigenständigen Betrieb der Module. Ferner kann für jedes Stimulationselement eine eigene Steuereinheit 10 vorgesehen sein (vgl. Fig. 50B). Dies ermöglicht die größte Vielseitigkeit beim Betrieb der Stimulationselemente, jedoch werden dadurch Gewicht und Abmessungen der Module vergrößert. Als weitere Alternative kann die Steuereinheit 10 zentral in dem Verbindungsmodul 360 platziert werden (vgl. Fig. 50C). Vorteilhaft daran sind geringes Gewicht und Größe der Module sowie eine kostengünstige Herstellung. Allerdings können bei dieser Ausgestaltung die Module nicht unabhängig von dem Verbindungsmodul 360 betrieben werden.

Fig. 51 zeigt schematisch eine Vorrichtung 5100, die eine wie z.B. oben beschriebene erste Stimulationseinheit mit Stimulationselementen 311 bis 314 zur Applikation der spezifischen taktilen, vibratorischen und/oder thermischen ersten Reize 21 und eine zweite Stimulationseinheit 12 zur Applikation der zweiten, unspezifischen Reize aufweist. Der Patient trägt die Stimulationselemente 311 bis 314 im Bereich des betroffenen Körperteils bzw. bei inneren Organen der zugehörigen Headschen Zone. In der vorliegenden Ausführungsform sind die Stimulationselemente 311 bis 314 an einem Arm des Patienten befestigt. Als zweite Stimulationseinheit 12 weist die Vorrichtung 5100 die in Fig. 4A und 4B gezeigte Konditionierungsuhr auf. Die zweiten, unspezifischen Reize können alternativ auch mittels einer anders ausgestalteten zweiten Stimulationseinheit 12 generiert werden.

**Patentansprüche**

1.   Vorrichtung (100) umfassend:

eine erste Stimulationseinheit (11), die derart ausgeführt ist, dass sie erste Reize (21) erzeugt, die bei einer Verabreichung an einen Patienten eine krankhaft synchrone Aktivität von Neuronen im Gehirn und/oder Rückenmark des Patienten unterdrücken,
eine zweite Stimulationseinheit (12) zur Erzeugung von optischen und/oder akustischen und/oder taktilen und/ oder vibratorischen und/oder thermischen zweiten Reizen (22), und
eine Steuereinheit (10) zur Steuerung der ersten und zweiten Stimulationseinheit (11, 12), wobei
die erste und zweite Stimulationseinheit (11, 12) nicht-invasive Stimulationseinheiten sind,
die Erzeugung der ersten und zweiten Reize (21, 22) wahlweise in einem ersten oder einem zweiten Betriebsmodus erfolgt, **gekennzeichnet dadurch, dass** die Steuereinheit (10) die erste und zweite Stimulationseinheit (11, 12) derart ansteuert, dass im ersten Betriebsmodus die Erzeugung von mindestens 60% der zweiten Reize (22) zeitlich an die Erzeugung der ersten Reize (21) gekoppelt ist und im zweiten Betriebsmodus die Erzeugung von mindestens 60% der zweiten Reize (22) ohne die Erzeugung der ersten Reize (21) erfolgt,
die Steuereinheit (10) anschließend an den ersten Betriebsmodus den zweiten Betriebsmodus wählt, und
die zweiten Reize (22) für sich alleine angewandt und ohne den vorherigen Betrieb der Vorrichtung (100) im ersten Betriebsmodus keine Unterdrückung einer krankhaft synchronen Aktivität von Neuronen im Gehirn und/ oder Rückenmark bewirken.

2.   Vorrichtung (100) nach Anspruch 1, wobei die ersten Reize (21) optische und/oder akustische und/oder taktile und/ oder vibratorische und/oder thermische Reize sind.

3.   Vorrichtung (100) nach Anspruch 1 oder 2, wobei im ersten Betriebsmodus die Erzeugung von mindestens 80% der zweiten Reize (22) zeitlich an die Erzeugung der ersten Reize (21) gekoppelt ist und/oder im zweiten Betriebsmodus die Erzeugung von mindestens 80% der zweiten Reize (22) ohne die Erzeugung der ersten Reize (21) erfolgt.

4.   Vorrichtung (100) nach einem der vorhergehenden Ansprüche, wobei die erste Stimulationseinheit (11) eine Transmissionsbrille, eine Brille mit einer Mehrzahl von Lichtquellen, einen Schallgenerator, einen Gleichstrommotor, eine Schwingspule, einen piezoelektrischen Wandler, ein elektroaktives Polymer, ein Heizelement, ein Kühlelement und/ oder eine Infrarotlichtquelle umfasst.

**5.** Vorrichtung (300) nach einem der vorhergehenden Ansprüche, wobei die Vorrichtung (300) eine Messeinheit (15) zum Aufnehmen von Messsignalen (25) umfasst, welche die krankhaft synchrone Aktivität der Neuronen wiedergeben.

**6.** Vorrichtung (300) nach Anspruch 5, wobei die Steuereinheit (10) im zweiten Betriebsmodus die Zahl der ersten Reize (21), deren Erzeugung zeitlich an die Erzeugung der zweiten Reize (22) gekoppelt ist, erhöht, falls die Messsignale (25) einen vorgegebenen ersten Schwellwert überschreiten.

**7.** Vorrichtung (300) nach Anspruch 5 oder 6, wobei die Steuereinheit (10) von dem zweiten Betriebsmodus in den ersten Betriebsmodus wechselt, falls die Messsignale (25) einen vorgegebenen zweiten Schwellwert überschreiten.

**8.** Vorrichtung (300) nach Anspruch 7, wobei der erste Schwellwert kleiner als der zweite Schwellwert ist.

**9.** Vorrichtung (300) nach einem der Ansprüche 5 bis 8, wobei die erste Stimulationseinheit (11) die Messsignale (25) in erste Reize (21) umwandelt oder die Messsignale (25) nach einer Weiterverarbeitung in erste Reize (21) umwandelt.

**10.** Vorrichtung (100) nach einem der vorhergehenden Ansprüche, wobei die ersten Reize (21) in ersten Zeitabschnitten ($\Delta t_1$) erzeugt werden und die zweiten Reize (22) in zweiten Zeitabschnitten ($\Delta t_2$) erzeugt werden und wobei im ersten Betriebsmodus mindestens 60% der zweiten Zeitabschnitte ($\Delta t_2$) jeweils mit einem der ersten Zeitabschnitte ($\Delta t_1$) zeitlich überlappen.

**11.** Vorrichtung (100) nach Anspruch 10, wobei der zeitliche Überlapp ($\Delta t_{12}$) eines der ersten Zeitabschnitte ($\Delta t_1$) mit einem der zweiten Zeitabschnitte ($\Delta t_2$) mindestens 10% der Zeitdauer des zweiten Zeitabschnitts ($\Delta t_2$) beträgt.

**12.** Vorrichtung (100) nach einem der vorhergehenden Ansprüche, wobei die Vorrichtung (100) ein Programmiergerät umfasst, mittels welchem der Patient einen Wechsel von dem zweiten Betriebsmodus in den ersten Betriebsmodus bewirken kann.

**Claims**

**1.** An apparatus (100) comprising
a first stimulation unit (11) which is designed such that it generates first stimuli (21) which, on administration to a patient, suppress a pathologically synchronous activity of neurons in the brain and/or spinal cord of the patient;
a second stimulation unit (12) for the generation of optical and / or acoustic and/or tactile and/or vibratory and/or thermal second stimuli (22); and
a control unit (10) for the control of the first and second stimulation units (11, 12), wherein
the first and second stimulation units (11, 12) are non-invasive stimulation units;
the generation of the first and second stimuli (21, 22) takes place selectively in a first or om a second mode of operation; **characterized in that**
the control unit (10) controls the first and second stimulation units (11, 12) such that the generation of at least 60% of the second stimuli (22) is coupled in time to the generation of the first stimuli (21) in the first mode of operation and such that the generation of at least 60% of the second stimuli (22) takes place without the generation of the first stimuli (21) in the second mode of operation;
the control unit (10) selects the second operating mode subsequent to the first operating mode; and
the second stimuli (22), when used on their own and without the previous operation of the apparatus (100) in the first operating mode, do not bring about a suppression of a pathologically synchronous activity of neurons in the brain and/or the spinal cord.

**2.** An apparatus (100) in accordance with claim 1, wherein the first stimuli (21) are optical and/ or acoustic and/ or tactile and/ or vibratory and/ or thermal stimuli.

**3.** An apparatus (100) in accordance with claim 1 or claim 2, wherein the generation of at least 80% of the second stimuli (22) is coupled in time to the generation of the first stimuli (21) in the first mode of operation and/or the generation of at least 80% of the second stimuli (22) takes place without the generation of the first stimuli (21) in the second mode of operation.

**4.** An apparatus (100) in accordance with any one of the preceding claims, wherein the first stimulation unit (11) comprises transmission eyeglasses, eyeglasses having a plurality of light sources, a sound generator, a direct current motor, an oscillator coil, a piezoelectric transducer, an electroactive polymer, a heating element, a cooling element and/or an infrared light source.

**5.** An apparatus (300) in accordance with any one of the preceding claims, wherein the apparatus (300) comprises a measurement unit (15) for the recording of measurement signals (25) which reproduce the pathologically synchronous activity of the neurons.

**6.** An apparatus (300) in accordance with claim 5, wherein the control unit (10) increases the number of the first stimuli (21), the generation of which is coupled in time to the generation of the second stimuli (22) if the measurement signals (25) exceed a predetermined first threshold value.

**7.** An apparatus (300) in accordance with claim 5 or claim 6, wherein the control unit (10) changes from the second mode of operation into the first mode of operation if the measurement signals (25) exceed a predetermined second threshold value.

**8.** An apparatus (300) in accordance with claim 7, wherein the first threshold value is smaller than the second threshold value.

**9.** An apparatus (300) in accordance with any one of the claims 5 to 8, wherein the first stimulation unit (11) converts the measurement signals (25) into first stimuli (21) or converts the measurement signals (25) into first stimuli (21) following a further processing thereof.

**10.** An apparatus (100) in accordance with any one of the preceding claims, wherein the first stimuli (21) are generated in first sections of time ($\Delta t_1$) and the second stimuli (22) are generated in second sections of time ($\Delta t_2$) and wherein at least 60% of the second sections of time ($\Delta t_2$) respectively overlap in time with one of the first sections of time ($\Delta t_1$) in the first mode of operation.

**11.** An apparatus (100) in accordance with claim 10, wherein the overlap in time ($\Delta t_{12}$) of one of the first sections of time ($\Delta t_1$) with one of the second sections of time ($\Delta t_2$) amounts to at least 10% of the duration in time of the second section of time ($\Delta t_2$).

**12.** An apparatus (100) in accordance with any one of the preceding claims, wherein the apparatus (100) comprises a programming device by means of which the patient can bring about a change from the second mode of operation into the first mode of operation.

**Revendications**

**1.** Appareil (100) comprenant :

une première unité de stimulation (11) qui est réalisé de telle façon qu'elles engendrent des premières excitations (21) qui, lors de l'administration à un patient, suppriment une activité synchrone pathologique de neurones dans le cerveau et/ou dans la moelle épinière du patient,
une seconde unité de stimulation (12) pour engendrer des secondes excitations optiques et/ou acoustiques et/ou tactiles et/ou vibratoires et/ou thermiques (22), et
une unité de commande (10) pour commander la première et la seconde unité de stimulation (11, 12), dans lequel la première et la seconde unité de stimulation (11, 12) sont des unités de stimulation non-invasives,
la génération des premières et des secondes excitations (21, 22) a lieu au choix dans un premier ou dans un second mode de fonctionnement,
**caractérisé en ce que**
l'unité de commande (17) pilote la première et la seconde unité de stimulation (11, 12) de telle façon que dans le premier mode de fonctionnement la génération d'au moins 60 % des secondes excitations (22) est temporellement couplée à la génération des premières excitations (21), et dans le second mode de fonctionnement la génération d'au moins 60 % des secondes excitations (22) a lieu sans génération des premières excitations (21),
à la suite du premier mode de fonctionnement, l'unité de commande (10) choisit le second mode de fonctionnement, et

les secondes excitations (22) appliquées seules et sans fonctionnement précédent de l'appareil (100) dans le premier mode de fonctionnement, ne provoquent aucune suppression d'une activité synchrone pathologique de neurones dans le cerveau et/ou dans la moelle épinière.

2. Appareil (100) selon la revendication 1, dans lequel les premières excitations (21) sont des excitations optiques et/ou acoustiques et/ou tactiles et/ou vibratoires et/ou thermiques.

3. Appareil (100) selon la revendication 1 ou 2, dans lequel dans le premier mode de fonctionnement, la génération d'au moins 80 % des secondes excitations (22) est couplée temporellement à la génération des premières explications (21) et/ou dans le second mode de fonctionnement la génération d'au moins 80 % des secondes excitations (22) a lieu sans l'excitation des premières excitations (21).

4. Appareil (100) selon l'une des revendications précédentes, dans lequel la première unité de stimulation comprend des lunettes à transmission, des lunettes avec une pluralité de sources de lumière, un générateur acoustique, un moteur à courant continu, une bobine oscillante, un convertisseur piézoélectrique, un polymère électro-actif, un élément chauffant, un élément de refroidissement et/ou une source de lumière infrarouge.

5. Appareil (300) selon l'une des revendications précédentes, dans lequel l'appareil (300) inclut une unité de mesure (15) pour enregistrer des signaux de mesure (25) qui reproduisent l'activité synchrone pathologique des neurones.

6. Appareil (300) selon la revendication 5, dans lequel l'unité de commande (10) augmente dans le second mode de fonctionnement le nombre des premières excitations (21) dont la génération est couplée temporellement à la génération des secondes excitations (22) si les signaux de mesure (25) dépassent une première valeur seuil prédéterminée.

7. Appareil (300) selon la revendication 5 ou 6, dans lequel l'unité de commande (10) change du second mode de fonctionnement au premier mode de fonctionnement si les signaux de mesure (25) dépassent une seconde valeur seuil prédéterminée.

8. Appareil (300) selon la revendication 7, dans lequel la première valeur seuil est plus petite que la seconde valeur seuil.

9. Appareil (300) selon l'une des revendications 5 à 8, dans lequel la première unité de stimulation (11) convertit les signaux de mesure (25) en premières excitations (21), ou convertit les signaux de mesure (25) après un traitement ultérieur en premières excitations (21).

10. Appareil (100) selon l'une des revendications précédentes, dans lequel les premières excitations (21) sont engendrées dans des premières fenêtres temporelles ($\Delta t1$) et les secondes excitations (22) sont engendrées dans des secondes fenêtres temporelles ($\Delta t2$), et dans le premier mode de fonctionnement au moins 60 % des secondes fenêtres temporelles ($\Delta t2$) chevauchent respectivement temporellement l'une des premières fenêtres temporelles ($\Delta t1$).

11. Appareil (100) selon la revendication 10, dans lequel le chevauchement temporel ($\Delta t12$) de l'une des premières fenêtres temporelles ($\Delta t1$) avec l'une des secondes fenêtres temporelles ($\Delta t2$) s'élève à au moins 10 % de la durée temporelle de la seconde fenêtre temporelle ($\Delta t2$).

12. Appareil (100) selon l'une des revendications précédentes, dans lequel l'appareil (100) comprend un appareil de programmation moyen duquel le patient peut provoquer un changement du second mode de fonctionnement vers le premier mode de fonctionnement.

Fig. 1

$\Delta t_1$

$\Delta t_1$

21

t

$\Delta t_2$

$\Delta t_2$

22

t

$\Delta t_{12}$

$\Delta t_{Pause}$

$\Delta t_{12}$

P

P

P

P

P

Fig. 2A

$\Delta t_1$

21

t

$\Delta t_2$

22

t

U

P

U

U

P

Fig. 2B

EP 2 547 398 B1

Fig. 3

Fig. 4A

Fig. 4B

Fig.5

11

112

113

10

115

116

114

Fig. 6

y

117

118

112

$\varphi_{118}$

113

$\psi_{113}$

$\varphi_{113}$

x

Fig. 7

11

112

113

10

~26

15

115

116

114

810

Fig. 8

Fig. 9

Fig. 10

Fig. 11

Fig. 12

Fig. 13

Fig. 14

Fig. 15

Fig. 16

Fig. 17

Fig. 18

Fig. 49

Fig. 20

Fig. 21

Fig. 22

Fig.23

Fig. 24

Fig. 25

Fig. 26

Fig. 27

$t_{start}$

1

0

-1

0   2   4   6   8   10   12

t[s]

1
$f_1$
0

1
$f_2$
0

1
$f_3$
0

1
$f_4$
0

0   2   4   6   8   10   12

t[s]

Fig. 28

Fig. 23

Fig 30

Fig. 31A

Fig. 31B

Fig. 32

Fig. 33

Fig. 34A

Fig. 34B

Fig. 34C

Fig. 34D

Fig. 35

Fig. 36 A

Fig. 36B

Fig. 36C

58

Fig. 37

Fig. 38

Fig. 39

Fig. 40

Fig. 41

Fig. 42A  Fig. 42B  Fig. 42C

Fig. 43A  Fig. 43B

Fig. 44A

Fig. 44B

Fig. 44C

Fig. 45A

Fig. 45B

Fig. 45C

Fig. 46C

Fig. 46B

Fig. 46A

EP 2 547 398 B1

Fig. 47

Fig. 48

Fig. 49

Fig. 50A

Fig. 50B

Fig. 50C

Fig. 51

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2103288 A2
- WO 2009136931 A
- DE 102008012669
- DE 102008015259
- DE 102010000390

**In der Beschreibung aufgeführte Nicht-Patentliteratur**

- **VON B. A. WANDELL ; S. O. DUMOULIN ; A. A. BREWER.** Visual Field Maps in Human Cortex. *Neuron,* Oktober 2007, vol. 56, 366-383
- **VON D. BILECEN ; K. SCHEFFLER ; N. SCHMID ; K. TSCHOPP ; J. SEELIG.** Tonotopic organization of the human auditory cortex as detected by BOLD-FMRI. *Hearing Research,* 1998, vol. 126, 19-27
- **VON D. R. M. LANGERS ; W. H. BACKES ; P. VAN DIJK.** Representation of lateralization and tonotopy in primary versus secondary human auditory cortex. *NeuroImage,* 2007, vol. 34, 264-273
- **VON W. MÜHLNICKEL ; T. ELBERT ; E. TAUB ; H. FLOR.** Reorganization of auditory cortex in tinnitus. *Proc. Natl. Acad. Sci. USA,* 1998, vol. 95, 10340-10343
- **A. BENNINGHOFF et al.** Lehrbuch der Anatomie des Menschen. Dargestellt unter Bevorzugung funktioneller Zusammenhänge. 3. Bd. Nervensystem, Haut und Sinnesorgane. *Urban und Schwarzenberg,* 1964